(19) Europäisches Patentamt
European Patent Office
Office européen des brevets

(11) EP 4 778 919 A1

(12) **EUROPEAN PATENT APPLICATION**
published in accordance with Art. 153(4) EPC

(43) Date of publication:
**22.07.2026 Bulletin 2026/30**

(21) Application number: **24865226.5**

(22) Date of filing: **27.08.2024**

(51) International Patent Classification (IPC):
***C07D 277/56*** (2006.01) ***A01N 43/78*** (2006.01)
***A01P 3/00*** (2006.01)

(52) Cooperative Patent Classification (CPC):
**A01N 43/78; A01P 3/00; C07D 277/56**

(86) International application number:
**PCT/JP2024/030537**

(87) International publication number:
**WO 2025/057723 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.09.2023 JP 2023148047**
**13.09.2023 JP 2023148050**

(71) Applicant: **Ishihara Sangyo Kaisha, Ltd.**
**Osaka-shi, Osaka 550-0002 (JP)**

(72) Inventors:
- **FUSHIKIDA Kuni**
  **Osaka-shi, Osaka 550-0002 (JP)**
- **IWAMOTO Takuya**
  **Osaka-shi, Osaka 550-0002 (JP)**
- **HAYASHI Hiroyuki**
  **Osaka-shi, Osaka 550-0002 (JP)**
- **TOSAKI Tatsuhiko**
  **Osaka-shi, Osaka 550-0002 (JP)**
- **OHNO Masanari**
  **Osaka-shi, Osaka 550-0002 (JP)**
- **KATO Kohsuke**
  **Osaka-shi, Osaka 550-0002 (JP)**
- **SENUMA Wakana**
  **Osaka-shi, Osaka 550-0002 (JP)**
- **MAKINO Tatsuaki**
  **Osaka-shi, Osaka 550-0002 (JP)**

(74) Representative: **Wächtershäuser & Hartz Patentanwaltspartnerschaft mbB**
**Weinstraße 8**
**80333 München (DE)**

(54) **N-SUBSTITUTED OXYAMIDE COMPOUND OR SALT THEREOF**

(57) To provide a novel compound having excellent controlling effects against harmful plant diseases.
A compound represented by the formula (I) or a salt thereof:

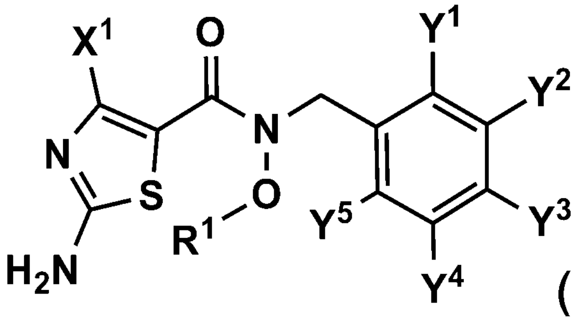

(I)

wherein the reference symbols are as defined in the specification, has excellent controlling effects against harmful plant diseases.

EP 4 778 919 A1

## Description

### TECHNICAL FIELD

The present invention relates to a novel N-substituted oxyamide compound or a salt thereof, and an agricultural and horticultural fungicide comprising it as an active ingredient.

### BACKGROUND ART

Existing agricultural and horticultural fungicides may be insufficient in practical controlling effects against harmful plant diseases in some application site, and may have problems of emergence of chemical resistant strains and of environmental burden (influence over surrounding plants and ecosystem). Accordingly, novel agricultural and horticultural fungicides have been desired. Further, from the viewpoint of prevention of environmental pollution, agricultural and horticultural fungicides showing excellent controlling effects against harmful plant diseases at the lowest possible dose have been desired.

Patent Document 1 discloses amide compounds and their application to control of plant diseases. However, Patent Document 1 does not at all specifically describe a compound represented by the following formula (I).

As a compound which can be used as a microbiocide, for example, N-alkoxycarboxamides have been known (Patent Document 2). However, Patent Document 2 does not at all specifically describe a compound represented by the following formula (I).

Non-Patent Document 1 discloses at Table I, No.15, 5-hydroxytryptophan decarboxylase inhibitory activity test and monoamine oxidase inhibitory activity test of N-Benzoyl-N-benzyl-O-2-propynylhydroxylamine. However, Non-Patent Document 1 does not at all describe controlling effects of the described compounds against harmful plant diseases. Further, Non-Patent Document 1 does not at all describe a compound represented by the following formula (I).

2-amino-N-benzyl-N-methoxy-1,3-thiazole-5-carboxamide (CAS Registry Number: 2301497-10-7) and 2-amino-N-benzyl-N-methoxy-4-methyl-1,3-thiazole-5-carboxamide (CAS Registry Number: 2299658-69-6) have been known. However, their application is unknown, and needless to say, effects of these compounds on plant diseases are not at all known.

### PRIOR ART DOCUMENTS

### PATENT DOCUMENTS

Patent Document 1: WO2010/134634
Patent Document 2: WO2011/023645

### NON-PATENT DOCUMENTS

Non-Patent Document 1: Journal of Medicinal Chemistry 1969, 12(1), 45-48

### DISCLOSURE OF INVENTION

### TECHNICAL PROBLEM

The object of the present invention is to provide a novel compound having excellent controlling effects against harmful plant diseases.

### SOLUTION TO PROBLEM

The present inventors have conducted extensive studies to achieve the above object and as a result found that a compound represented by the following formula (I) or a salt thereof has excellent controlling effects against harmful plant diseases.

That is, the present invention provides the following.

[1] A compound represented by the formula (I) or a salt thereof:

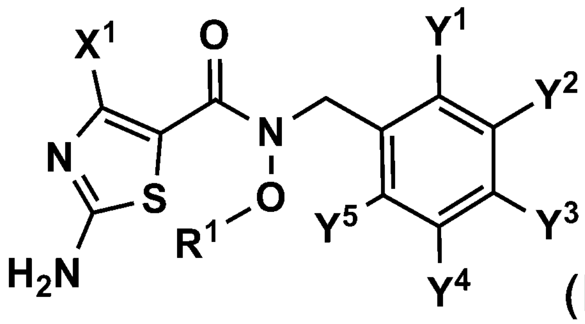

(I)

wherein $R^1$ is a ($C_1$-$C_5$)-chain hydrocarbon or a ($C_3$-$C_4$)-cycloalkyl (provided that the ($C_1$-$C_5$)-chain hydrocarbon and the ($C_3$-$C_4$)-cycloalkyl may be substituted with at least one $T^1$),
$T^1$ is a halogen, cyano or -$U^1$-$R^2$,
$U^1$ is O, S or C(=O)O,
$R^2$ is a ($C_1$-$C_3$)-alkyl,
$X^1$ is a halogen, and
$Y^1$, $Y^2$, $Y^3$, $Y^4$ and $Y^5$ are each independently a halogen, a ($C_1$-$C_6$)-alkyl, a ($C_1$-$C_6$)-haloalkyl, nitro, cyano or a hydrogen atom, provided that when $R^1$ is a ($C_1$-$C_4$)-alkyl or a ($C_3$-$C_4$)-cycloalkyl, all of $Y^1$, $Y^2$, $Y^3$, $Y^4$ and $Y^5$ are not hydrogen atoms at the same time (hereinafter referred to also as compound (I)).

[2] The compound or a salt thereof according to [1], wherein $R^1$ is a ($C_1$-$C_4$)-alkyl, a ($C_3$-$C_4$)-cycloalkyl or a ($C_3$-$C_5$)-alkynyl.
[3] The compound or a salt thereof according to [1], wherein the compound of the formula (I) is a N-(cyclo)alkoxyamide compound represented by the formula (IA):

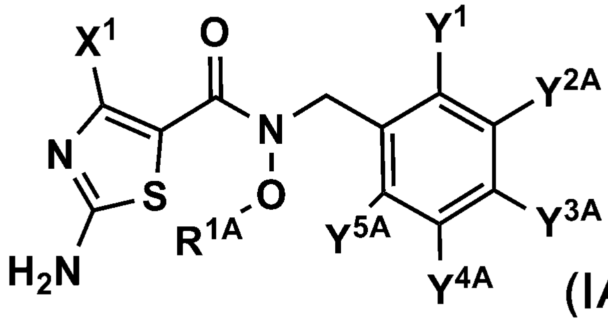

(IA)

**wherein** $R^{1A}$ is a ($C_1$-$C_4$)-alkyl or a ($C_3$-$C_4$)-cycloalkyl,
$X^1$ is a halogen,
$Y^1$ is a halogen, a ($C_1$-$C_6$)-alkyl, a ($C_1$-$C_6$)-haloalkyl, nitro, cyano or a hydrogen atom,
$Y^{2A}$, $Y^{3A}$ and $Y^{4A}$ are each independently a hydrogen atom or a halogen, and
$Y^{5A}$ is a hydrogen atom, provided that when $Y^1$ is a hydrogen atom, all of $Y^{2A}$, $Y^{3A}$ and $Y^{4A}$ are not hydrogen atoms at the same time (hereinafter referred to also as compound (IA)).

[4] The compound or a salt thereof according to [1], wherein the compound of the formula (I) is a N-alkynyloxyamide compound represented by the formula (IB):

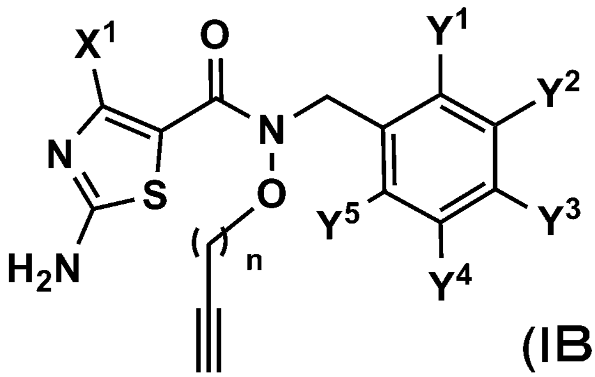

(IB)

wherein n is 1, 2 or 3,
$X^1$ is a halogen, and
$Y^1$, $Y^2$, $Y^3$, $Y^4$ and $Y^5$ are each independently a hydrogen atom, a halogen, a $(C_1-C_6)$-alkyl, a $(C_1-C_6)$-haloalkyl, nitro or cyano (hereinafter referred to also as compound (IB)).

[5] The compound or a salt thereof according to [1], wherein the compound of the formula (I) is a N-(cyclo)alkoxyamide compound of the formula (IA):

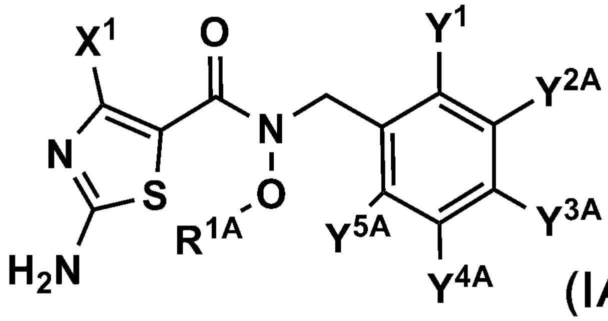

(IA)

wherein $R^{1A}$ is a $(C_1-C_4)$-alkyl or a $(C_3-C_4)$-cycloalkyl,
$X^1$ is a halogen,
$Y^1$ is a halogen, a $(C_1-C_6)$-alkyl, a $(C_1-C_6)$-haloalkyl, nitro, cyano or a hydrogen atom,
$Y^{2A}$, $Y^{3A}$ and $Y^{4A}$ are each independently a hydrogen atom or a halogen, and
$Y^{5A}$ is a hydrogen atom, provided that when $Y^1$ is a hydrogen atom, all of $Y^{2A}$, $Y^{3A}$ and $Y^{4A}$ are not hydrogen atoms at the same time,
or a N-alkynyloxyamide compound of the formula (IB):

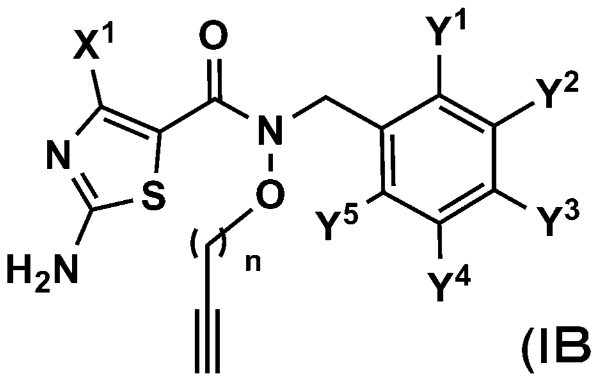

(IB)

wherein n is 1, 2 or 3,
$X^1$ is a halogen, and
$Y^1$, $Y^2$, $Y^3$, $Y^4$ and $Y^5$ are each independently a hydrogen atom, a halogen, a $(C_1-C_6)$-alkyl, a $(C_1-C_6)$-haloalkyl, nitro or cyano.

[6] The compound or a salt thereof according to any one of [1] to [5], wherein $Y^1$ is a halogen, a $(C_1-C_6)$-alkyl, a $(C_1-C_6)$-haloalkyl, nitro or cyano.

[7] The compound or a salt thereof according to [3] or [5], wherein $R^{1A}$ is methyl, ethyl or cyclopropyl.

[8] The compound or a salt thereof according to any one of [1], [2] and [4] to [6], wherein $Y^5$ is a hydrogen atom.

[9] An agricultural and horticultural fungicide, which comprises as an active ingredient the compound or a salt thereof as defined in any one of [1] to [8].

[10] A method for controlling harmful plant diseases, which comprises applying an effective amount of the compound or a salt thereof as defined in any one of [1] to [8] to a plant body, phytopathogen or soil.

ADVANTAGEOUS EFFECTS OF INVENTION

The compound (I) of the present invention has excellent controlling effects against harmful plant diseases and is useful as an agricultural and horticultural fungicide.

DESCRIPTION OF EMBODIMENTS

Various substituents and chemical structures described in this specification will be described. The compound (I) includes compound (IA) and compound (IB), and they will sometimes generically be referred to as Invention Compound.

The halogen or the halogen as a substituent may, for example be a fluorine, chlorine, bromine or iodine atom. The number of the halogen as the substituent may be one or more, and when two or more, the respective halogen atoms may be identical or different from one another. The position of the halogen as the substituent may be any position.

The expression "$C_P-C_T$" means that the number of carbon atoms is P to T. For example, the expression "$C_1-C_4$" means that the number of carbon atoms is 1 to 4. Further, the expression "which may be substituted" means having a substituent or being unsubstituted.

The $(C_1-C_5)$-chain hydrocarbon means a $(C_1-C_4)$-alkyl, a $(C_3-C_5)$-alkynyl or a $(C_3-C_5)$-alkenyl.

The $(C_1-C_4)$-alkyl may, for example, be a linear or branched alkyl group having 1 to 4 carbon atoms, such as methyl, ethyl, n-propyl, isopropyl, n-butyl, isobutyl, sec-butyl or tert-butyl.

The $(C_3-C_5)$-alkynyl may, for example, be a linear or branched alkynyl group having 3 to 5 carbon atoms, having at least one triple bond at an optional position, such as 1-propynyl, propargyl (hereinafter sometimes referred to simply as 2-propynyl), 1-butynyl, 2-butynyl, 3-butynyl, 3-butyn-2-yl, buta-1,3-diynyl, 1-pentynyl, 2-pentynyl, 3-pentynyl, 4-pentynyl, 3-methyl-1-butynyl, 3-pentyn-2-yl, 1-pentyn-3-yl, 4-pentyn-2-yl, 2-methyl-3-butynyl, penta-1,3-diynyl, penta-1,4-diynyl or penta-2,4-diynyl.

The $(C_3-C_5)$-alkenyl may, for example, be a linear or branched alkenyl group having 3 to 5 carbon atoms, having at least one double bond at an optional position, such as allyl (hereinafter sometimes referred to simply as 2-propenyl), 1-propenyl, isopropenyl, 1-butenyl, 2-butenyl, 3-butenyl, buta-1,3-dienyl, buta-1,3-dien-2-yl, 2-methyl-1-propenyl, 2-buten-2-yl, 3-buten-2-yl, 2-methyl-2-propenyl, 3-buten-3-yl, 1-pentenyl, 2-pentenyl, 3-pentenyl, 4-pentenyl, 2-penten-2-yl, 3-methyl-2-buten-2-yl, 3-methyl-2-butenyl, penta-1,3-dienyl, penta-2,4-dienyl or penta-2,4-dien-2-yl. Further, in a case where such a group has geometrical isomers, the group may be one of E-isomer and Z-isomer or may be a mixture of E-isomer and Z-isomer in an optional ratio and is not particularly limited so long as it has carbon atoms within a specified range.

The $(C_1-C_6)$-alkyl may, for example, be a linear or branched alkyl group having 1 to 6 carbon atoms, such as methyl, ethyl, n-propyl, isopropyl, n-butyl, isobutyl, sec-butyl, tert-butyl, n-pentyl, isopentyl, neopentyl, sec-pentyl, 1-ethylpropyl, tert-pentyl, n-hexyl, isohexyl, sec-hexyl, 2-methylpentyl, 3-methylpentyl, 1-ethylbutyl, 2-ethylbutyl, 1,2-dimethylbutyl, 1,3-dimethylbutyl, 2,3-dimethylbutyl, tert-hexyl, 2,2-dimethylbutyl, neohexyl, 1-ethyl-2-methylpropyl or 1-ethyl-1-methylpropyl.

The $(C_1-C_3)$-alkyl is a linear or branched alkyl group having 1 to 3 carbon atoms. Its specific examples correspond to the alkyl groups having 1 to 3 carbon atoms among the above specific examples of the $(C_1-C_6)$-alkyl.

The $(C_3-C_4)$-cycloalkyl means a cyclic hydrocarbon group having 3 to 4 carbon atoms, which can form a 3- to 4-membered monocyclic structure. In a case where a 3-membered monocyclic structure is formed, it may be substituted with a methyl group at an optional position within a specified number of carbon atoms. For example, a cycloalkyl group having 3 to 4 carbon atoms, such as cyclopropyl, 1-methylcyclopropyl, 2-methylcyclopropyl or cyclobutyl may be mentioned.

The $(C_1-C_5)$-chain hydrocarbon and the $(C_3-C_4)$-cycloalkyl may be substituted with at least one $T^1$. In a case where the $(C_1-C_5)$-chain hydrocarbon is substituted with $T^1$, the $(C_1-C_5)$-chain hydrocarbon may be substituted with one to nine $T^1$. In a case where the $(C_3-C_4)$-cycloalkyl is substituted with $T^1$, the $(C_3-C_4)$-cycloalkyl may be substituted with one to seven $T^1$.

In a case where the $(C_1-C_5)$-chain hydrocarbon or the $(C_3-C_4)$-cycloalkyl is substituted with at least one $T^1$, the position of substitution with $T^1$ may be any position on the $(C_1-C_5)$-chain hydrocarbon or the $(C_3-C_4)$-cycloalkyl.

In a case where the $(C_1-C_5)$-chain hydrocarbon or the $(C_3-C_4)$-cycloalkyl is substituted with two or more $T^1$, the

respective substituents $T^1$ may be identical or different from one another.

For example, in a case where $R^1$ in the compound (I) is a $(C_1\text{-}C_4)$-alkyl substituted with at least one $T^1$, specifically, the following substituents may be mentioned. For example, fluoromethyl, difluoromethyl, trifluoromethyl, chloromethyl, dichloromethyl, trichloromethyl, bromomethyl, dibromomethyl, tribromomethyl, chlorodifluoromethyl, 1-fluoroethyl, 2-fluoroethyl, 1,1-difluoroethyl, 2,2-difluoroethyl, 2,2,2-trifluoroethyl, perfluoroethyl, 3,3-difluoropropyl, 3,3,3-trifluoropropyl, perfluoropropyl, perfluoroisopropyl, 4-fluorobutyl, 4,4-difluorobutyl, 4,4,4-trifluorobutyl, cyanomethyl, 2-cyanoethyl, 3-cyanopropyl, methoxymethyl, ethoxymethyl, propoxymethyl, 2-methoxyethyl, methylthiomethyl, ethylthiomethyl, propylthiomethyl, 2-methylthioethyl, methoxycarbonylmethyl, ethoxycarbonylmethyl, propoxycarbonylmethyl, 2-(methoxycarbonyl)ethyl or isopropoxycarbonylmethyl may be mentioned.

For example, in a case where $R^1$ in the compound (I) is a $(C_3\text{-}C_4)$-cycloalkyl substituted with at least one $T^1$, specifically, the following substituents may be mentioned. For example, 1-fluorocyclopropyl, 2-fluorocyclopropyl, 2,2-difluorocyclopropyl, 1-cyanocyclopropyl, 2-cyanocyclopropyl, 1-methoxycyclopropyl, 2-methoxycyclopropyl, 1-methylthiocyclopropyl, 2-methylthiocyclopropyl, 1-methoxycarbonylcyclopropyl, 2-methoxycarbonylcyclopropyl, 1-fluorocyclobutyl, 2-fluorocyclobutyl, 3-fluorocyclobutyl, 2,2-difluorocyclobutyl, 3,3-difluorocyclobutyl, 1-cyanocyclobutyl, 2-cyanocyclobutyl, 3-cyanocyclobutyl, 1-methoxycyclobutyl, 2-methoxycyclobutyl, 3-methoxycyclobutyl, 1-methylthiocyclobutyl, 2-methylthiocyclobutyl, 3-methylthiocyclobutyl, 1-methoxycarbonylcyclobutyl, 2-methoxycarbonylcyclobutyl or 3-methoxycarbonylcyclobutyl may be mentioned.

The $(C_1\text{-}C_6)$-haloalkyl may, for example, be a linear or branched alkyl group having 1 to 6 carbon atoms, partially or completely substituted with 1 to 13 halogen atoms which are identical or different from one another, such as fluoromethyl, difluoromethyl, trifluoromethyl, chloromethyl, dichloromethyl, trichloromethyl, bromomethyl, dibromomethyl, tribromomethyl, chlorodifluoromethyl, dichlorofluoromethyl, chlorofluoromethyl, 1-fluoroethyl, 2-fluoroethyl, 1,1-difluoroethyl, 2,2-difluoroethyl, 2,2,2-trifluoroethyl, perfluoroethyl, 1-chloroethyl, 2-chloroethyl, 1,1-dichloroethyl, 2,2-dichloroethyl, 2,2,2-trichloroethyl, 1-chloro-1-fluoroethyl, 2-chloro-2-fluoroethyl, 1-fluoropropyl, 2-fluoropropyl, 1-fluoro-2-propyl, 3-fluoropropyl, 2-fluoro-2-propyl, 1,1-difluoropropyl, 2,2-difluoropropyl, 3,3-difluoropropyl, 3,3,3-trifluoropropyl, perfluoropropyl, perfluoroisopropyl, 2,2,3,3,3-pentafluoropropyl, 1,1,1,3,3,3-hexafluoroisopropyl, 1-fluorobutyl, 2-fluorobutyl, 3-fluorobutyl, 4-fluorobutyl, 1,1-difluorobutyl, 2,2-difluorobutyl, 3,3-difluorobutyl, 4,4-difluorobutyl, 4,4,4-trifluorobutyl, 3,3,4,4,4-pentafluorobutyl, 2,2,3,3,4,4,4-heptafluorobutyl, perfluorobutyl, 2,3,3,3,4,4,4-heptafluoroisobutyl, 1-fluoropentyl, 2-fluoropentyl, 3-fluoropentyl, 4-fluoropentyl, 5-fluoropentyl, 1,1-difluoropentyl, 2,2-difluoropentyl, 3,3-difluoropentyl, 4,4-difluoropentyl, 5,5-difluoropentyl, 5,5,5-trifluoropentyl, 4,4,5,5,5-pentafluoropentyl, 3,3,4,4,5,5,5-heptafluoropentyl, 2,2,3,3,4,4,5,5,5-nonafluoropentyl, perfluoropentyl, 1-fluorohexyl, 2-fluorohexyl, 3-fluorohexyl, 4-fluorohexyl, 5-fluorohexyl, 6-fluorohexyl, 1,1-difluorohexyl, 2,2-difluorohexyl, 3,3-difluorohexyl, 4,4-difluorohexyl, 5,5-difluorohexyl, 6,6-difluorohexyl, 6,6,6-trifluorohexyl, 5,5,6,6,6-pentafluorohexyl, 4,4,5,5,6,6,6-heptafluorohexyl, 3,3,4,4,5,5,6,6,6-nonafluorohexyl, 2,2,3,3,4,4,5,5,6,6,6-undecafluorohexyl or perfluorohexyl.

The $(C_1\text{-}C_3)$-haloalkyl means a linear or branched haloalkyl group having 1 to 3 carbon atoms. Its specific examples correspond to the haloalkyl groups having 1 to 3 carbon atoms among the above specific examples of the $(C_1\text{-}C_6)$-haloalkyl.

In the above formula (IB), n is 1, 2 or 3. Thus, the compound (IB) is specifically represented by the following formulae (IB-a) to (IB-c).

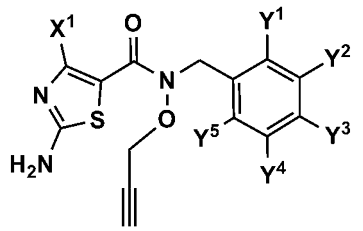

(IB-a)

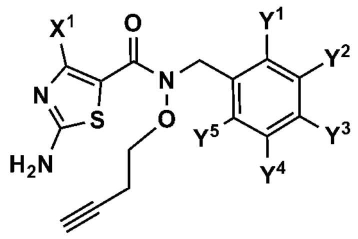

(IB-b)

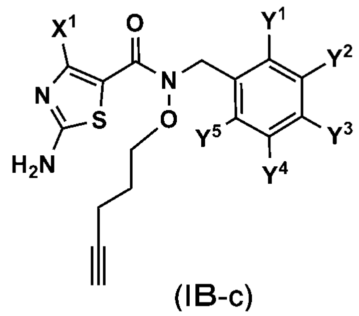

(IB-c)

Among the compounds represented by the formula (I), preferred compounds include the N-(cyclo)alkoxyamide compound of the formula (IA), the N-alkynyloxyamide compound of the formula (IB), and compounds represented by the following formulae (I-a) to (I-m).

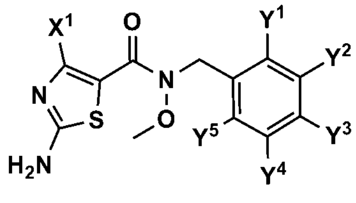

(I-a)

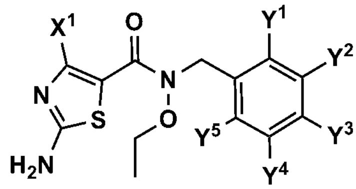

(I-b)

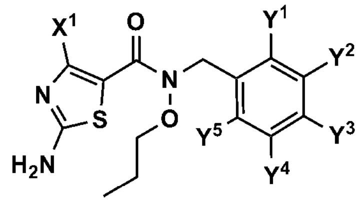

(I-c)

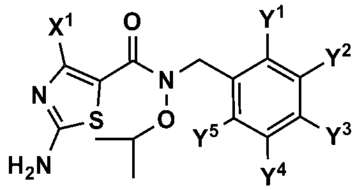

(I-d)

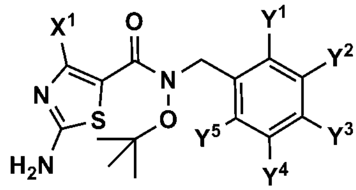

(I-e)

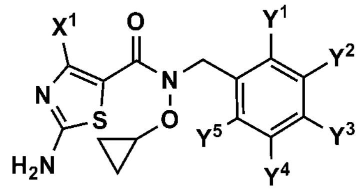

(I-f)

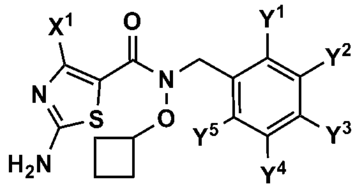

(I-g)

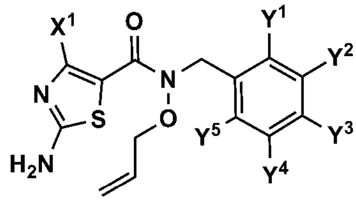

(I-h)

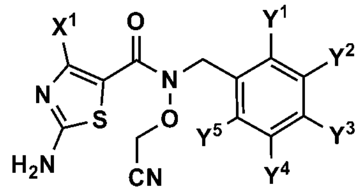

(I-i)

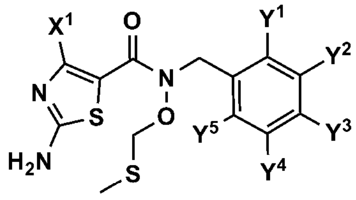

(I-j)

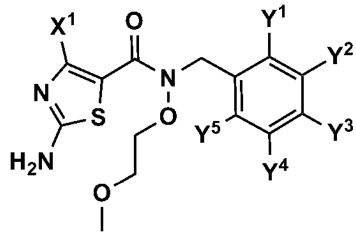

(I-k)

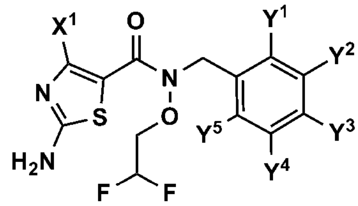

(I-l)

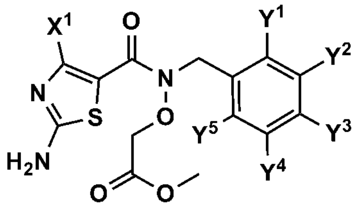

(I-m)

The salts of the compound (I) include all kinds of salts so long as they are agriculturally acceptable, and, for example, alkali metal salts (such as a sodium salt and a potassium salt), alkaline earth metal salts (such as a magnesium salt and a calcium salt), amine salts (such as a dimethylamine salt and a triethylamine salt), inorganic acid salts (such as a hydrochloride, a perchlorate, a sulfate and a nitrate), and organic acid salts (such as an acetate, a methanesulfonate, a p-toluenesulfonate and an oxalate) may be mentioned.

As the compound (I), isomers such as optical isomers and geometrical isomers may sometimes be present, and the present invention includes the respective isomers and isomer mixtures. In the compound (I), various isomers other than the above-mentioned isomers are included within a range of common knowledge in this technical field. Further, an individual isomer may be synthesized by common knowledge and general experimental means in this technical field.

Further, depending upon the type of an isomer, it may have a chemical structure different from the described structural formula, but for those skilled in the art, it is sufficiently recognizable that such a chemical structure is in a relation of an isomer, and such an isomer is evidently within the scope of the present invention.

Now, the method for producing the compound (I) will be described.

The compound (I) may be produced in accordance with the following Reactions A to E and a conventional method for producing a salt, however, the method to obtain the compound is not limited to such a method. For example, the compound (I) of the present invention may be produced by applying a substituent transformation (such as alkylation, haloalkylation, cross coupling reaction such as Suzuki coupling, Sandmeyer reaction, halogenation, oxidation or reduction) known in this technical field, on the substituent on the phenyl group. Further, in the production of the Invention Compound, protection and deprotection reaction commonly employed in this technical field may be applied as the case requires. The reaction may be carried out in an atmosphere of an inert gas such as nitrogen or argon as the case requires, and a salt reagent may be used.

[Reaction A]

Reaction A is a deprotection reaction, which is a method of removing a Boc group from a compound of the formula (XX-a) to obtain the compound of the formula (I). The Boc group is a tert-butoxycarbonyl group.

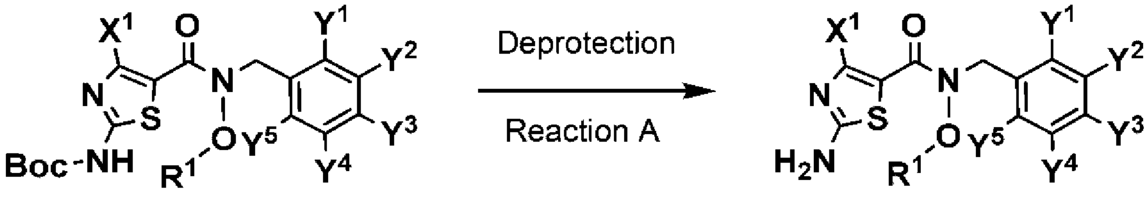


(XX-a) (I)

Symbols in the formulae are as defined above.

Reaction A may be carried out under known conditions employed to remove the Boc group, for example, in accordance with the method described in Greene's PROTECTIVE GROUPS in ORGANIC SYNTHESIS (John Wiley and Sons, 2007, Peter G.M.Wuts, Theodora W.Greene). More specifically for example, the compound of the formula (XX-a) is reacted with an acid such as trifluoroacetic acid or hydrogen chloride in the presence of a solvent, or reacted with trimethylsilyl triflate in the presence of a solvent and a base such as 2,6-lutidine.

[Reaction B] and [Reaction C]

Reaction B is a method of reacting a compound of the formula (II) with a compound of the formula (III) to obtain a compound of the formula (XX-b). Reaction C is a method of reacting a compound of the formula (II-a) with a compound of the formula (III) to obtain a compound of the formula (XX-b).

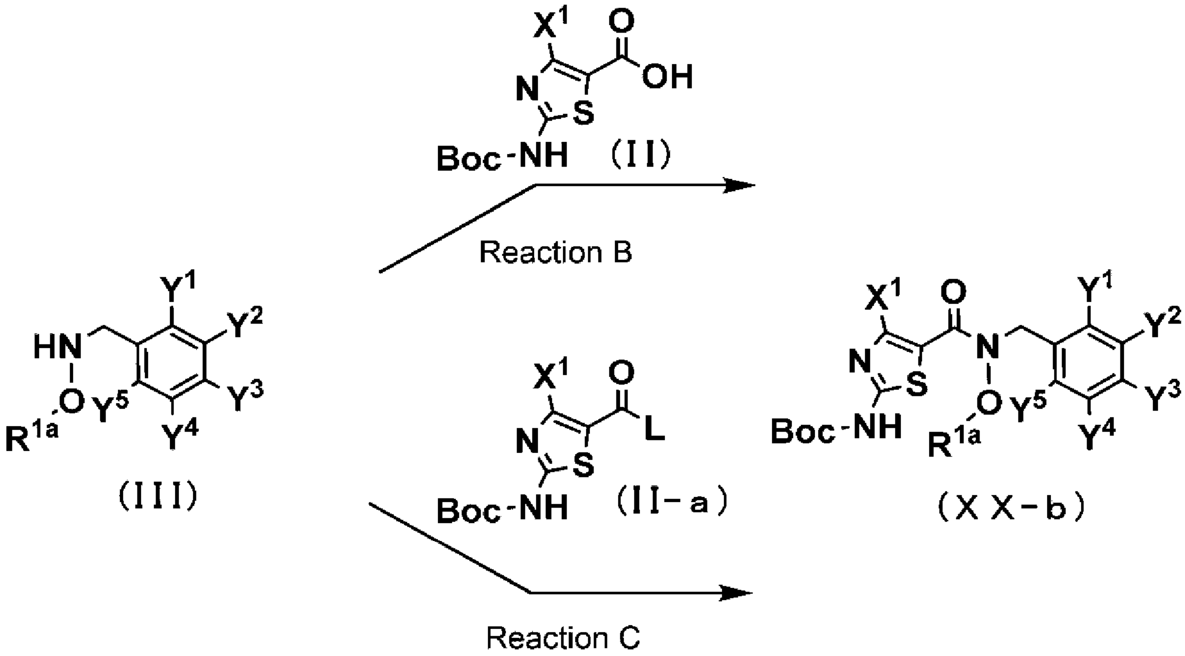


(II)

(III)

(II-a)

(XX-b)

In the formulae, $R^{1a}$ is H, a $(C_1-C_5)$-chain hydrocarbon or a $(C_3-C_4)$-cycloalkyl (provided that the $(C_1-C_5)$-chain hydrocarbon and the $(C_3-C_4)$-cycloalkyl may be substituted with at least one $T^1$). L is a leaving group, and may, for example, be halogen, alkoxy, aryloxy, alkylcarbonyloxy or arylcarbonyloxy. The other symbols are as defined above.

Reaction B may be carried out usually in the presence of a dehydration condensation agent and a solvent, and optionally with the addition of a base. In Reaction B, the compound of the formula (III) may be used in an amount of 0.5 to 3 equivalents, preferably 0.8 to 1.5 equivalents to 1 equivalent of the compound of the formula (II) (equivalents means molar equivalents, the same applies hereinafter).

In Reaction B, the dehydration condensation agent may, for example, be a carbodiimide condensation agent such as N,N'-dicyclohexylcarbodiimide (DCC), 1-[3-(dimethylamino)propyl]-3-ethylcarbodiimide (EDC) or its hydrochloride; an imidazole condensation agent such as 1,1'-carbonyldiimidazole (CDI); a triazine condensation agent such as 4-(4,6-dimethoxy-1,3,5-triazin-2-yl)-4-methylmorphonium chloride (DMT-MM); a phosphonium condensation agent such as 1H-benzotriazol-1-yloxy-tripyrrolidinophosphonium hexafluorophosphate (PyBOP); an uronium condensation agent such as 1-[bis(dimethylamino)methylene]-1H-1,2,3-triazolo[4,5-b]pyridinium 3-oxide hexafluorophosphate (HATU); an acid anhydride such as 2-methyl-6-nitrobenzoic anhydride (MNBA); a 2-halopyridinium salt such as 2-chloro-1-methylpyridinium p-toluenesulfonate; propylphosphonic acid anhydride (cyclic trimer) (T3P); and diphenylphosphoryl azide (DPPA); but is not limited thereto. Optionally, a conventional additive used in combination with a dehydration condensation agent, such as 1-hydroxybenzotriazole (HOBt), may be added. The dehydration condensation agent may be used in an amount of 0.5 to 5 equivalents, preferably 1 to 2 equivalents to 1 equivalent of the compound of the formula (II), and the additive may be used in an amount of 0.2 to 5 equivalents, preferably 1 to 2 equivalents to 1 equivalent of the compound of the formula (II).

In Reaction B, as the base, one or more may properly be selected and mixed from carbonates such as sodium carbonate, potassium carbonate and cesium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; and alkali metal carboxylates such as sodium acetate and potassium acetate. The base may be used in an amount of 0.5 to 10 equivalents, preferably 1 to 5 equivalents to 1 equivalent of the compound of the formula (II).

In Reaction B, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; protic polar solvents such as methanol and ethanol; and water.

In Reaction B, the reaction temperature is usually about -20°C to 150°C, preferably about 0°C to 100°C, and the reaction time is usually about 0.5 to 48 hours, preferably about 1 to 24 hours.

Reaction C may be carried out in the presence of a solvent, and optionally with the addition of a base. In Reaction C, the compound of the formula (III) may be used in an amount of 0.5 to 3 equivalents, preferably 0.8 to 1.5 equivalents to 1 equivalent of the compound of the formula (II-a).

In Reaction C, as the base, one or more may properly be selected and mixed from carbonates such as sodium carbonate, potassium carbonate and cesium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; and alkali metal carboxylates such as sodium acetate and potassium acetate. The base may be used in an amount of 0.1 to 10 equivalents, preferably 0.5 to 5 equivalents to 1 equivalent of the compound of the formula (II-a).

In Reaction C, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; protic polar solvents such as methanol and ethanol; and water.

In Reaction C, the reaction temperature is usually about -20°C to 150°C, preferably about 0°C to 100°C, and the reaction time is usually about 0.5 to 48 hours, preferably about 1 to 24 hours.

The compound of the formula (III) used in Reaction B or Reaction C may be produced in accordance with the following Reaction 2-2 or Reaction 2-4.

The compound of the formula (II) used in Reaction B may be produced in accordance with the following Reaction 1-1, Reaction 1-2 or a known method, or may be commercially available.

The compound of the formula (II-a) used in Reaction C may be produced from the compound of the formula (II) in accordance with the following Reaction 1-4 or a known method. The compound of the formula (II-a) may be commercially available.

[Reaction D]

Reaction D is a method of reacting a compound of the formula (XX-c) with a compound of the formula (IV) to obtain a compound of the formula (XX-a).

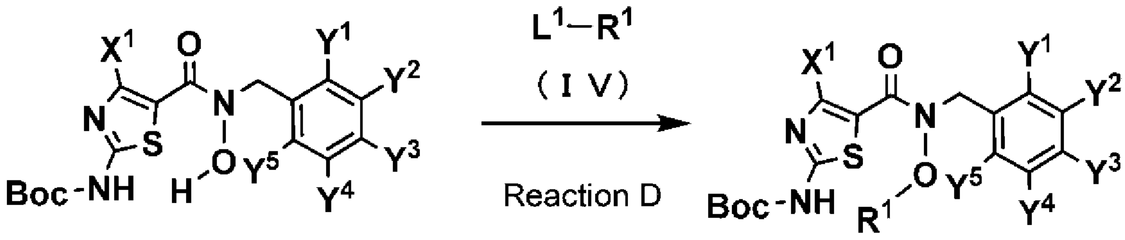


(XX-c) (XX-a)

In the formulae, $L^1$ is a leaving group, such as halogen, trifluoromethanesulfonyloxy, methanesulfonyloxy or p-toluenesulfonyloxy, and the other symbols are as defined above.

Reaction D may be carried out usually in the presence of a base and a solvent, and optionally with the addition of a phase transfer catalyst. In Reaction D, the compound of the formula (IV) may be used in an amount of 1 to 5 equivalents, preferably 1 to 3 equivalents to 1 equivalent of the compound of the formula (XX-c).

In Reaction D, as the base, one or more may properly be selected and mixed from alkali metal alkoxides such as sodium methoxide, sodium ethoxide and potassium tert-butoxide; carbonates such as sodium carbonate, potassium carbonate and cesium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; organic lithium compounds such as n-butyllithium and lithium diisopropylamide; and alkali metal carboxylates such as sodium acetate and potassium acetate. The base may be used in an amount of 1 to 10 equivalents, preferably 1 to 5 equivalents to 1 equivalent of the compound of the formula (XX-c).

In Reaction D, the solvent may be any solvent so long as it is inert to the reaction, and one or more may properly be selected from aromatic hydrocarbons such as benzene, toluene, xylene and chlorobenzene; aliphatic hydrocarbons such

as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; alcohols such as methanol, ethanol, propanol and tert-butanol; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; and water.

In Reaction D, the phase transfer catalyst may, for example, be quaternary ammonium salts such as tetrabutylammonium bromide, benzyltriethylammonium chloride or tetrabutylammonium hydrogen sulfate; or crown ethers such as 18-crown-6-ether. The phase transfer catalyst may be used in an amount of 0.1 to 3 equivalents to 1 equivalent of the compound of the formula (XX-c).

In Reaction D, the reaction temperature is usually about -20°C to 150°C, preferably about 0°C to 100°C, and the reaction time is usually about 10 minutes to about 48 hours, preferably about 1 to 24 hours.

The compound of the formula (IV) used in Reaction D may be produced in accordance with a known method, or may be commercially available.

[Reaction E]

Reaction E is a method of reacting a compound of the formula (II-b) with a compound of the formula (V) to obtain the compound of the formula (XX-b).

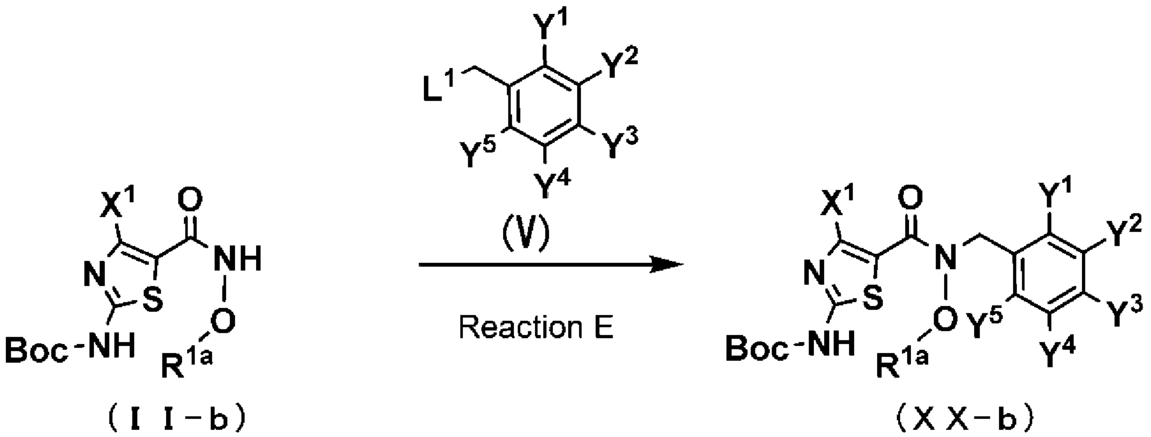


Symbols in the formulae are as defined above.

Reaction E may be carried out in accordance with the above Reaction D. In Reaction E, the compound of the formula (V) may be used in an amount of 1 to 5 equivalents, preferably 1 to 2 equivalents to 1 equivalent of the compound of the formula (II-b). In Reaction E, the base may be used in an amount of 1 to 10 equivalents, preferably about 1 to 5 equivalents to 1 equivalent of the compound of the formula (II-b). In Reaction E, the phase transfer catalyst may be used in an amount of 0.1 to 3 equivalents to 1 equivalent of the compound of the formula (II-b).

The compound of the formula (II-b) used in Reaction E may be produced in accordance with the following Reaction 1-3 or a known method, or may be commercially available. The compound of the formula (V) used in Reaction E may be produced in accordance with a known method, or may be commercially available.

The compounds used in Reaction A to Reaction E may be produced in accordance with the following Intermediate Production Process (Reaction 1-1 to Reaction 1-4 and Reaction 2-1 to Reaction 2-4) and a conventional method for producing a salt, but their production is not limited to these methods. Such compounds may be produced in accordance with a known method, or may be commercially available.

Intermediate Production Process

[Reaction 1-1], [Reaction 1-2] and [Reaction 1-3]

Reaction 1-1 is a method of oxidizing a compound of the formula (1) to obtain the compound of the formula (II). Reaction 1-2 is a method of hydrolyzing a compound of the formula (2) to obtain the compound of the formula (II). Reaction 1-3 is a method of reacting the compound of the formula (II) or the compound of the formula (II-a) with a compound of the formula (3) to obtain the compound of the formula (II-b).

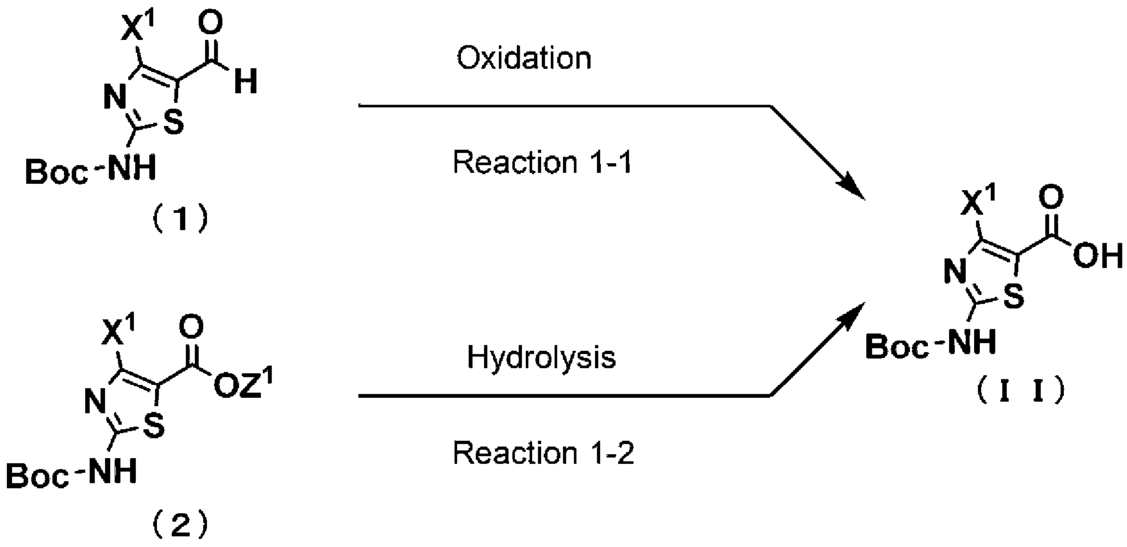

(1)
(2)
(I I)

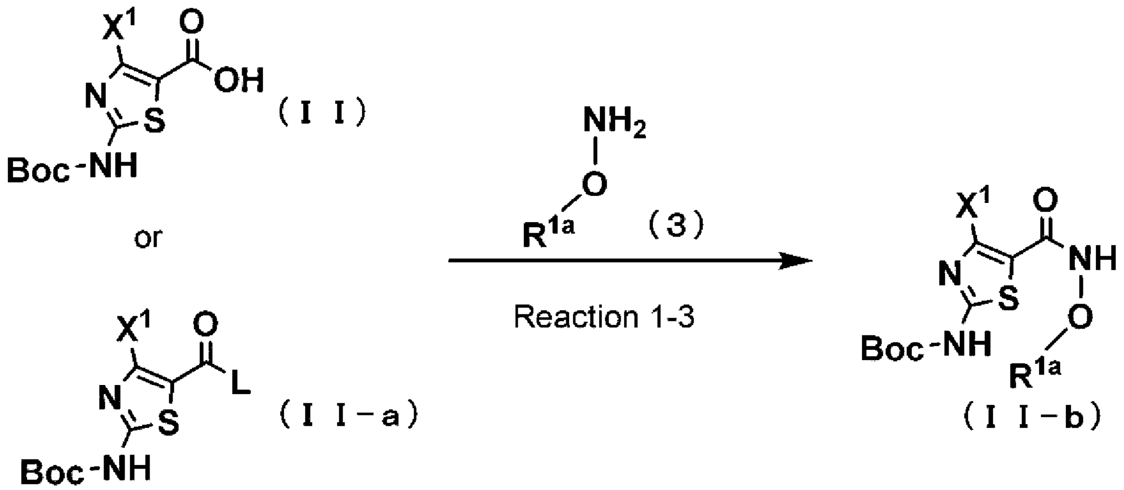

(I I)
or
(I I-a)
(I I-b)

In the formulae, $Z^1$ is an alkyl, and the other symbols are as defined above.

Reaction 1-1 may be carried out under conventional Pinnick oxidation conditions, for example, in accordance with the method described in Bioorganic & Medicinal Chemistry, 2004, 12, 6171-6182.

The compound of the formula (1) used in Reaction 1-1 may be obtained by a known method, for example, in accordance with the method described in Bioorganic & Medicinal Chemistry, 2004, 12, 6171-6182, Journal of Organic Chemistry, 2005, 70, 567-574, WO2020/028141, or Organic Process Research and Development, 2021, 25, 1167-1175, or may be commercially available.

Reaction 1-2 may be carried out under conventional conditions for hydrolysis of esters, for example, in accordance with the method described in WO2009/100171.

The compound of the formula (2) used in Reaction 1-2 may be produced by a known method, for example, in accordance with the method described in WO2012/006760, or may be commercially available.

Reaction 1-3 may be carried out in accordance with the above Reaction B or Reaction C. In Reaction 1-3, the compound of the formula (3) may be used in an amount of 0.5 to 10 equivalents, preferably 0.7 to 5 equivalents to 1 equivalent of the compound of the formula (II) or the compound of the formula (II-a).

The compound of the formula (3) used in Reaction 1-3 may be produced by a known method, for example, in accordance with the method described in WO2006/138350, US Publication 2014/0378399, Organic & Biomolecular Chemistry, 2020, 18, 3281-3287, The Journal of Antibiotics, 2000, 53, 1071-1085., or may be commercially available.

[Reaction 1-4]

Reaction 1-4 is a method to obtain the compound of the formula (II-a) from the compound of the formula (II) by halogenation, esterification or carbonylation.

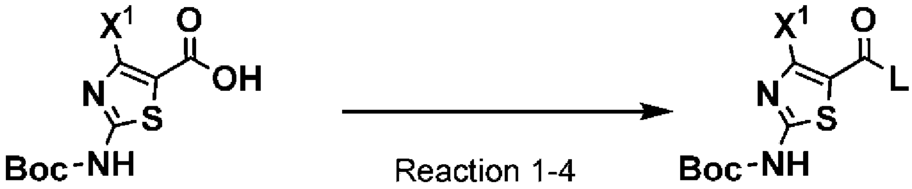


(I I)

(I I-a)

Symbols in the formulae are as defined above.

Reaction conditions in a case where Reaction 1-4 is halogenation will be described below.

In Reaction 1-4, in a case where L in the compound of the formula (II-a) is a halogen, usually, the halogenation can be carried out by reacting the compound of the formula (II) with a halogenating agent in the presence of a solvent, and optionally with the addition of N,N-dimethylformamide.

In Reaction 1-4, the halogenating agent may, for example, be oxalyl chloride, thionyl chloride, phosphorus oxychloride, phosphorus oxybromide, phosphorus trichloride, phosphorus tribromide, phosphorus pentachloride or sulfuryl chloride. The halogenating agent may be used in an amount of 1 to 10 equivalents, preferably 1 to 3 equivalents to 1 equivalent of the compound of the formula (II), or may be used in excess within a range not to impair the reaction.

In Reaction 1-4, in a case where N,N-dimethylformamide is used, the amount of N,N-dimethylformamide is a catalytic amount and is for example 0.01 to 0.3 equivalents to 1 equivalent of the compound of the formula (II).

Reaction conditions in a case where Reaction 1-4 is esterification will be described below.

In Reaction 1-4, in a case where L in the compound of the formula (II-a) is an alkoxy or an aryloxy, usually, the compound of the formula (II) is reacted with an alcohol or an arylhydroxy usually in the presence of a solvent and a dehydration condensation agent, and optionally with the addition of a base, for esterification.

In Reaction 1-4, the alcohol may, for example, be methanol or ethanol. In Reaction 1-4, the arylhydroxy may, for example, be phenol. The alcohol and the arylhydroxy may be used in an amount of 0.5 to 5 equivalents, preferably 0.8 to 1.5 equivalents, to 1 equivalent of the compound of the formula (II), or may be used in excess within a range not to impair the reaction.

As the dehydration condensation agent in Reaction 1-4, ones mentioned for the above Reaction B may be used. In Reaction 1-4, in a case where the dehydration condensation agent is used, optionally a conventional additive (for example 1-hydroxybenzotriazole (HOBt)) used with a dehydration condensation agent may be added. The dehydration condensation agent may be used in an amount of 0.5 to 5 equivalents, preferably 1 to 2 equivalents to 1 equivalent of the compound of the formula (II), and the additive may be used in an amount of 0.2 to 5 equivalents, preferably 1 to 2 equivalents to 1 equivalent of the compound of the formula (II).

In Reaction 1-4, in a case where a base is used with the dehydration condensation agent, as the base, ones mentioned for the above Reaction B may be used. The base may be used in an amount of 0.5 to 10 equivalents, preferably 1 to 5 equivalents to the compound of the formula (II).

In Reaction 1-4, in a case where L in the compound of the formula (II-a) is an alkoxy, the compound of the formula (II) is reacted with an alkyl halide usually with the addition of a solvent and a base for esterification.

In Reaction 1-4, the alkyl halide may, for example, be methyl iodide or ethyl iodide. The alkyl halide may be used in an amount of 0.5 to 5 equivalents, preferably 0.8 to 1.5 equivalents to 1 equivalent of the compound of the formula (II).

In Reaction 1-4, as the base to be used for the reaction with the alkyl halide, ones mentioned for the above Reaction D may be used. The base may be used in an amount of 0.5 to 10 equivalents, preferably 1 to 5 equivalents to 1 equivalent of the compound of the formula (II).

Reaction conditions in a case where Reaction 1-4 is carbonylation will be described below.

In Reaction 1-4, in a case where L in the compound of the formula (II-a) is an alkylcarbonyloxy or an arylcarbonyloxy, the compound of the formula (II) is reacted with a carbonylation agent usually in the presence of a solvent and a base for carbonylation.

In Reaction 1-4, the carbonylation agent may, for example, be acetyl chloride, pivaloyl chloride, benzoyl chloride, acetic anhydride or benzoic anhydride. The carbonylation agent may be used in an amount of 0.5 to 10 equivalents, preferably 1 to 5 equivalents to 1 equivalent of the compound of the formula (II), or may be used in excess within a range not to impair the reaction.

In a case where Reaction 1-4 is carbonylation, as the base used, ones mentioned for the above Reaction B may be used. The base may be used in an amount of 0.5 to 10 equivalents, preferably 1 to 5 equivalents to 1 equivalent of the compound of the formula (II).

In Reaction 1-4, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and

cyclohexane; ethers such as dioxane, tetrahydrofuran, diethyl ether and dimethoxyethane; esters such as methyl acetate and ethyl acetate; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; protic polar solvents such as methanol and ethanol; and water.

In Reaction 1-4, the reaction temperature is usually about -50°C to 200°C, preferably about -20°C to 100°C, and the reaction time is usually about 0.1 to 12 hours.

[Reaction 2-1] to [Reaction 2-4]

Reaction 2-1 is a method of reacting a compound of the formula (10) with the compound of the formula (3) to obtain a compound of the formula (11). Reaction 2-2 is a method of reducing the compound of the formula (11) to obtain the compound of the formula (III). Reaction 2-1 and Reaction 2-2 may also be carried out sequentially without isolating the compound of the formula (11).

Reaction 2-3 is a method of reacting a compound of the formula (11-a) with the compound of the formula (IV) to obtain a compound of the formula (11-b).

Reaction 2-4 is a method of reacting the compound of the formula (V) with the compound of the formula (3) to obtain the compound of the formula (III).

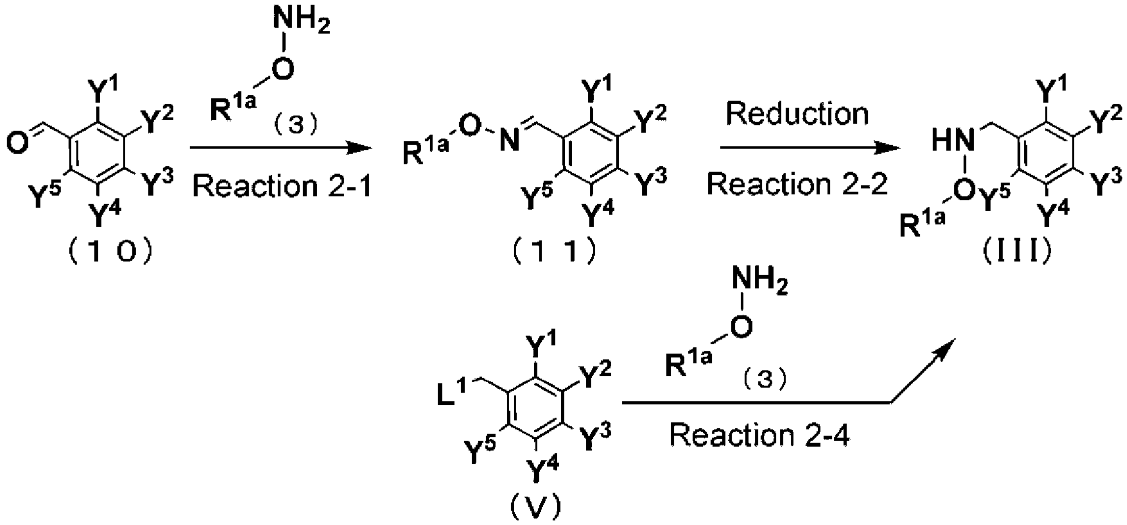


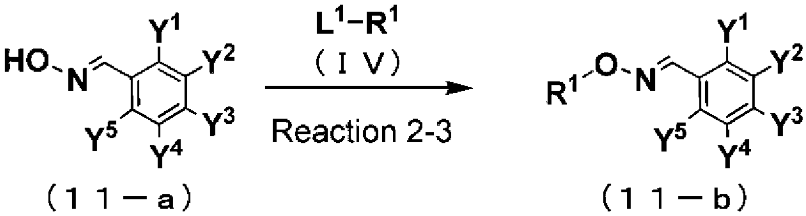


Symbols in the formulae are as defined above.

Reaction 2-1 may be carried out optionally with the addition of an acid, a base or a dehydrating agent. Reaction 2-1 may also be carried out in the presence of a solvent. In such a case, in Reaction 2-1, the compound of the formula (3) may be used in an amount of 1 to 5 equivalents to 1 equivalent of the compound of the formula (10), or may be used in excess within a range not to impair the reaction.

In Reaction 2-1, the acid may be either an inorganic acid or an organic acid, the inorganic acid may be hydrochloric acid or sulfuric acid, and the organic acid may be acetic acid, methanesulfonic acid or p-toluenesulfonic acid. The acid may be used in an amount of usually 0.1 to 10 equivalents to 1 equivalent of the compound of the formula (10), or may be used in excess within a range not to impair the reaction.

In Reaction 2-1, as the base, alkali metal alkoxides such as sodium methoxide, sodium ethoxide and potassium tert-butoxide; carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; and alkali metal carboxylates such as sodium acetate and potassium acetate; may be mentioned. The base may be used in an amount of 0.5 to 5 equivalents to 1 equivalent of the compound of the formula (3).

In Reaction 2-1, the dehydrating agent may, for example, be anhydrous magnesium sulfate, anhydrous sodium sulfate or molecular sieve.

In Reaction 2-1, the solvent may be any solvent so long as it is inert to the reaction, and one or more may properly be selected from halogenated aliphatic hydrocarbons such as dichloromethane and chloroform; aromatic hydrocarbons such as toluene and xylene; ethers such as dioxane, tetrahydrofuran, diethyl ether and dimethoxyethane; esters such as methyl acetate and ethyl acetate; aprotic polar solvents such as acetonitrile; protic solvents such as methanol and ethanol; and water.

In Reaction 2-1, the reaction temperature is usually about -20°C to 200°C, preferably about 0°C to 150°C, and the reaction time is usually about 0.5 to 48 hours, preferably about 1 to 24 hours.

Reaction 2-1 may also be carried out under conditions of conventional dehydration condensation of an aldehyde and a hydroxylamine or an alkoxyamine, for example, in accordance with the method described in e.g. HETEROCYCLES, 2009, 78, 463-470. In a case where Reaction 2-1 is carried out under conditions of conventional dehydration condensation, the compound of the formula (3) in Reaction 2-1 may be used in an amount of 0.7 to 5 equivalents, preferably 1 to 2 equivalents to 1 equivalent of the compound of the formula (10).

The compound of the formula (10) and the compound of the formula (3) used in Reaction 2-1 may be produced in accordance with a known method, or may be commercially available.

Reaction 2-2 may be carried out usually in the presence of a reducing agent and a solvent, and optionally with the addition of an acid.

In Reaction 2-2, the reducing agent may, for example, be sodium borohydride, sodium cyanoborohydride, sodium triacetoxyborohydride or 2-picoline borane complex. The reducing agent may be used in an amount of 0.5 to 10 equivalents, preferably 1 to 5 equivalents to 1 equivalent of the compound of the formula (11). The acid may be either an inorganic acid or an organic acid, and the inorganic acid may be hydrochloric acid or the like, and the organic acid may be acetic acid, trifluoroacetic acid or the like. The acid may be used in an amount of 1 to 10 equivalents to 1 equivalent of the compound of the formula (11).

In Reaction 2-2, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; protic polar solvents such as methanol and ethanol; and water.

In Reaction 2-2, the reaction temperature is usually about -20°C to 150°C, preferably about 0°C to 100°C, and the reaction time is usually about 0.5 to 48 hours, preferably about 1 to 24 hours.

Reaction 2-2 may also be carried out under conditions of conventional reduction of oxime or oxime ether, for example in accordance with the method described in e.g. HETEROCYCLES, 2009, 78, 463-470.

The compound of the formula (11) used in Reaction 2-2 may be obtained in accordance with the above Reaction 2-1 or a known method, or may be commercially available.

Reaction 2-3 may be carried out in accordance with Reaction D. The compound of the formula (IV) may be used in an amount of 1 to 5 equivalents, preferably 1 to 2 equivalents to 1 equivalent of the compound of the formula (11-a). In Reaction 2-3, the base may be used in an amount of 1 to 10 equivalents, preferably 1 to 5 equivalent to 1 equivalent of the compound of the formula (11-a). In Reaction 2-3, the phase transfer catalyst may be used in an amount of 0.1 to 3 equivalents to 1 equivalent of the compound of the formula (11-a).

Reaction 2-3 may also be carried out under conditions of known oxime alkylation, for example in accordance with the method described in WO2010/049946.

The compound of the formula (11-a) used in Reaction 2-3 may be produced in accordance with the above Reaction 2-1 or a known method, or may be commercially available. The compound of the formula (IV) used in Reaction 2-3 may be produced in accordance with a known method, or may be commercially available.

Reaction 2-4 may be carried out usually in the presence of a base and a solvent, and optionally with the addition of a phase transfer catalyst. In Reaction 2-4, the compound of the formula (3) may be used in an amount of 1 to 5 equivalents, preferably 1 to 3 equivalents to 1 equivalent of the compound of the formula (V).

In Reaction 2-4, as the base, one or more may properly be selected and mixed from alkali metal alkoxides such as sodium methoxide, sodium ethoxide and potassium tert-butoxide; carbonates such as sodium carbonate and potassium carbonate; hydrogencarbonates such as sodium hydrogencarbonate and potassium hydrogencarbonate; metal hydroxides such as sodium hydroxide and potassium hydroxide; metal hydrides such as sodium hydride and potassium hydride; amines such as triethylamine and N,N-diisopropylethylamine; pyridines such as pyridine, 4-dimethylaminopyridine and 2,6-lutidine; organic lithium compounds such as n-butyllithium and lithium diisopropylamide; and alkali metal carboxylates such as sodium acetate and potassium acetate. The base may be used in an amount of 1 to 10 equivalents, preferably 1 to 5 equivalents to 1 equivalent of the compound of the formula (V).

In Reaction 2-4, the solvent may be any solvent so long as it is inert to the reaction, and one or more may properly be selected from aromatic hydrocarbons such as benzene, toluene, xylene and chlorobenzene; aliphatic hydrocarbons such as carbon tetrachloride, methyl chloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; alcohols such as methanol, ethanol, propanol and tert-butanol; aprotic polar solvents such as dimethyl sulfoxide, sulfolane, N,N-dimethylacetamide, N,N-dimethylformamide, N-methylpyrrolidone, pyridine, acetonitrile and propionitrile; ketones such as acetone and methyl ethyl ketone; and water.

In Reaction 2-4, the phase transfer catalyst may, for example, be quaternary ammonium salts such as tetrabutylammonium bromide, benzyltriethylammonium chloride or tetrabutylammonium hydrogen sulfate; or crown ethers such as 18-crown-6-ether. The phase transfer catalyst may be used in an amount of 0.1 to 3 equivalents to 1 equivalent of the compound of the formula (V).

In Reaction 2-4, the reaction temperature is usually about -20°C to 150°C, preferably about 0°C to 100°C, and the reaction time is usually about 10 minutes to about 48 hours, preferably about 1 to 24 hours.

Reaction 2-4 may also be carried out under known alkoxyamine alkylation conditions, for example in accordance with the method described in Organic Letters, 2004, 6, 2361-2364.

The compound of the formula (V) and the compound of the formula (3) used in Reaction 2-4 may be produced in accordance with a known method, or may be commercially available.

The compound (I) is useful as an active ingredient of an agricultural and horticultural fungicide which can control harmful plant diseases at a low dose.

The compound (I) can control plant diseases caused by phytopathogen belonging to e.g. Oomycota, Endomyxa, Olpidiomycota, Ascomycota, Basidiomycota and Blastocladiomycota. It is particularly effective for controlling plant diseases caused by phytopathogen belonging to Oomycota, Endomyxa and Olpidiomycota.

As the above phytopathogen, the following may be mentioned.

Oomycota: phytopathogen belonging to Albuginales, Anisolpidiales, Lagenidiales, Leptomitales, Myzocytiopsidales, Olpidiopsidales, Peronosporales, Pythiales, Rhipidiales, Saprolegniales and Sclerosporales.
Endomyxa: phytopathogen belonging to Haplosporida, Paradiniida, Paramyxida, Gromiida, Phagomyxida, Plasmodiophorida and Vampyrellida.
Olpidiomycota: phytopathogen belonging to Olpidiales.
Ascomycota: phytopathogen belonging to Cladosporiales, Diaporthales, Erysiphales, Glomerellales, Helotiales, Hypocreales, Magnaporthales, Mycosphaerellales, Myriangiales, Pleosporales and Venturiales.
Basidiomycota: phytopathogen belonging to Agaricales, Cantharellales, Pucciniales and Ustilaginales.
Blastocladiomycota: phytopathogen belonging to Physodermatales.

As specific examples of the phytopathogen, the following may be mentioned.

Genus Phytophthora such as potato or tomato late blight (Phytophthora infestans), fig white powdery rot (Phytophthora palmivora), pear Phytophthora fruit rot or strawberry crown or root rot (Phytophthora cactorum), winter melon or pumpkin Phytophthora rot, sweet pepper or hot pepper Phytophthora blight (Phytophthora capsici), tomato gray late blight (Phytophthora capsici), eggplant or watermelon brown rot (Phytophthora capsici), citrus brown rot (Phytophthora citricola), adzuki bean stem rot (Phytophthora vignae f. sp. adzukicola), Edamame stem rot (Phytophthora megasperma var. sojae) and onion or allium chinense white tip (Phytophthora porri); genus Pseudoperonospora such as cucumber, pumpkin, melon or zucchini downy mildew (Pseudoperonospora cubensis) and hop downy mildew (Pseudoperonospora humuli); genus Plasmopara such as grape downy mildew (Plasmopara viticola) and Japanese honeywort downy mildew (Plasmopara nivea); genus Hyaloperonospora such as cabbage or Chinese cabbage downy mildew (Hyaloperonospora brassicae); genus Bremia such as lettuce downy mildew (Bremia lactucae); genus Pythium such as rice seedling blight (Pythium graminicola), wheat browning root rot (Pythium iwayamai), Chinese cabbage Pythium rot (Pythium aphanidermatum), Japanese ginger rhizome rot (Pythium zingiberis) and ginger rhizome rot (Pythium ultimum var. ultimum); genus Aphanomyces such as Japanese radish black-root (Aphanomyces raphanin) and spinach root rot (Aphanomyces cochlioides); genus Albugo such as spinach, turnip, Japanese radish or turnip rape white rust (Albugo macrospora), wasabi white rust (Albugo wasabiae), and water spinach white rust (Albugo ipomoeae-aquaticae); and genus Peronospora such as soybean downy mildew (Peronospora manshurica), broccoli or turnip downy mildew (Peronospora parasitica), leek, tree onion or onion downy mildew (Peronospora destructor), spinach downy mildew (Peronospora farinosa f. sp. Spinaciae) and basil downy mildew (Peronospora belbahrii).

Genus Plasmodiophora such as Chinese cabbage, cabbage, cauliflower, turnip rapa, broccoli or turnip clubroot (Plasmodiophora brassicae); genus Polymyxa such as Polymyxa betae transmitting sugar beet rhizomania (Beet necrotic yellow vein virus); genus Spongospora such as potato powdery scab (Spongospora subterranea); and genus Olpidium such as Olpidium virulentus transmitting lettuce big vein virus (Mirafiori lettuce biqvein virus).

Genus Erysiphe such as wheat powdery mildew (Erysiphe graminis); genus Setosphaeria such as corn leaf

blight (Setosphaeria turcica); genus Sphaerotheca such as cucumber powdery mildew (Sphaerotheca fuliginea), and strawberry powdery mildew (Sphaerotheca humuli); genus Uncinula such as grape powdery mildew (Uncinula necator); genus Podosphaera such as apple powdery mildew (Podosphaera leucotricha); genus Mycosphaerella such as pea Mycosphaerella blight (Mycosphaerella pinodes), apple black spot (Mycosphaerella pomi), banana black sigatoka (Mycosphaerella musicola), Japanese persimmon circular leaf spot (Mycosphaerella nawae), and strawberry leaf spot (Mycosphaerella fragariae); genus Venturia such as apple scab (Venturia inaequalis) and pear scab (Venturia nashicola); genus Pyrenophora such as barley net blotch (Pyrenophora teres) and barley stripe (Pyrenophora graminea); genus Sclerotinia such as kidney bean stem rot, cucumber, cabbage, Chinese cabbage, hot pepper or sweet pepper sclerotinia rot or onion watery soft rot (Sclerotinia sclerotiorum), wheat Sclerotinia snow blight (Sclerotinia borealis), tomato white mold (Sclerotinia minor), and alfalfa Sclerotinia root and crown rot (Sclerotinia trifoliorum).

Genus Botryotinia such as peanut small sclerotinia rot (Botrvotinia arachidis); genus Cochliobolus such as rice brown spot (Cochliobolus miyabeanus); genus Didymella such as cucumber gummy stem blight (Didymella bryoniae); genus Gibberella such as rice bakanae (Gibberella fujikuroi); genus Elsinoe such as grape anthracnose (Elsinoe ampelina) and citrus scab (Elsinoe fawcettii); genus Diaporthe such as citrus melanose (Diaporthe citri) and grape swelling arm (Diaporthe sp.); genus Monilinia such as apple Monilia leaf blight (Monilinia mali) and peach brown rot (Monilinia fructicola), and genus Glomerella such as grape ripe rot (Glomerella cingulata).

Genus Rhizoctonia such as rice sheath blight (Rhizoctonia solani); genus Ustilago such as wheat loose smut (Ustilago nuda); genus Puccinia such as oat crown rust (Puccinia coronata), wheat brown rust (Puccinia recondita), and wheat yellow rust (Puccinia striiformis); genus Phakopsora such as Asian soybean rust (Phakopsora pachyrhizi); and genus Typhula such as wheat or barley snow mold (Typhula incarnata and Typhula ishikariensis).

Genus Septoria such as wheat Septoria nodorum blotch (Septoria nodorum) and wheat Septoria tritici blotch (Septoria tritici); genus Botrytis such as grape, citrus, cucumber, tomato, strawberry, eggplant, kidney bean, adzuki bean, pea, peanut, hot pepper, sweet pepper, lettuce, onion, statice, carnation, pansy or sunflower gray mold or rose Botrytis blight (Botrytis cinerea), onion gray mold (Botrytis allii), and onion leaf blight or leaf spot (Botrytis squamosa, Botrytis byssoidea and Botrytis tulipae); genus Fusarium such as wheat head blight (Fusarium graminearum) and cucumber Fusarium wilt (Fusarium oxysporum); genus Pyricularia such as rice blast (Pyricularia oryzae); genus Cercospora such as sugar beet Cercospora leaf spot (Cercospora beticola), and Japanese persimmon Cercospora leaf spot (Cercospora kakivora); genus Colletotrichum such as cucumber anthracnose (Colletotrichum orbiculare), and coffee berry disease (Colletotrichum coffeanum); genus Alternaria such as apple Alternaria blotch (Alternaria alternata apple pathotype), pear black spot (Alternaria alternata Japanese pear pathotype), potato or tomato early blight (Alternaria solani), cabbage or Chinese cabbage Alternaria leaf spot (Alternaria brassicae), cabbage dark leaf spot (Alternaria brassicicola) and onion or leek purple blotch (Alternaria porri); genus Phoma such as cabbage black leg (Phoma lingam); genus Pseudocercosporella such as wheat eyespot (Pseudocercosporella herpotrichoides); genus Pseudocercospora such as grape isariopsis leaf spot (Pseudocercospora vitis); genus Rhynchosporium such as barley scald (Rhynchosporium secalis); genus Cladosporium such as peach scab (Cladosporium carpophilum); genus Phomopsis such as peach Phomopsis rot (Phomopsis sp.); genus Gloeosporium such as Japanese persimmon anthracnose (Gloeosporium kaki); genus Fulvia such as tomato leaf mold (Fulvia fulva); genus Corynespora such as cucumber target spot (Corynespora cassiicola); and genus Physoderma such as corn brown spot (Physoderma maydis).

The compound (I) can control the above various phytopathogens, and thus can control various diseases preventively or curatively. Particularly, the compound (I) is effective for controlling plant diseases which are problematic in agricultural and horticultural fields, including diseases of Gramineae crops, such as diseases of rice, such as damping-off caused by Pythium, blast caused by Pyricularia, Bakanae disease caused by Fusarium, brown spot caused by Cochliobolus, and sheath blight caused by Rhizoctonia; diseases of wheat and the like such as powdery mildew caused by Erysiphe, scab or crown rot caused by Fusarium, leaf rust caused by Puccinia, browning root rot caused by Pythium, loose smut caused by Ustilago, eyespot caused by Pseudocercosporella, and speckled leaf blotch or glume blotch caused by Septoria; diseases of corn such as Gibberella ear rot caused by Fusarium, brown spot caused by Physoderma, leaf rust caused by Puccinia, leaf blight caused by Setosphaeria, leaf spot caused by Cochliobolus, browning root rot caused by Pythium, and smut caused by Ustilago; and diseases of sugar cane such as smut caused by Ustilago, leaf scorch caused by Stagonospora, leaf rust caused by Puccinia, top rot caused by Gibberella, sooty mold caused by Caldariomyces, and leaf blight caused by Pseudocercosporella; diseases of Leguminosae crops, such as powdery mildew caused by Oidium, rust caused by Phakopsora, downy mildew caused by Peronospora, leaf blight or root and stem rot caused by Phytophthora, anthracnose caused by Colletotrichum, stem rot caused by Sclerotinia, gray mold caused by Botrytis, and root rot or foot rot caused by Fusarium; diseases of Brassicaceae crops, such as yellows caused by Fusarium, downy mildew caused by Peronospora or Hyaloperonospora, Alternaria leaf spot caused by Alternaria, root rot caused by Phoma, clubroot caused by Plasmodiophora, black root caused by Aphanomyces, and Pythium rot caused by Pythium; diseases of Asteraceae crops, such as downy mildew caused by Bremia, Phytophthora rot caused by Phytophthora, Botrytis blight caused by Botrytis, stem rot caused by Sclerotinia, and rust caused by Aecidium; diseases of Solanaceae crops, such as diseases of tomato such as early blight caused by Alternaria, leaf mold caused by Fulvia, late blight or Phytophthora blight

caused by Phytophthora, gray mold caused by Botrytis, powdery mildew caused by Oidium, Fusarium wilt caused by Fusarium, and Cercospora leaf mold caused by Pseudocercosporella; and diseases of potato such as early blight caused by Alternaria, late blight or tuber rot caused by Phytophthora, Sclerotial rot caused by Sclerotinia, and dry rot caused by Fusarium; diseases of Cucurbitaceae crops, such as anthracnose caused by Colletotrichum, powdery mildew caused by Sphaerotheca, gummy stem blight caused by Didymella, downy mildew caused by Pseudoperonospora, Phytophthora rot or brown rot caused by Phytophthora, Corynespora leaf spot caused by Corynespora, and Fusarium wilt caused by Fusarium; diseases of Amaryllidaceae allium crops, such as downy mildew caused by Peronospora, Phytophthora rot caused by Phytophthora, gray mold caused by Botrytis, neck rot caused by Sclerotinia, and rust caused by Puccinia; diseases of Umbelliferae crops, such as downy mildew caused by Plasmopara, leaf blight or Alternaria black rot caused by Alternaria, gray mold caused by Botrytis, Sclerotinia rot caused by Sclerotinia, powdery mildew caused by Erysiphe, and leaf spot caused by Cercospora; diseases of Liliaceae crops, such as Botrytis blight caused by Botrytis, Phytophthora blight caused by Phytophthora, and stem blight caused by Phomopsis; diseases of Polygonaceae crops, such as downy mildew caused by Peronospora, powdery mildew caused by Erysiphe, and damping-off caused by Rhizoctonia; diseases of Convolvulaceae crops, such as white rust caused by Albugo, stem rot caused by Fusarium, black rot caused by Ceratocystis, and soil rot caused by Streptomyces; diseases of Chenopodiaceae crops, such as root rot caused by Aphanomyces, white rust caused by Albugo, downy mildew caused by Peronospora, late blight caused by Phytophthora, gray mold caused by Botrytis, root rot caused by Sclerotinia, powdery mildew caused by Oidium, and brown spot caused by Cercospora; diseases of Vitaceae crops, such as anthracnose caused by Elsinoe, ripe rot caused by Colletotrichum, powdery mildew caused by Erysiphe, downy mildew caused by Plasmopara, gray mold caused by Botrytis, leaf blight caused by Pseudocercospora, and swelling arm caused by Diaporthe; diseases of Rosaceae crops, such as diseases of strawberry, such as powdery mildew caused by Sphaerotheca, gray mold caused by Botrytis, crown rot caused by Glomerella, and crown and root rot caused by Fusarium; diseases of apple, such as blossom blight caused by Monilinia, powdery mildew caused by Podosphaera, Alternaria blotch caused by Alternaria, scab caused by Venturia, bitter rot caused by Glomerella, blotch caused by Diplocarpon, ring rot caused by Botryosphaeria, fly speck caused by Zygophiala, sooty blotch caused by Gloeodes, and fruit spot caused by Mycosphaerella; diseases of pear, such as scab caused by Venturia, black spot caused by Alternaria, powdery mildew caused by Phyllactinia, Phytophthora fruit rot caused by Phytophthora, and Fusarium fruit rot caused by Fusarium; and diseases of peach, such as brown rot caused by Monilinia, scab caused by Cladosporium, and Phomopsis rot caused by Phomopsis; diseases of Rutaceae crops, such as diseases of citrus, such as melanose caused by Diaporthe, scab caused by Elsinoe, and Fusarium wilt caused by Fusarium; diseases of Ebenaceae crops, such as anthracnose caused by Gloeosporium, leaf spot caused by Cercospora, powdery mildew caused by Phyllactinia, and fly speck caused by Zygophiala; diseases of Theaceae crops, such as anthracnose caused by Colletotrichum, gray mold caused by Pestalotiopsis, bacterial shoot blight caused by Pseudomonas, and leaf and stem gall caused by Exobasidium; diseases of Zingiberaceae crops, such as root rot caused by Pythium; diseases of Cannabaceae crops, such as downy mildew caused by Pseudoperonospora; and diseases of Lamiaceae crops, such as downy mildew caused by Peronospora.

The compound (I) is effective also for seed borne diseases, including diseases of Gramineae crops, such as diseases of wheat and the like, such as scab or crown rot caused by Fusarium, anthracnose caused by Colletotrichum, bunt caused by Tilletia, loose smut caused by Ustilago, Cephalosporium stripe caused by Cephalosporium, and glume blotch caused by Septoria; diseases of corn, such as leaf spot caused by Bipolaris, anthracnose caused by Colletotrichum, and seedling blight caused by Fusarium; and diseases of sugar cane, such as red rot caused by Glomerella, pineapple disease caused by Ceratocysti, and downy mildew caused by Sclerospora; diseases of Leguminosae crops, such as diseases of soybean, such as purple stain caused by Cercospora, downy mildew caused by Peronospora, Fusarium blight caused by Fusarium, Septoria brown spot caused by Septoria, pod and stem blight caused by Diaporthe, anthracnose caused by Colletotrichum, and sleeping-blight caused by Septogloeum; diseases of Brassicaceae crops, such as diseases of cabbage, such as Alternaria leaf spot or Alternaria sooty spot caused by Alternaria, downy mildew caused by Peronospora, bacterial leaf spot caused by Pseudomonas, black root caused by Xanthomonas, and black leg caused by Phoma; diseases of Japanese radish, such as Alternaria leaf spot caused by Alternaria, yellows caused by Fusarium, and black rot caused by Xanthomonas; and diseases of Chinese cabbage, such as Alternaria leaf spot caused by Alternaria, black rot caused by Xanthomonas, and yellows caused by Verticillium; diseases of Solanaceae crops, such as diseases of tomato, such as early blight caused by Alternaria, bacterial canker caused by Clavibacter, and bacterial spot caused by Xanthomona; diseases of eggplant, such as early blight caused by Alternaria, and brown spot caused by Phomopsis; and diseases of potato, such as scab caused by Streptomyces, silver scurf caused by Helminthosporium, and powdery scab caused by Sponqospora; diseases of Cucurbitaceae crops, such as diseases of cucumber, such as leaf blight caused by Alternaria, bacterial spot caused by Pseudomonas, and bacterial brown spot caused by Xanthomonas; diseases of Amaryllidaceae allium crops, such as Alternaria leaf spot caused by Alternaria, gray-mold neck rot or mycelial rot caused by Botrytis, dry rot caused by Fusarium, downy mildew caused by Peronospora, and leaf blight or Phytophthora rot caused by Phytophthora; diseases of Umbelliferae crops, such as diseases of carrot, such as leaf blight or Alternaria black rot caused by Alternaria, and bacterial blight caused by Xanthomonas; and diseases of celery, such as late blight

caused by Septoria, stem rot caused by Sclerotinia, and bacterial leaf blight caused by Pseudomonas; and diseases of Chenopodiaceae crops, such as diseases of spinach, such as downy mildew caused by Peronospora, Fusarium wilt caused by Fusarium, and anthracnose caused by Colletotrichum.

The compound (I) is effective also for controlling soil diseases caused by phytopathogen such as Fusarium, Pythium, Rhizoctonia, Verticillium, Plasmodiophora, and Thielaviopsis.

The compound (I) can control the above various diseases preventively or curatively. By test methods disclosed in the following Examples, certain Invention Compounds exhibit excellent preventive or curative controlling effects at low dose (for example, 100 ppm, 25 ppm, 12.5 ppm, 6.3 ppm, 3.1 ppm, 1.6 ppm, 0.8 ppm, 0.4 ppm, 0.2 ppm or 0.1 ppm).

The compound (I) has excellent rainfastness, residual effects and penetration effects, and thus by applying the compound (I) to a plant body, it is possible to control harmful fungi on above-ground parts of the plant for a certain period.

For controlling various harmful plant diseases, an effective amount of the compound (I) may be applied to a plant body, phytopathogen or soil.

The an effective amount means an application amount of the compound (I) at which the compound (I) exhibits a control effect against various harmful plant diseases.

The plant body means above-ground parts such as trunks, stems, leaves, flowers, heads and fruits, and below-ground parts such as tubers, rhizomes and roots, and seeds of plant, seedlings, transplanted seedlings, etc.

The soil means a field where plants are grown, including e.g. agricultural fields such as crop plant fields, paddy fields and orchards, and non-agricultural fields such as lawn and forest land.

The plants to which the compound (I) is applied are not particularly limited so long as they are agriculturally or horticulturally useful plants, and their examples include Gramineae crops (such as rice, wheat, barley, oat, rye, corn and sugar cane), Leguminosae crops (such as soybean, kidney bean, adzuki bean, pea, peanut, Edamame and alfalfa), Brassicaceae crops (such as cabbage, Chinese cabbage, Japanese radish, turnip, broccoli, cauliflower, Turnip rape, rapeseed and horseradish), Asteraceae crops (such as lettuce, burdock, crown daisy and sunflower), Solanaceae crops (such as potato, eggplant, tomato, sweet pepper, tobacco and hot pepper), Cucurbitaceae crops (such as cucumber, pumpkin, melon, watermelon, winter melon and zucchini), Amaryllidaceae allium crops (such as leek, chinese chive, allium chinense, garlic, onion and shallot), Umbelliferae crops (such as celery, carrot, parsley and Japanese honeywort), Liliaceae crops (such as lily, tulip and asparagus), Polygonaceae crops (such as buckwheat), Convolvulaceae crops (such as sweet potato and water spinach), Chenopodiaceae crops (such as spinach and sugar beet), Vitaceae crops (such as grape), Rosaceae crops (such as rose, strawberry, apple, pear, peach, loquat and almond), Rutaceae crops (such as mandarin orange, lemon, orange and citrus), Ebenaceae crops (such as Japanese persimmon), Moraceae crops (such as fig), Theaceae crops (such as tea), Oleaceae crops (such as olive and jasmine), Malvaceae crops (such as cotton, cacao and okra), Musaceae crops (such as banana), Zingiberaceae crops (such as ginger and Japanese ginger), Cannabaceae crops (such as hop), Lamiaceae crops (such as basil), Rubiaceae crops (such as coffee plant), Bromeliaceae crops (such as pineapple and ananas), Plumbaginaceae crops (such as statice), Caryophyllaceae crops (such as carnation), and Violaceae crops (such as pansy).

The plants include plants generated by gene recombination or gene editing, for example, plants having imparted resistance to environmental stress, resistance to herbicides, resistance to pests and resistance to phytopathogen, and plants having growth, fertility, quality or yield of crops modified.

The compound (I) may be obtained usually by mixing the compound (I) with additives and applied in the form of various formulations such as dusts, granules, water dispersible granules, wettable powders, water-based suspensions, oil-based suspensions, water soluble powders, emulsifiable concentrates, liquid, pastes, aerosols, ultra low-volume formulations and microcapsules. It may be formed into any usual formulation used in this field, so long as the object of the present invention is thereby met. The additives used for formulation may be a solid carrier or a liquid carrier and as the case requires, a surfactant and other additives for formulation may be used.

Specific examples of the solid carrier include diatomaceous earth, slaked lime, calcium carbonate, talc, white carbon, kaoline, bentonite, kaolinite, sericite, clay, sodium carbonate, sodium bicarbonate, salt cake, zeolite, starch and pulverized silica.

Specific examples of the liquid carrier include water, toluene, xylene, solvent naphtha, dioxane, acetone, isophorone, methyl isobutyl ketone, chlorobenzene, cyclohexane, dimethyl sulfoxide, N,N-dimethylformamide, N,N-dimethylacetamide, N-methyl-2-pyrrolidone and an alcohol.

The surfactant may, for example, be an anionic surfactant or spreader such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether phosphate, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester or a salt of naphthalene sulfonate condensed with formaldehyde; or a nonionic surfactant or spreader such as a sorbitan

fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyethylene glycol, a polyoxyethylene fatty acid ester, a polyoxyethylene sorbitan fatty acid ester, a polyoxyethylene glycerin fatty acid ester, a polyoxyethylene hydrogenated castor oil or a polyoxypropylene fatty acid ester.

Specific examples of other additives for formulation include vegetable oils and mineral oils such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil and liquid paraffins; silicone; and xanthan gum.

These additive components may suitably be selected for use alone or in combination as a mixture of two or more of them, so long as the object of the present invention is met. Further, in addition to the above, various additives known in this technical field, such as a filler, a thickener, an anti-settling agent, an anti-freezing agent, a dispersion stabilizer, a safener and an anti-mold agent, may be used. The mixing ratio of the compound (I) to such various additives is usually 0.001:99.999 to 95:5, preferably 0.005:99.995 to 90:10, by weight ratio. In the actual application of such a formulation, it may be used as it is, or may be diluted to a predetermined concentration with a diluent such as water, and may be mixed with various spreaders (e.g. surfactants, vegetable oils or mineral oils), as the case requires.

The application of the compound (I) cannot generally be defined, as it varies depending upon the weather conditions, the type of the formulation, the plants to be treated, the application season, the application site, the type or degree of outbreak of harmful plant diseases, etc. However, an effective amount of the compound (I) may be applied by a conventional application method, that is spraying, soil treatment, seed treatment, etc. When an effective amount of the compound (I) is applied by a conventional application method, it is possible to apply the compound (I) at an active ingredient concentration of 0.1 to 10,000 ppm, preferably 1 to 2,000 ppm, more preferably 1 to 1,000 ppm.

In a conventional application method, a composition containing the compound (I) as an active ingredient (hereinafter sometimes referred to as the present composition) or a diluted product thereof may also be applied. In the case of spraying, the application amount is such that the amount of the present composition is about 10 g to 100,000 g, per hectare. In the case of soil treatment, the application amount is such that the amount of the present composition is about 0.01 g to 1,000 g, per hectare. In the case of seed treatment, the application amount is such that the amount of the present composition is about 0.001 g to 100 g, preferably about 0.01 g to 1 g per 1 kg of seeds.

The spraying is an application method of spraying an effective amount of the present composition to the surface of the trunk, bud, stem, leaf, flower, head or fruit of a plant, or to phytopathogen for controlling the phytopathogen. For example, foliar spraying, trunk spraying, etc. may be mentioned.

The soil treatment is to apply the present composition to soil so that an effective amount of the compound (I) penetrates into the plant body e.g. from the root of the plant, for protecting crops from being damaged by phytopathogen. The soil application includes, for example, irrigation application (the present composition is applied by irrigation to soil in which a plant is to be grown), soil mixing application (soil in which a plant is to be grown and the present composition are mixed), planting hole application, application to the plant foot or to soil between plant feet (by spraying or irrigation), and mixing with culture soil or soil to be used for a nursery box, a nursery tray, a paper pot or a seed bed.

The seed treatment is to apply the present composition to seeds, bulbs or the like of a crop plant directly or to the vicinity thereof, to control phytopathogen, for protecting crops from being damaged by phytopathogen. For example, coating, dusting or dipping treatment may be mentioned.

Further, seedling treatment (by irrigation or dipping), dipping treatment of bulbs, tubers, scaly bulbs, roots or the like in the present composition, and hydroponics in which the present composition is mixed with a hydroponics culture medium, may be mentioned. The application may be to the whole plant or to a part of a plant (e.g. stem, leaf, bud, flower, head, fruit, trunk, seed, bulb, tuber, scaly bulb or root).

The present composition may be mixed with or may be used in combination with other components selected from other agricultural and horticultural chemicals, fertilizers and safeners, whereby more excellent effects or activities may sometimes be obtained.

Mixing with or use in combination with other components means that the present composition and other components are used simultaneously, separately or with an interval.

Such other agricultural and horticultural chemicals include, for example, a herbicide, an insecticide, a miticide, a nematicide, a soil pesticide, a fungicide, an antivirus agent, an attractant, an antibiotic, a plant hormone, a plant growth regulator, etc. Especially, a mixed fungicidal composition having the present composition mixed with or used in combination with one or more active ingredient compounds of other fungicide, may improve the application range, the application time, the controlling activities, etc. to preferred directions. The present composition and the active ingredient compounds of other fungicide may separately be formulated so that they are mixed for use at the time of application, or they may be formulated together. The present invention includes such a mixed fungicidal composition.

The mixing ratio of the compound (I) to the active ingredient compounds of other fungicide can not generally be defined as it varies depending upon the weather conditions, the types of formulations, the crop plants to be treated, the application time, the application site, the types or degree of outbreak of harmful plant diseases, etc., but it is usually within a

range of 1:300 to 300:1, preferably 1:100 to 100:1, by weight ratio. Further, the dose for the application is such that the total amount of the active ingredient compounds is 0.1 to 70,000 g, preferably 1 to 30,000 g, per hectare. The present invention includes a method for controlling harmful plant diseases by application of such a mixed fungicidal composition.

When the present composition is applied, it may be used in combination with other agricultural and horticultural chemicals, such as a fungicide, an insecticide, a miticide, a nematicide, a soil insect pesticide, an antivirus agent, an attractant, a herbicide and a plant growth regulator.

The active ingredient compounds of a fungicide in the above-mentioned other agricultural and horticultural chemicals may properly be selected, for example, from the following group of compounds (by common names). In a case where these compounds have their salts, alkyl esters, various structural isomers such as optical isomers, etc., all of them are included, even if no specific disclosure thereof is made.

Anilinopyrimidine compounds such as mepanipyrim, pyrimethanil and cyprodinil;

triazolopyrimidine compounds such as ametoctradin;
triazolobenzothiazole compounds such as tricyclazole;
pyridinamine compounds such as fluazinam;
azole compounds such as triadimefon, bitertanol, triflumizole, etaconazole, propiconazole, penconazole, flusilazole, myclobutanil, cyproconazole, tebuconazole, hexaconazole, furconazole-cis, prochloraz, metconazole, epoxiconazole, tetraconazole, oxpoconazole fumarate, prothioconazole, triadimenol, flutriafol, difenoconazole, fluquinconazole, fenbuconazole, bromuconazole, diniconazole, simeconazole, pefurazoate, ipconazole, imibenconazole, azaconazole, triticonazole, imazalil, ipfentrifluconazole and mefentrifluconazole;
quinoxaline compounds such as chinomethionat;
dithiocarbamate compounds such as maneb, zineb, mancozeb, polycarbamate, metiram, propineb and thiram;
organic chlorine compounds such as phthalide, chlorothalonil and quintozene;
imidazole compounds such as benomyl, thiophanate-methyl, carbendazim, thiabendazole and fuberiazole;
cyanoacetamide compounds such as cymoxanil;
acylamino acid compounds such as metalaxyl, metalaxyl-M (another name: mefenoxam), oxadixyl, ofurace, benalaxyl, benalaxyl-M (another name: kiralaxyl, chiralaxyl), furalaxyl and valifenalate;
anilide compounds such as cyprofuram, carboxin, oxycarboxin, thifluzamide, boscalid, fenhexamid, isotianil, tiadinil and pyraziflumid;
sulfamide compounds such as dichlofluanid;
copper compounds such as cupric hydroxide, oxine copper, anhydrous copper sulfate, copper nonylphenolsulfonate, copper 8-hydroxyquinoline and dodecylbenzenesulfonic acid bisethylenediamine copper(II) complex salt (another name: DBEDC);
organophosphorus compounds such as fosetyl-Al, tolclofos-Methyl, edifenphos, and iprobenfos;
phthalimide compounds such as captan, captafol and folpet;
dicarboxyimide compounds such as procymidone, iprodione and vinclozolin;
benzanilide compounds such as flutolanil, mepronil, benodanil and flufenoxadiazam;
amide compounds such as carpropamid, diclocymet, silthiofam and fenoxanil;
pyrazole carboxamide compounds such as benzovindiflupyr, bixafen, fluindapyr, fluxapyroxad, furametpyr, isopyrazam, penflufen, penthiopyrad, pydiflumetofen, sedaxane, isoflucypram, inpyrfluxam, pyrapropoyne and fenopyramid;
benzamide compounds such as fluopicolide, fluopyram, zoxamide and fluopimomide;
flanilide compounds such as fenfuram;
thiophene amide compounds such as isofetamide;
piperazine compounds such as triforine;
pyridine compounds such as pyrifenox, pyrisoxazole and aminopyrifen;
pyrimidine compounds such as fenarimol, ferimzone, nuarimol and flumetylsulforim;
piperidine compounds such as fenpropidin;
morpholine compounds such as fenpropimorph and tridemorph;
organotin compounds such as fentin hydroxide and fentin acetate;
urea compounds such as pencycuron;
carboxylic acid amide compounds such as dimethomorph, flumorph, pyrimorph, iprovalicarb, benthiavalicarb-isopropyl and mandipropamid;
phenyl carbamate compounds such as diethofencarb;
cyanopyrrole compounds such as fludioxonil and fenpiclonil;
strobilurin compounds such as azoxystrobin, kresoxim-methyl, metominostrobin, trifloxystrobin, picoxystrobin, oryzastrobin, dimoxystrobin, pyraclostrobin, fluoxastrobin, Pyraoxystrobin, Pyrametostrobin, coumoxystrobin, enoxastrobin, fenaminstrobin, flufenoxystrobin, triclopyricarb, mandestrobin;

oxazole compounds such as famoxadone and oxathiapiprolin;
thiazolecarboxamide compounds such as ethaboxam;
imidazolinone compounds such as fenamidone;
benzenesulfonamide compounds such as flusulfamide;
oxime ether compounds such as cyflufenamid;
anthraquinone compounds such as dithianon;
crotonic acid compounds such as meptyldinocap;
antibiotics such as validamycin, kasugamycin, streptomycin and polyoxins;
guanidine compounds such as iminoctadine, dodine and guazatine;
aliphatic nitrogen compounds such as butylamine and seboctylamine;
quinoline compounds such as tebufloquin, quinoxyfen, quinofumelin, ipflufenoquin and feneptamidoquin;
thiazolidine compounds such as flutianil;
carbamate compounds such as propamocarb hydrochloride, pyribencarb and tolprocarb;
tetrazole compounds such as picarbutrazox and metyltetraprole;
sulfonamide compounds such as amisulbrom and cyazofamid;
allyl phenyl ketone compounds such as metrafenone and pyriofenone;
benzothiazole compounds such as probenazole and dichlobentiazox;
phenylpyrazole compounds such as fenpyrazamine;
dithiolane compounds such as isoprothiolane;
picolinamide compounds such as fenpicoxamid and florylpicoxamid;
sulfur compounds such as sulfur and lime sulfur;
other compounds such as pyroquilon, diclomezine, chloropicrin, dazomet, metam-sodium, proquinazid, spiroxamine and dipymetitrone;
microbial fungicides such as Bacillus amyloliqefaciens strain QST713, Bacillus amyloliqefaciens strain FZB24, Bacillus amyloliqefaciens strain MBI600, Bacillus amyloliqefaciens strain D747, Pseudomonas fluorescens, Bacillus subtilis and Trichoderma atroviride SKT-1; and
plant extracts such as tea tree oil.

The active ingredient compounds of an insecticide, a nematicide, a miticide or a soil pesticide, in the above-mentioned other agricultural and horticultural chemicals may properly be selected, for example, from the following group of compounds (by common names). In a case where these compounds have their salts, alkyl esters, various structural isomers such as optical isomers, etc., all of them are included, even if no specific disclosure thereof is made.

Organic phosphoric acid ester compounds such as profenofos, dichlorvos, fenamiphos, fenitrothion, EPN ((RS)-(O-ethyl O-4-nitrophenyl phenylphosphonothioate)), diazinon, chlorpyrifos, chlorpyrifos-methyl, acephate, prothiofos, fosthiazate, cadusafos, disulfoton, isoxathion, isofenphos, ethion, etrimfos, quinalphos, dimethylvinphos, dimethoate, sulprofos, thiometon, vamidothion, pyraclofos, pyridaphenthion, pirimiphos-methyl, propaphos, phosalone, formothion, malathion, tetrachlorvinphos, chlorfenvinphos, cyanophos, trichlorfon, methidathion, phenthoate, oxydeprofos, (another name ESP), azinphos-methyl, fenthion, heptenophos, parathion, phosphocarb, demeton-S-methyl, monocrotophos, methamidophos, imicyafos, parathion-methyl, terbufos, phosphamidon, phosmet and phorate;

carbamate compounds such as carbaryl, propoxur, aldicarb, carbofuran, thiodicarb, methomyl, oxamyl, ethiofencarb, pirimicarb, fenobucarb, carbosulfan, benfuracarb, bendiocarb, furathiocarb, isoprocarb, metolcarb, xylylcarb, XMC (3,5-xylyl methylcarbamate) and fenothiocarb;
nereistoxin derivatives such as cartap, thiocyclam, thiocyclam oxalate, thiocyclam hydrochloride, bensultap, thiosultap, monosultap (another name thiosultap-monosodium), bisultap (another name thiosultap-disodium) and polythialan;
organic chlorine compounds such as dicofol, tetradifon, endosulfan, dienochlor, dieldrin and methoxychlor;
organic metal compounds such as fenbutatin oxide and cyhexatin;
pyrethroid compounds such as fenvalerate, permethrin, cypermethrin, alpha-cypermethrin, zeta-cypermethrin, theta-cypermethrin, beta-cypermethrin, deltamethrin, cyhalothrin, gamma-cyhalothrin, lambda-cyhalothrin, tefluthrin, kappa-tefluthrin, ethofenprox, flufenprox, cyfluthrin, beta-cyfluthrin, fenpropathrin, flucythrinate, fluvalinate, cycloprothrin, pyrethrins, esfenvalerate, tetramethrin, resmethrin, protrifenbute, bifenthrin, kappa-bifenthrin, acrinathrin, allethrin, tau-fluvalinate, tralomethrin, profluthrin, metofluthrin, epsilon-metofluthrin, heptafluthrin, phenothrin, flumethrin, momfluorothrin, epsilon-momfluorothrin, silafluofen and chloroprallethrin;
benzoylurea compounds such as diflubenzuron, chlorfluazuron, teflubenzuron, flufenoxuron, lufenuron, novaluron, triflumuron, hexaflumuron, bistrifluron, noviflumuron, fluazuron and flufenoxuron;
juvenile hormone-like compounds such as methoprene, pyriproxyfen, fenoxycarb and diofenolan;
pyridazinone compounds such as pyridaben;

pyrazole compounds such as fenpyroximate, fipronil, ethiprole, acetoprole, pyrafluprole, pyriprole, cyenopyrafen and flufiprole;
pyrazole carboxamide compounds such as pyflubumide, tebufenpyrad, tolfenpyrad and dimpropyridaz;
pyridylpyrazole compounds such as chlorantraniliprole, cyantraniliprole, cyclaniliprole, tetraniliprole, tyclopyrazoflor, fluchlordiniliprole and tiorantraniliprole;
neonicotinoid compounds such as imidacloprid, nitenpyram, acetamiprid, thiacloprid, thiamethoxam, clothianidin, nidinotefuran, dinotefuran and nithiazine;
hydrazine compounds such as tebufenozide, methoxyfenozide, chromafenozide and halofenozide;
pyridine compounds such as pyridalyl and flonicamid;
tetronic acid compounds such as spirodiclofen, spiromesifen and spirobudifen;
tetramic acid compounds such as spirotetramat and spiropidion;
strobilurin compounds such as fluacrypyrim, pyriminostrobin and flupyroxystrobin;
pyrimidinamine compounds such as flufenerim and pyrimidifen;
organic sulfur compounds such as malathion;
triazine compounds such as cyromazine;
hydrazone compounds such as hydramethylnon;
diamide compounds such as flubendiamide, broflanilide, cyhalodiamide, pioxaniliprole and piperflanilide;
thiourea compounds such as diafenthiuron and chloromethiuron;
formamidine compounds such as amitraz, chlordimeform and chloromebuform;
pyridine azomethine compounds such as pymetrozine and pyrifluquinazone;
isoxazolinone compounds such as afoxolaner, fluralaner, fluxametamide, sarolaner and isoflualanam;
other compounds such as buprofezin, hexythiazox, triazamate, chlorfenapyr, indoxacarb, acequinocyl, etoxazole, 1,3-dichloropropene, benclothiaz, bifenazate, propargite, clofentezine, metaflumizone, cyflumetofen, fenazaquin, amidoflumet, sulfluramid, hydramethylnon, metaldehyde, sulfoxaflor, fluensulfone, verbutin, dicloromezotiaz, triflumezopyrim, fluhexafon, tioxazafen, afidopyropen, flometoquin, flupyradifurone, fluazaindolizine, acynonapyr, benzpyrimoxan, flupyrimin, oxazosulfyl, sulfiflumin, bisulflufen, cybenzoxasulfyl, galquin, vedescana, tiapyrachlor and bentioflumin.

Further, the present composition may be mixed with or used in combination with the following compounds.
Microbial agricultural chemicals, such as insecticidal crystal proteins produced by Bacillus thuringiensis such as Bacillus thuringiensis aizawai, Bacillus thuringiensis kurstaki, Bacillus thuringiensis israelensis, Bacillus thuringiensis japonensis, Bacillus thuringiensis tenebrionis, insect viruses, entomopathogenic fungi, and nematophagous fungi:

antibiotics or semisynthetic antibiotics, such as abermectin, emamectin benzoate, ivermectin, milbemectin, milbemycin oxime, lepimectin, spinosad and spinetoram;
natural products, such as azadirachtin, rotenone, and ryanodine;
repellents, such as deet;
physical preservatives such as a paraffin oil and a mineral oil; and
RNAi pesticides such as ledprona and vadescana.

Preferred embodiments of the present invention will be described below. It should be understood that the present invention is by no means restricted thereto.

[1] A compound represented by the formula (I) or a salt thereof.
[2] The compound or a salt thereof according to [1], wherein the compound of the formula (I) is a N-(cyclo)alkoxyamide compound of the formula (IA) or a N-alkynyloxyamide compound of the formula (IB).
[3] The compound or a salt thereof according to [1], wherein the compound of the formula (I) is a N-(cyclo)alkoxyamide compound of the formula (IA)
[4] The compound or a salt thereof according to [1], wherein the compound of the formula (I) is a N-alkynyloxyamide compound of formula (IB)
[5] The compound or a salt thereof according to [1], wherein $X^1$ is a fluorine atom, a chlorine atom or a bromine atom.
[6] The compound or a salt thereof according to [1], wherein $X^1$ is a fluorine atom or a chlorine atom.
[7] The compound or a salt thereof according to [1], wherein $X^1$ is a fluorine atom or a bromine atom.
[8] The compound or a salt thereof according to [1], wherein $X^1$ is a chlorine atom or a bromine atom.
[9] The compound or a salt thereof according to [1], wherein $X^1$ is a fluorine atom.
[10] The compound or a salt thereof according to [1], wherein $X^1$ is a chlorine atom.
[11] The compound or a salt thereof according to [1], wherein $X^1$ is a bromine atom.
[12] The compound or a salt thereof according to any one of [1] and [5] to [11], wherein R' is a $(C_1-C_4)$-alkyl,

$(C_3-C_4)$-cycloalkyl, $(C_3-C_5)$-alkenyl or $(C_3-C_5)$-alkynyl, which may be substituted with $T^1$.

[13] The compound or a salt thereof according to any one of [1] and [5] to [11], wherein R' is a $(C_1-C_4)$-alkyl, a $(C_3-C_4)$-cycloalkyl or a $(C_3-C_5)$-alkynyl.

[14] The compound or a salt thereof according to any one of [1] and [5] to [11], wherein R' is methyl, ethyl, n-propyl, isopropyl, tert-butyl, cyclopropyl, cyclobutyl, propargyl, allyl, methylthiomethyl, cyanomethyl, 2-methoxyethyl, 2,2-difluoroethyl or methoxycarbonylmethyl.

[15] The compound or a salt thereof according to any one of [1] and [5] to [11], wherein R' is methyl, ethyl, cyclopropyl or propargyl.

[16] The compound or a salt thereof according to any one of [1] and [5] to [11], wherein $R^1$ is methyl, ethyl or cyclopropyl.

[17] The compound or a salt thereof according to any one of [1] and [5] to [11], wherein $R^1$ is methyl, ethyl or propargyl.

[18] The compound or a salt thereof according to any one of [1] and [5] to [11], wherein $R^1$ is methyl or ethyl.

[19] The compound or a salt thereof according to any one of [1] and [5] to [11], wherein $R^1$ is methyl or cyclopropyl.

[20] The compound or a salt thereof according to any one of [1] and [5] to [11], wherein $R^1$ is methyl or propargyl.

[21] The compound or a salt thereof according to any one of [1] and [5] to [11], wherein $R^1$ is methyl.

[22] The compound or a salt thereof according to any one of [1] and [5] to [11], wherein $R^1$ is ethyl.

[23] The compound or a salt thereof according to any one of [1] and [5] to [11], wherein $R^1$ is cyclopropyl.

[24] The compound or a salt thereof according to any one of [1] and [5] to [11], wherein $R^1$ is propargyl.

[25] The compound or a salt thereof according to any one of [1] and [5] to [24], wherein $Y^1$ is a hydrogen atom, a halogen, a $(C_1-C_6)$-alkyl, a $(C_1-C_6)$-haloalkyl, nitro or cyano.

[26] The compound or a salt thereof according to any one of [1] and [5] to [24], wherein $Y^1$ is a halogen, a $(C_1-C_3)$-alkyl, a $(C_1-C_3)$-haloalkyl, nitro or cyano.

[27] The compound or a salt thereof according to any one of [1] and [5] to [24], wherein $Y^1$ is a hydrogen atom, a fluorine atom, a chlorine atom, a bromine atom, methyl, trifluoromethyl, difluoromethyl, nitro or cyano.

[28] The compound or a salt thereof according to any one of [1] and [5] to [24], wherein $Y^1$ is a fluorine atom, a chlorine atom, a bromine atom, methyl, trifluoromethyl, difluoromethyl, nitro or cyano.

[29] The compound or a salt thereof according to any one of [1] and [5] to [24], wherein $Y^1$ is a fluorine atom, a chlorine atom, a bromine atom or methyl.

[30] The compound or a salt thereof according to any one of [1] and [5] to [24], wherein $Y^1$ is a fluorine atom.

[31] The compound or a salt thereof according to any one of [1] and [5] to [24], wherein $Y^1$ is a chlorine atom.

[32] The compound or a salt thereof according to any one of [1] and [5] to [24], wherein $Y^1$ is a bromine atom.

[33] The compound or a salt thereof according to any one of [1] and [5] to [24], wherein $Y^1$ is methyl.

[34] The compound or a salt thereof according to any one of [1] and [5] to [33], wherein $Y^2$ is a halogen, a $(C_1-C_6)$-alkyl or a hydrogen atom.

[35] The compound or a salt thereof according to any one of [1] and [5] to [33], wherein $Y^2$ is a fluorine atom, a chlorine atom, a $(C_1-C_3)$-alkyl or a hydrogen atom.

[36] The compound or a salt thereof according to any one of [1] and [5] to [33], wherein $Y^2$ is a fluorine atom, a chlorine atom, methyl or a hydrogen atom.

[37] The compound or a salt thereof according to any one of [1] and [5] to [33], wherein $Y^2$ is a fluorine atom or a hydrogen atom.

[38] The compound or a salt thereof according to any one of [1] and [5] to [37], wherein $Y^3$ is a halogen, a $(C_1-C_6)$-alkyl or a hydrogen atom.

[39] The compound or a salt thereof according to any one of [1] and [5] to [37], wherein $Y^3$ is a fluorine atom, a chlorine atom, a $(C_1-C_3)$-alkyl or a hydrogen atom.

[40] The compound or a salt thereof according to any one of [1] and [5] to [37], wherein $Y^3$ is a fluorine atom, a chlorine atom, methyl or a hydrogen atom.

[41] The compound or a salt thereof according to any one of [1] and [5] to [37], wherein $Y^3$ is a fluorine atom or a hydrogen atom.

[42] The compound or a salt thereof according to any one of [1] and [5] to [41], wherein $Y^4$ is a halogen or a hydrogen atom.

[43] The compound or a salt thereof according to any one of [1] and [5] to [41], wherein $Y^4$ is a fluorine atom, a chlorine atom or a hydrogen atom.

[44] The compound or a salt thereof according to any one of [1] and [5] to [41], wherein $Y^4$ is a fluorine atom or a hydrogen atom.

[45] The compound or a salt thereof according to any one of [1] and [5] to [44], wherein $Y^5$ is a halogen or a hydrogen atom.

[46] The compound or a salt thereof according to any one of [1] and [5] to [44], wherein $Y^5$ is a fluorine atom, a chlorine atom or a hydrogen atom.

[47] The compound or a salt thereof according to any one of [1] and [5] to [44], wherein $Y^5$ is a fluorine atom or a hydrogen atom.
[48] The compound or a salt thereof according to any one of [1] and [5] to [44], wherein $Y^5$ is a hydrogen atom.
[49] A N-(cyclo)alkoxyamide compound represented by the formula (IA) or a salt thereof.
[50] The N-(cyclo)alkoxyamide compound or a salt thereof according to [49], wherein $Y^1$ is a halogen, a $(C_1\text{-}C_6)$-alkyl, a $(C_1\text{-}C_6)$-haloalkyl, nitro or cyano.
[51] The N-(cyclo)alkoxyamide compound or a salt thereof according to [49], wherein $Y^1$ is a hydrogen atom, a fluorine atom, a chlorine atom, a bromine atom, methyl, trifluoromethyl, difluoromethyl, nitro or cyano.
[52] The N-(cyclo)alkoxyamide compound or a salt thereof according to [49], wherein $Y^1$ is a fluorine atom, a chlorine atom, a bromine atom, methyl, trifluoromethyl, difluoromethyl, nitro or cyano.
[53] The N-(cyclo)alkoxyamide compound or a salt thereof according to any one of [49] to [52], wherein $Y^{2A}$ is a hydrogen atom, a fluorine atom or a chlorine atom.
[54] The N-(cyclo)alkoxyamide compound or a salt thereof according to any one of [49] to [52], wherein $Y^{2A}$ is a hydrogen atom or a fluorine atom.
[55] The N-(cyclo)alkoxyamide compound or a salt thereof according to any one of [49] to [54], wherein $Y^{3A}$ is a hydrogen atom, a fluorine atom or a chlorine atom.
[56] The N-(cyclo)alkoxyamide compound or a salt thereof according to any one of [49] to [54], wherein $Y^{3A}$ is a hydrogen atom or a fluorine atom.
[57] The N-(cyclo)alkoxyamide compound or a salt thereof according to any one of [49] to [56], wherein $Y^{4A}$ is a hydrogen atom or a fluorine atom.
[58] The N-(cyclo)alkoxyamide compound or a salt thereof according to any one of [49] to [57], wherein $R^{1A}$ is methyl, ethyl, n-propyl, isopropyl, tert-butyl, cyclopropyl or cyclobutyl.
[59] The N-(cyclo)alkoxyamide compound or a salt thereof according to any one of [49] to [57], wherein $R^{1A}$ is methyl, ethyl or cyclopropyl.
[60] A N-alkynyloxyamide compound represented by the formula (IB) or a salt thereof.
[61] The N-alkynyloxyamide compound or a salt thereof according to [60], wherein $Y^5$ is a hydrogen atom.
[62] The N-alkynyloxyamide compound or a salt thereof according to [60] or [61], wherein $Y^1$ is a halogen, a $(C_1\text{-}C_6)$-alkyl, a $(C_1\text{-}C_6)$-haloalkyl, nitro or cyano.
[63] The N-alkynyloxyamide compound or a salt thereof according to [60] or [61], wherein $Y^1$ is a fluorine atom, a chlorine atom, a bromine atom, methyl, difluoromethyl, nitro or cyano.
[64] The N-alkynyloxyamide compound or a salt thereof according to any one of [60] to [63], wherein $Y^2$ is a hydrogen atom or halogen.
[65] The N-alkynyloxyamide compound or a salt thereof according to any one of [60] to [63], wherein $Y^2$ is a hydrogen atom or a fluorine atom.
[66] The N-alkynyloxyamide compound or a salt thereof according to any one of [60] to [65], wherein $Y^3$ is a hydrogen atom or halogen.
[67] The N-alkynyloxyamide compound or a salt thereof according to any one of [60] to [65], wherein $Y^3$ is a hydrogen atom or a fluorine atom.
[68] The N-alkynyloxyamide compound or a salt thereof according to any one of [60] to [67], wherein $Y^4$ is a hydrogen atom or halogen.
[69] The N-alkynyloxyamide compound or a salt thereof according to any one of [60] to [67], wherein $Y^4$ is a hydrogen atom or a fluorine atom.
[70] The N-alkynyloxyamide compound or a salt thereof according to any one of [60] to [69], wherein $X^1$ is a fluorine atom, a chlorine atom or a bromine atom.
[71] An agricultural and horticultural fungicide, which comprises as an active ingredient the compound or a salt thereof as defined in any one of [1] to [70].
[72] A method for controlling harmful plant diseases, which comprises applying an effective amount of the compound or a salt thereof as defined in any one of [1] to [70] to a plant body, phytopathogen or soil.

EXAMPLE 1

Now, examples of the present invention will be described, however, the present invention is by no means restricted thereto. The melting point as a physical property of the Invention Compound was measured by Buchi M-565. The $^1$H-NMR spectrum data was measured in a measurement solvent by JEOL JNM-ECX (500 MHz) or Bruker AVANCE III HD (300 MHz) (1H-nuclear magnetic resonance spectroscopy). The measurement solvent includes tetramethylsilane (TMS) as an internal standard in some cases.
In this specification, room temperature means about 10 to 30°C.

[Synthesis Examples]

Synthesis Example 1: Synthesis of

2-amino-4-bromo-N-(2-chlorobenzyl)-N-methoxythiazole-5-carboxamide (Compound No. A-94)

(1) 2-chlorobenzyl bromide (543 mg) and O-methylhydroxylamine hydrochloride (662 mg) were mixed with N,N-dimethylformamide (10 mL), and to the obtained mixture, diisopropylethylamine (2.31 mL) was added at room temperature, followed by stirring at 50°C for 5 hours. After cooling to room temperature, the reaction solution was quenched with water. To the quenched reaction solution, ethyl acetate and heptane were sequentially added, and the aqueous layer was extracted with ethyl acetate/heptane mixture. The organic layer obtained by extraction was sequentially washed with water and brine, dried over anhydrous sodium sulfate, subjected to filtration and concentrated under reduced pressure. The obtained residue was purified by column chromatography (eluent: ethyl acetate/heptane) to obtain oily N-(2-chlorobenzyl)-O-methylhydroxylamine (266 mg). Its $^1$H-NMR spectrum data was as follows.

$^1$H NMR ($CDCl_3$/300 MHz):$\delta$ (ppm)= 7.42-7.36 (m, 2H), 7.27-7.22 (m, 2H), 5.91 (brs, 1H), 4.18 (s, 2H), 3.54 (s, 3H).

(2) To a solution of tert-butyl(4-bromo-5-formylthiazol-2-yl) carbamate (9.7 g) synthesized in accordance with Example 18, Step F in WO2012/008999 at page 56, in tert-butanol (310 mL), aqueous sodium dihydrogen phosphate (11.3 g) solution (20 mL) was added at room temperature, followed by stirring to obtain a mixture. 2-Methyl-2-butene (43 mL) was added to the obtained mixture. 15 Minutes later, aqueous sodium chlorite (21.4 g, 80%) solution (50 mL) was added, followed by stirring for 3.5 hours. Hydrochloric acid was added to acidify the reaction solution, which was extracted with ethyl acetate. The extracted organic layer was sequentially washed with water and brine, dried over anhydrous sodium sulfate, subjected to filtration, and concentrated under reduced pressure. The obtained solid was dissolved in saturated aqueous sodium hydrogen carbonate solution, and the aqueous solution was washed with ethyl acetate. Hydrochloric acid was added to the aqueous layer washed with ethyl acetate to acidify the aqueous layer. Ethyl acetate was added to the acidified aqueous layer and the aqueous layer was extracted with ethyl acetate. The organic layer obtained by extraction was sequentially washed with water and brine, dried over anhydrous sodium sulfate, subjected to filtration, and concentrated under reduced pressure to obtain white solid 2-[(tert-butoxycarbonyl) amino]-4-bromothiazole-5-carboxylic acid (9.2 g). Its $^1$H-NMR spectrum data was as follows.

$^1$H NMR (DMSO-$d_6$/300 MHz):$\delta$ (ppm)= 13.36 (brs, 1H), 12.18 (s, 1H), 1.49 (s, 9H).

(3) To a solution of 2-[(tert-butoxycarbonyl)amino]-4-bromothiazole-5-carboxylic acid (339 mg) in tetrahydrofuran (10 mL), under cooling with ice, oxalyl chloride (166 mg) and a catalytic amount of N,N-dimethylformamide were added, followed by stirring in a nitrogen atmosphere. 10 minutes later, the mixture was warmed to room temperature and stirred further for 40 minutes. The reaction solution was concentrated under reduced pressure to obtain 2-[(tert-butoxycarbonyl)amino]-4-bromothiazole-5-carboxylic acid chloride. Tetrahydrofuran (5 mL) was added to the obtained acid chloride to dissolve, and then a solution of N-(2-chlorobenzyl)-O-methylhydroxylamine (150 mg) in tetrahydrofuran (5 mL) and diisopropylethylamine (0.31 mL) were sequentially added at room temperature, followed by stirring at 50°C for 1 hour and 45 minutes. After cooling to room temperature, the reaction solution was quenched with saturated aqueous sodium hydrogen carbonate solution. To the quenched reaction solution, ethyl acetate was added, and the aqueous layer was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain amorphous tert-butyl{4-bromo-5-[(2-chlorobenzyl)(methoxy)carbamoyl]thiazol-2-yl} carbamate (376 mg). Its $^1$H-NMR spectrum data was as follows.

$^1$H NMR (DMSO-$d_6$/300 MHz):$\delta$ (ppm)= 12.12 (brs, 1H), 7.51-7.49 (m, 1H), 7.37-7.35 (m, 3H), 5.02 (s, 2H), 3.67 (s, 3H), 1.48 (s, 9H).

(4) To a solution of tert-butyl{4-bromo-5-[(2-chlorobenzyl)(methoxy)carbamoyl] thiazol-2-yl} carbamate (376 mg) in dichloromethane (6 mL), trifluoroacetic acid (1.2 mL) was added, followed by stirring overnight under reflux with heating. After cooling to room temperature, saturated aqueous sodium hydrogen carbonate solution was slowly added to the reaction solution to basify the reaction solution. The aqueous layer of the basified reaction solution was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain solid desired product (208 mg). Its $^1$H-NMR spectrum data and melting point were as follows.

$^1$H NMR (DMSO-$d_6$/300 MHz):$\delta$ (ppm)= 7.96 (brs, 2H), 7.49-7.46 (m, 1H), 7.36-7.29 (m, 3H), 4.96 (s, 2H), 3.65 (s,

3H).
Melting point: 184.8°C

Synthesis Example 2: Synthesis of

2-amino-4-bromo-N-(4-fluoro-2-methylbenzyl)-N-methoxythiazole-5-carboxamide (Compound No. A-105)

(1) To a solution of O-methylhydroxylamine hydrochloride (302 mg) in methanol (4 mL), triethylamine (0.5 mL) was added at 0°C, followed by stirring to obtain a mixture. To the obtained mixture, 4-fluoro-2-methylbenzaldehyde (500 mg) in methanol (6 mL) and acetic acid (1 mL) were sequentially added, and 30 minutes later, the mixture was warmed to room temperature. 3.5 Hours later, 2-picoline borane (774 mg) was added at 0°C, and 15 minutes later, 3N aqueous hydrochloric acid solution (6 mL) was added, followed by stirring for 2.5 hours to obtain a reaction solution. Saturated aqueous sodium hydrogen carbonate solution was added to the reaction solution to basify the reaction solution, and methanol was distilled off. The residue was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain oily N-(4-fluoro-2-methylbenzyl)-O-methylhydroxylamine(392 mg). Its $^1$H-NMR spectrum data was as follows.
$^1$H NMR ($CDCl_3$/300 MHz):$\delta$ (ppm)= 7.26-7.21 (m, 1H), 6.90-6.82 (m, 2H), 5.59 (brs, 1H), 4.03 (s, 2H), 2.35 (s, 3H), 2.39 (s, 3H).
(2) To a solution of 2-[(tert-butoxycarbonyl)amino]-4-bromothiazole-5-carboxylic acid (339 mg) in tetrahydrofuran (10 mL), under cooling with ice, oxalyl chloride (0.11 mL) and a catalytic amount of N,N-dimethylformamide were added, followed by stirring in a nitrogen atmosphere. 10 minutes later, the mixture was warmed to room temperature and stirred further for 40 minutes. The reaction solution was concentrated under reduced pressure to obtain 2-[(tert-butoxycarbonyl)amino]-4-bromothiazole-5-carboxylic acid chloride. Tetrahydrofuran (5 mL) was added to the obtained acid chloride to dissolve, and then a solution of N-(4-fluoro-2-methylbenzyl)-O-methylhydroxylamine (148 mg) in tetrahydrofuran (5 mL) and diisopropylethylamine (0.31 mL) were added at room temperature, followed by stirring at 50°C for 1 hour and 45 minutes. After cooling to room temperature, the reaction solution was quenched with saturated aqueous sodium hydrogen carbonate solution. To the quenched reaction solution, ethyl acetate was added and the aqueous layer was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain amorphous tert-butyl{4-bromo-5-[(4-fluoro-2-methylbenzyl)(methoxy)carbamoyl]thiazol-2-yl} carbamate (370 mg). Its $^1$H-NMR spectrum data was as follows.
$^1$H NMR (DMSO-$d_6$/300 MHz):$\delta$ (ppm)= 12.10 (brs, 1H), 7.24 (dd, 1H), 7.10-6.99 (m, 2H), 4.90 (s, 2H), 3.62 (s, 3H), 2.31 (s, 3H), 1.48 (s, 9H).
(3) To a solution of tert-butyl{4-bromo-5-[(4-fluoro-2-methylbenzyl)(methoxy) carbamoyl]thiazol-2-yl} carbamate (370 mg) in dichloromethane (6 mL), trifluoroacetic acid (1.2 mL) was added, followed by stirring overnight under reflux with heating. After cooling to room temperature, saturated aqueous sodium hydrogen carbonate solution was slowly added to the reaction solution to basify the reaction solution. The aqueous layer of the basified reaction solution was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain solid desired product (262 mg). Its $^1$H-NMR spectrum data and melting point were as follows.

$^1$H NMR (DMSO-$d_6$/300 MHz):$\delta$ (ppm)= 7.93 (brs, 2H), 7.21 (dd, 1H), 7.08-6.97 (m, 2H), 4.84 (s, 2H), 3.59 (s, 3H), 2.29 (s, 3H).
Melting point: 164.0°C

Synthesis Example 3: Synthesis of

2-amino-4-chloro-N-(2-fluorobenzyl)-N-(2-propynyloxy)thiazole-5-carboxamide (Compound No. B-55)

(1) 2-fluorobenzyl bromide (500 mg) and O-2-propynylhydroxylamine hydrochloride (754 mg) synthesized in

accordance with Synthetic Example 1-2 of US Publication 2014/0378399 at page 16 were mixed with N,N-dimethylformamide (10 mL), and to the obtained mixture, diisopropylethylamine (2.31 mL) was added at room temperature, followed by stirring at 50°C for 5 hours. After cooling to room temperature, the reaction solution was quenched with water. To the quenched reaction solution, ethyl acetate and heptane were sequentially added, and the aqueous layer of the reaction solution was extracted with ethyl acetate/heptane mixture. The organic layer obtained by extraction was sequentially washed with water and brine, dried over anhydrous sodium sulfate, subjected to filtration and concentrated under reduced pressure. The obtained residue was purified by column chromatography (eluent: ethyl acetate/heptane) to obtain oily N-(2-fluorobenzyl)-O-(2-propynyl)hydroxylamine (69 mg). Its $^1$H-NMR spectrum data was as follows.
$^1$H NMR ($CDCl_3$/300 MHz):δ (ppm)= 7.38 (dt, 1H), 7.31-7.24 (m, 1H), 7.11 (dt, 1H), 7.08-7.02 (m, 1H), 6.02 (t, 1H), 4.29 (d, 2H), 4.18 (d, 2H), 2.41 (t, 1H).
(2) To a solution of 2-[(tert-butoxycarbonyl)amino]-4-chlorothiazole-5-carboxylic acid (128 mg) synthesized in accordance with Experimental 5.1.10 in Bioorganic & Medicinal Chemistry, 2004, 12, 6171-6182 at page 6177, in tetrahydrofuran (5 mL) under cooling with ice, oxalyl chloride (0.05 mL) and a catalytic amount of N,N-dimethylformamide were added, followed by stirring in a nitrogen atmosphere. 10 Minutes later, the mixture was warmed to room temperature and stirred further for 1 hour. The reaction solution was concentrated under reduced pressure to obtain 2-[(tert-butoxycarbonyl)amino]-4-chlorothiazole-5-carboxylic acid chloride. Tetrahydrofuran (2 mL) was added to the obtained acid chloride to dissolve, and then a solution of N-(2-fluorobenzyl)-O-2-propynylhydroxylamine (69 mg) in tetrahydrofuran (3 mL) and diisopropylethylamine (0.13 mL) were sequentially added at room temperature, followed by stirring at 50°C for 2 hours. After cooling to room temperature, the reaction solution was quenched with saturated aqueous sodium hydrogen carbonate solution. To the quenched reaction solution, ethyl acetate was added and the aqueous layer was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain amorphous tert-butyl{4-chloro-5-[(2-fluorobenzyl)(2-propynyloxy)carbamoyl]thiazol-2-yl} carbamate (87 mg). Its $^1$H-NMR spectrum data was as follows.
$^1$H NMR (DMSO-$d_6$/300 MHz):δ (ppm)= 12.09 (brs, 1H), 7.41-7.33 (m, 2H), 7.25-7.18 (m, 2H), 5.03 (s, 2H), 4.71 (d, 2H), 3.66 (t, 1H), 1.48 (s, 9H).
(3) To a solution of tert-butyl{4-chloro-5-[(2-fluorobenzyl)(2-propynyloxy)carbamoyl] thiazol-2-yl} carbamate (87 mg) in dichloromethane (1.5 mL), trifluoroacetic acid (0.3 mL) was added, followed by stirring overnight under reflux with heating. After cooling to room temperature, saturated aqueous sodium hydrogen carbonate solution was slowly added to the reaction solution to basify the reaction solution. The aqueous layer of the basified reaction solution was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain solid desired product (55 mg). Its $^1$H-NMR spectrum data and melting point were as follows.

$^1$H NMR (DMSO-$d_6$/300 MHz):δ (ppm)= 7.98 (brs, 2H), 7.40-7.29 (m, 2H), 7.23-7.16 (m, 2H), 4.97 (s, 2H), 4.64 (d, 2H), 3.68 (t, 1H).
Melting point: 154.1°C

Synthesis Example 4: Synthesis of

2-amino-4-chloro-N-(2,4-difluorobenzyl)-N-(2-propynyloxy)thiazole-5-carboxamide (Compound No. B-57)

(1) To a solution of O-2-propynylhydroxylamine hydrochloride (389 mg) in methanol (4 mL), triethylamine (0.5 mL) was added at 0°C, followed by stirring to obtain a mixture. To the obtained mixture, 2,4-difluorobenzaldehyde (514 mg) in methanol (6 mL) and acetic acid (1 mL) were sequentially added, and 30 minutes later, the mixture was warmed to room temperature. 3.5 Hours later, the mixture was cooled to 0°C and 2-picoline borane (967 mg) was added thereto, and 15 minutes later, 3N aqueous hydrochloric acid solution (7.2 mL) was added, followed by stirring for 3 hours to obtain a reaction solution. Saturated aqueous sodium hydrogen carbonate solution was added to the reaction solution to basify the reaction solution, and methanol was distilled off. The residue was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain oily N-(2,4-difluorobenzyl)-O-2-propynylhydroxylamine (471 mg). Its $^1$H-NMR spectrum data was as follows.

$^1H$ NMR ($CDCl_3$/300 MHz):δ (ppm)= 7.40-7.32 (m, 1H), 6.89-6.77 (m, 2H), 5.96 (t, 1H), 4.27 (d, 2H), 4.13 (d, 2H), 2.41 (t, 1H).

(2) To a solution of 2-[(tert-butoxycarbonyl)amino]-4-chlorothiazole-5-carboxylic acid (291 mg) in tetrahydrofuran (10 mL), under cooling with ice, oxalyl chloride (0.11 mL) and a catalytic amount of N,N-dimethylformamide were added, followed by stirring in a nitrogen atmosphere. 10 Minutes later, the mixture was warmed to room temperature and stirred further for 1 hour. The reaction solution was concentrated under reduced pressure to obtain 2-[(tert-butoxycarbonyl)amino]-4-chlorothiazole-5-carboxylic acid chloride. Tetrahydrofuran (5 mL) was added to the obtained acid chloride to dissolve, and then a solution of N-(2,4-difluorobenzyl)-O-2-propynylhydroxylamine (172 mg) in tetrahydrofuran (5 mL) and diisopropylethylamine (0.30 mL) were sequentially added at room temperature, followed by stirring at 50°C for 2 hours. After cooling to room temperature, the reaction solution was quenched with saturated aqueous sodium hydrogen carbonate solution. To the quenched reaction solution, ethyl acetate was added and the aqueous layer was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain amorphous tert-butyl{4-chloro-5-[(2,4-difluorobenzyl)(2-propynyloxy)carbamoyl]thiazol-2-yl} carbamate (204 mg). Its $^1H$-NMR spectrum data was as follows.

$^1H$ NMR (DMSO-$d_6$/300 MHz):δ (ppm)= 12.09 (brs, 1H), 7.46-7.38 (m, 1H), 7.31-7.24 (m, 1H), 7.14-7.08 (m, 1H), 5.00 (s, 2H), 4.71 (d, 2H), 3.66 (t, 1H), 1.48 (s, 9H).

(3) To a solution of tert-butyl{4-chloro-5-[(2,4-difluorobenzyl)(2-propynyloxy) carbamoyl]thiazol-2-yl} carbamate (204 mg) in dichloromethane (3.5 mL), trifluoroacetic acid (0.69 mL) was added, followed by stirring overnight under reflux with heating. After cooling to room temperature, saturated aqueous sodium hydrogen carbonate solution was slowly added to the reaction solution to basify the reaction solution. The aqueous layer of the basified reaction solution was extracted with ethyl acetate. The organic layer obtained by extraction was sequentially washed with water and brine, dried over anhydrous sodium sulfate, subjected to filtration, and concentrated under reduced pressure. The obtained residue was purified by column chromatography (eluent: ethyl acetate/heptane) to obtain solid desired product (132 mg). Its $^1H$-NMR spectrum data and melting point were as follows.

$^1H$ NMR (DMSO-$d_6$/300 MHz):δ (ppm)= 7.98 (brs, 2H), 7.42-7.34 (m, 1H), 7.30-7.22 (m, 1H), 7.13-7.06 (m, 1H), 4.93 (s, 2H), 4.64 (d, 2H), 3.68 (t, 1H).

Melting point: 171.5°C

Now, representative examples of the compound (I) are specifically shown in Tables 1-1 and 1-2. These compounds can be synthesized in accordance with the above production method and Synthesis Examples, and methods known in this technical field.

In Tables, No. represents Compound No. In Tables 1-1 and 1-2, abbreviations used in the columns $R^1$, $Y^1$, $Y^2$, $Y^3$, $Y^4$ and $Y^5$ represent the following substituents. Me is a methyl group, Et is an ethyl group, n-Pr is a n-propyl group, i-Pr is an isopropyl group, c-Pr is a cyclopropyl group, t-Bu is a tert-butyl group, c-Bu is a cyclobutyl group, CN is a cyano group, and $NO_2$ is a nitro group. In Table 1-1, = represents a double bond.

Further, in the case of salts of the compound (I), types of the salts are shown in the Remarks column in Tables 1-1 and 1-2. For examples, a compound indicated with HCl salt in the Remarks column in Table 1-1, is a hydrochloride, a compound indicated with TsOH salt is a p-toluenesulfonate, and a compound indicated with Na salt is a sodium salt. For example, compound No. A-125 is hydrochloride of compound No. A-20. Compound No. A-126 is p-toluenesulfonate of compound No. A-20. Compound No. A-127 is sodium salt of compound No. A-20.

A compound indicated with HCl salt in the Remarks column in Table 1-2, is a hydrochloride, a compound indicated with TsOH salt is a p-toluenesulfonate, and a compound indicated with Na salt is a sodium salt. For example, compound No. B-52 is hydrochloride of compound No. B-2. Compound No. B-53 is p-toluenesulfonate of compound No. B-2. Compound No. B-54 is sodium salt of compound No. B-2.

Table 1-1

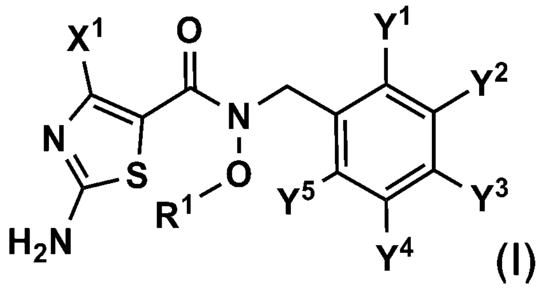

| No. | $R^1$ | $X^1$ | $Y^1$ | $Y^2$ | $Y^3$ | $Y^4$ | $Y^5$ | Remarks |
|---|---|---|---|---|---|---|---|---|
| A-1 | Me | Cl | Cl | H | F | F | H | |
| A-2 | Me | Br | Cl | H | F | F | H | |
| A-3 | Me | I | Cl | H | F | F | H | |
| A-4 | Me | F | Cl | H | F | F | H | |
| A-5 | Et | Cl | Cl | H | F | F | H | |
| A-6 | Me | Cl | Br | H | F | H | H | |
| A-7 | Et | Cl | Br | H | F | H | H | |
| A-8 | n-Pr | Cl | Cl | H | F | F | H | |
| A-9 | n-Pr | Cl | Br | H | F | H | H | |
| A-10 | Me | Cl | Cl | H | F | H | H | |
| A-11 | Me | Cl | F | H | F | H | H | |
| A-12 | Me | Cl | Me | H | F | H | H | |
| A-13 | Me | Cl | F | H | F | F | H | |
| A-14 | Me | Cl | Br | H | F | F | H | |
| A-15 | Me | Cl | $CF_3$ | H | F | H | H | |
| A-16 | Me | Cl | $CHF_2$ | H | F | H | H | |
| A-17 | Me | Cl | $NO_2$ | H | F | H | H | |
| A-18 | Me | Cl | $NO_2$ | H | F | F | H | |
| A-19 | Me | Cl | Me | H | F | F | H | |
| A-20 | Me | Cl | Cl | H | H | H | H | |
| A-21 | Me | Cl | Cl | F | H | H | H | |
| A-22 | Me | Cl | Cl | H | H | F | H | |
| A-23 | Me | Cl | Cl | F | F | H | H | |
| A-24 | Et | Cl | Cl | H | H | H | H | |
| A-25 | Et | Cl | Cl | F | H | H | H | |
| A-26 | Et | Cl | Cl | H | F | H | H | |
| A-27 | Et | Cl | Cl | H | H | F | H | |
| A-28 | Et | Cl | Cl | F | F | H | H | |
| A-29 | Me | Cl | F | H | H | H | H | |
| A-30 | Et | Cl | F | H | H | H | H | |
| No. | $R^1$ | $X^1$ | $Y^1$ | $Y^2$ | $Y^3$ | $Y^4$ | $Y^5$ | Remarks |
| A-31 | Me | Cl | F | F | H | H | H | |

(continued)

| No. | $R^1$ | $X^1$ | $Y^1$ | $Y^2$ | $Y^3$ | $Y^4$ | $Y^5$ | Remarks |
|---|---|---|---|---|---|---|---|---|
| A-32 | Et | Cl | F | F | H | H | H | |
| A-33 | Et | Cl | F | H | F | H | H | |
| A-34 | Me | Cl | F | H | H | F | H | |
| A-35 | Et | Cl | F | H | H | F | H | |
| A-36 | Me | Cl | F | F | F | H | H | |
| A-37 | Et | Cl | F | F | F | H | H | |
| A-38 | Me | Cl | F | F | H | F | H | |
| A-39 | Et | Cl | F | F | H | F | H | |
| A-40 | Et | Cl | F | H | F | F | H | |
| A-41 | Me | Cl | F | F | F | F | H | |
| A-42 | Et | Cl | F | F | F | F | H | |
| A-43 | Me | Cl | Me | H | H | H | H | |
| A-44 | Et | Cl | Me | H | H | H | H | |
| A-45 | Me | Cl | Me | F | H | H | H | |
| A-46 | Et | Cl | Me | F | H | H | H | |
| A-47 | Et | Cl | Me | H | F | H | H | |
| A-48 | Me | Cl | Me | H | H | F | H | |
| A-49 | Et | Cl | Me | H | H | F | H | |
| A-50 | Me | Cl | Me | F | F | H | H | |
| A-51 | Et | Cl | Me | F | F | H | H | |
| A-52 | Me | Cl | Me | F | H | F | H | |
| A-53 | Et | Cl | Me | F | H | F | H | |
| A-54 | Et | Cl | Me | H | F | F | H | |
| A-55 | Me | Cl | CN | H | H | H | H | |
| A-56 | Et | Cl | CN | H | H | H | H | |
| A-57 | Me | Cl | CN | F | H | H | H | |
| A-58 | Me | Cl | CN | H | F | H | H | |
| A-59 | Me | Cl | CN | H | H | F | H | |
| A-60 | Me | Cl | CN | H | F | F | H | |
| A-61 | Et | Cl | CN | F | H | H | H | |
| A-62 | Et | Cl | CN | H | F | H | H | |
| A-63 | Et | Cl | CN | H | H | F | H | |
| A-64 | Et | Cl | CN | H | F | F | H | |
| A-65 | Me | Cl | Br | H | H | H | H | |
| A-66 | Et | Cl | Br | H | H | H | H | |
| A-67 | Me | Cl | Br | F | H | H | H | |
| A-68 | Et | Cl | Br | F | H | H | H | |
| A-69 | Me | Cl | Br | H | H | F | H | |
| A-70 | Et | Cl | Br | H | H | F | H | |

(continued)

| No. | $R^1$ | $X^1$ | $Y^1$ | $Y^2$ | $Y^3$ | $Y^4$ | $Y^5$ | Remarks |
|---|---|---|---|---|---|---|---|---|
| A-71 | Me | Cl | Br | F | F | H | H | |
| A-72 | Et | Cl | Br | F | F | H | H | |
| A-73 | Me | Cl | Br | F | H | F | H | |
| A-74 | Et | Cl | Br | F | H | F | H | |
| A-75 | Et | Cl | Br | H | F | F | H | |
| A-76 | Me | Cl | H | Cl | H | H | H | |
| A-77 | Me | Cl | H | H | Cl | H | H | |
| A-78 | Me | Cl | H | F | H | H | H | |
| A-79 | Me | Cl | H | H | F | H | H | |
| A-80 | Me | F | I | H | F | F | H | |
| A-81 | Me | F | I | F | H | F | H | |
| A-82 | Me | Cl | $CF_3$ | H | H | H | H | |
| A-83 | Me | Cl | $CF_3$ | F | H | H | H | |
| A-84 | Me | Cl | $CF_3$ | H | H | F | H | |
| A-85 | Me | Cl | $CF_3$ | H | F | F | H | |
| A-86 | Me | Cl | $NO_2$ | H | H | H | H | |
| A-87 | Me | Cl | $NO_2$ | F | H | H | H | |
| A-88 | Me | Cl | $NO_2$ | H | H | F | H | |
| A-89 | Me | Cl | $NO_2$ | F | H | F | H | |
| A-90 | n-Pr | Cl | Cl | H | F | H | H | |
| A-91 | Me | Cl | H | F | H | F | H | |
| A-92 | Me | Cl | H | F | F | H | H | |
| A-93 | Me | Cl | H | F | F | F | H | |
| A-94 | Me | Br | Cl | H | H | H | H | |
| A-95 | Me | Br | Cl | H | F | H | H | |
| A-96 | Me | Br | Cl | F | H | H | H | |
| A-97 | Me | Br | Cl | H | H | F | H | |
| A-98 | Me | Br | Br | H | H | H | H | |
| A-99 | Me | Br | Br | H | F | H | H | |
| A-100 | Me | Br | Br | H | F | F | H | |
| A-101 | Me | Cl | $CHF_2$ | H | F | F | H | |
| A-102 | Me | Br | Cl | F | F | H | H | |
| A-103 | Me | Br | Cl | F | H | F | H | |
| A-104 | Me | Br | Me | H | H | H | H | |
| A-105 | Me | Br | Me | H | F | H | H | |
| A-106 | Me | Br | Me | H | F | F | H | |
| A-107 | Me | Br | CN | H | H | H | H | |
| A-108 | Me | Br | CN | H | F | H | H | |
| A-109 | Me | Br | CN | H | F | F | H | |

(continued)

| No. | $R^1$ | $X^1$ | $Y^1$ | $Y^2$ | $Y^3$ | $Y^4$ | $Y^5$ | Remarks |
|---|---|---|---|---|---|---|---|---|
| A-110 | Me | Br | $NO_2$ | H | H | H | H | |
| A-111 | Me | Br | $NO_2$ | H | F | H | H | |
| A-112 | Me | Br | $NO_2$ | H | F | F | H | |
| A-113 | Me | Cl | Cl | F | H | F | H | |
| A-114 | Et | Cl | Cl | F | H | F | H | |
| A-115 | Me | F | Cl | H | H | H | H | |
| A-116 | Me | F | Cl | H | F | H | H | |
| A-117 | Me | F | Cl | F | H | H | H | |
| A-118 | Me | F | Cl | H | H | F | H | |
| A-119 | Me | F | Br | H | F | H | H | |
| A-120 | Me | F | Me | H | F | H | H | |
| A-121 | Me | F | CN | H | F | H | H | |
| A-122 | Me | F | $NO_2$ | H | F | H | H | |
| A-123 | c-Pr | Cl | Cl | H | F | H | H | |
| A-124 | c-Pr | Cl | Cl | H | F | F | H | |
| A-125 | Me | Cl | Cl | H | H | H | H | HCl salt |
| A-126 | Me | Cl | Cl | H | H | H | H | TsOH salt |
| A-127 | Me | Cl | Cl | H | H | H | H | Na salt |
| A-128 | Me | F | Br | H | H | H | H | |
| A-129 | Me | F | Me | H | H | H | H | |
| A-130 | Me | F | CN | H | H | H | H | |
| A-131 | Me | F | $NO_2$ | H | H | H | H | |
| A-132 | Me | F | Br | H | F | F | H | |
| A-133 | Me | F | Me | H | F | F | H | |
| A-134 | Me | F | CN | H | F | F | H | |
| A-135 | Me | F | $NO_2$ | H | F | F | H | |
| A-136 | i-Pr | Cl | Cl | H | H | H | H | |
| A-137 | i-Pr | Cl | Cl | F | H | H | H | |
| A-138 | i-Pr | Cl | Cl | H | F | H | H | |
| A-139 | i-Pr | Cl | Cl | H | H | F | H | |
| A-140 | i-Pr | Cl | Cl | H | F | F | H | |
| A-141 | i-Pr | Cl | Cl | F | F | H | H | |
| A-142 | i-Pr | Cl | Cl | F | H | F | H | |
| A-143 | t-Bu | Cl | Cl | H | H | H | H | |
| A-144 | t-Bu | Cl | Cl | F | H | H | H | |
| A-145 | t-Bu | Cl | Cl | H | F | H | H | |
| A-146 | t-Bu | Cl | Cl | H | H | F | H | |
| A-147 | t-Bu | Cl | Cl | H | F | F | H | |
| A-148 | t-Bu | Cl | Cl | F | F | H | H | |

(continued)

| No. | $R^1$ | $X^1$ | $Y^1$ | $Y^2$ | $Y^3$ | $Y^4$ | $Y^5$ | Remarks |
|---|---|---|---|---|---|---|---|---|
| A-149 | t-Bu | Cl | Cl | F | H | F | H | |
| A-150 | c-Bu | Cl | Cl | H | H | H | H | |
| A-151 | c-Bu | Cl | Cl | F | H | H | H | |
| A-152 | c-Bu | Cl | Cl | H | F | H | H | |
| A-153 | c-Bu | Cl | Cl | H | H | F | H | |
| A-154 | c-Bu | Cl | Cl | H | F | F | H | |
| A-155 | c-Bu | Cl | Cl | F | F | H | H | |
| A-156 | c-Bu | Cl | Cl | F | H | F | H | |
| A-157 | c-Pr | Cl | Cl | H | H | H | H | |
| A-158 | c-Pr | Cl | Cl | F | H | H | H | |
| A-159 | c-Pr | Cl | Cl | H | H | F | H | |
| A-160 | c-Pr | Cl | Cl | F | F | H | H | |
| A-161 | c-Pr | Cl | Cl | F | H | F | H | |
| A-162 | c-Pr | Cl | F | H | H | H | H | |
| A-163 | c-Pr | Cl | F | H | F | H | H | |
| A-164 | c-Pr | Cl | F | H | F | F | H | |
| A-165 | c-Pr | Br | Cl | H | H | H | H | |
| A-166 | c-Pr | Br | Cl | F | H | H | H | |
| A-167 | c-Pr | Br | Cl | H | F | H | H | |
| A-168 | c-Pr | Br | Cl | H | H | F | H | |
| A-169 | c-Pr | Br | Cl | H | F | F | H | |
| A-170 | c-Pr | Br | Cl | F | F | H | H | |
| A-171 | c-Pr | Br | Cl | F | H | F | H | |
| A-172 | c-Pr | F | Cl | H | H | H | H | |
| A-173 | c-Pr | F | Cl | F | H | H | H | |
| A-174 | c-Pr | F | Cl | H | F | H | H | |
| A-175 | c-Pr | F | Cl | H | H | F | H | |
| A-176 | c-Pr | F | Cl | H | F | F | H | |
| A-177 | c-Pr | F | Cl | F | F | H | H | |
| A-178 | c-Pr | F | Cl | F | H | F | H | |
| A-179 | Me | Cl | $CHF_2$ | H | H | H | H | |
| A-180 | Me | Cl | $CHF_2$ | F | H | H | H | |
| A-181 | Me | Cl | $CHF_2$ | H | H | F | H | |
| A-182 | Me | Cl | $CHF_2$ | F | F | H | H | |
| A-183 | Me | Cl | $CHF_2$ | F | H | F | H | |
| A-184 | Me | F | $CHF_2$ | H | F | H | H | |
| A-185 | Me | F | $CHF_2$ | H | F | F | H | |
| A-186 | Me | Cl | I | H | H | H | H | |
| A-187 | Me | Cl | I | F | H | H | H | |

(continued)

| No. | $R^1$ | $X^1$ | $Y^1$ | $Y^2$ | $Y^3$ | $Y^4$ | $Y^5$ | Remarks |
|---|---|---|---|---|---|---|---|---|
| A-188 | Me | Cl | I | H | F | H | H | |
| A-189 | Me | Cl | I | H | H | F | H | |
| A-190 | Me | Cl | I | F | F | H | H | |
| A-191 | Me | Cl | I | H | F | F | H | |
| A-192 | Me | Cl | I | F | H | F | H | |
| A-193 | Me | F | I | H | H | H | H | |
| A-194 | Me | F | I | F | H | H | H | |
| A-195 | Me | F | I | H | F | H | H | |
| A-196 | Me | F | I | H | H | F | H | |
| A-197 | Me | F | I | F | F | H | H | |
| A-198 | Me | F | Cl | F | F | H | H | |
| A-199 | Me | F | Cl | F | H | F | H | |
| A-200 | Me | F | F | H | F | F | H | |
| A-201 | Me | F | Br | F | H | H | H | |
| A-202 | Me | F | Br | H | H | F | H | |
| A-203 | Me | F | Br | F | F | H | H | |
| A-204 | Me | F | Br | F | H | F | H | |
| A-205 | Et | F | Cl | H | F | H | H | |
| A-206 | Et | F | Cl | H | F | F | H | |
| A-207 | Me | F | Me | F | H | H | H | |
| A-208 | Me | F | Me | H | H | F | H | |
| A-209 | Me | F | Me | F | F | H | H | |
| A-210 | Me | F | Me | F | H | F | H | |
| A-211 | Me | F | F | H | H | H | H | |
| A-212 | Me | F | F | F | H | H | H | |
| A-213 | Me | F | F | H | F | H | H | |
| A-214 | Me | F | F | H | H | F | H | |
| A-215 | Me | F | F | F | F | H | H | |
| A-216 | Me | F | F | F | H | F | H | |
| A-217 | Me | F | F | F | F | F | H | |
| A-218 | Me | F | $NO_2$ | F | H | H | H | |
| A-219 | Me | F | $NO_2$ | H | H | F | H | |
| A-220 | Me | F | $NO_2$ | F | H | F | H | |
| A-221 | $CH_2SCH_3$ | Cl | Cl | H | H | H | H | |
| A-222 | $CH_2SCH_3$ | Cl | Cl | F | H | H | H | |
| A-223 | $CH_2SCH_3$ | Cl | Cl | H | F | H | H | |
| A-224 | $CH_2SCH_3$ | Cl | Cl | H | H | F | H | |
| A-225 | $CH_2SCH_3$ | Cl | Cl | F | F | H | H | |
| A-226 | $CH_2SCH_3$ | Cl | Cl | H | F | F | H | |

(continued)

| No. | $R^1$ | $X^1$ | $Y^1$ | $Y^2$ | $Y^3$ | $Y^4$ | $Y^5$ | Remarks |
|---|---|---|---|---|---|---|---|---|
| A-227 | Me | Cl | F | Cl | H | H | F | |
| A-228 | Et | Cl | F | Cl | H | H | F | |
| A-229 | $CH_2SCH_3$ | Cl | F | Cl | H | H | F | |
| A-230 | Me | Cl | H | Me | H | H | H | |
| A-231 | Me | Cl | H | H | Me | H | H | |
| A-232 | $CH_2CN$ | Cl | Cl | H | F | H | H | |
| A-233 | $CH_2CH{=}CH_2$ | Cl | Cl | H | F | H | H | |
| A-234 | $CH_2CH{=}CH_2$ | Cl | Cl | H | F | F | H | |
| A-235 | $CH_2C(=O)OCH_3$ | Cl | Cl | H | F | H | H | |
| A-236 | $CH_2C(=O)OCH_3$ | Cl | Cl | H | F | F | H | |
| A-237 | $CH_2CHF_2$ | Cl | Cl | H | F | H | H | |
| A-238 | $CH_2CH_2OCH_3$ | Cl | Cl | H | F | H | H | |
| A-239 | Me | Cl | Et | H | H | H | H | |
| A-240 | Me | Cl | n-Pr | H | H | H | H | |
| A-241 | Me | Cl | i-Pr | H | F | H | H | |
| A-242 | Me | Cl | $CH_2CF_3$ | H | F | H | H | |
| A-243 | Me | Cl | $CF_2CF_3$ | H | F | H | H | |
| A-244 | Me | Cl | $CH_2CHF_2$ | H | F | H | H | |
| A-245 | Me | Cl | $CH_2CH_2CF_3$ | H | F | H | H | |
| A-246 | Me | Cl | Et | F | H | H | H | |
| A-247 | Me | Cl | Et | H | H | F | H | |
| A-248 | Me | Cl | Et | F | F | H | H | |
| A-249 | Me | Cl | Et | F | H | F | H | |
| A-250 | Me | Cl | Et | H | F | F | H | |
| A-251 | Me | F | $CHF_2$ | F | F | H | H | |
| A-252 | Me | F | $CHF_2$ | F | H | H | H | |
| A-253 | Me | F | $CF_3$ | H | H | H | H | |
| A-254 | Me | Cl | Me | H | Me | H | H | |
| A-255 | Me | Br | H | F | F | H | H | |
| A-256 | Me | Cl | $NO_2$ | H | H | H | F | |

Table 1-2

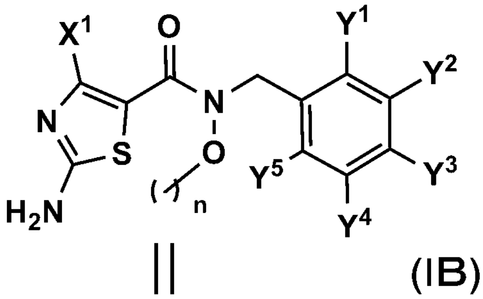

(IB)

| No. | n | $X^1$ | $Y^1$ | $Y^2$ | $Y^3$ | $Y^4$ | $Y^5$ | Remarks |
|---|---|---|---|---|---|---|---|---|
| B-1 | 1 | Cl | Br | H | F | H | H | |
| B-2 | 1 | Cl | Cl | H | H | H | H | |
| B-3 | 1 | Cl | Cl | F | H | H | H | |
| B-4 | 1 | Cl | Cl | H | F | H | H | |
| B-5 | 1 | Cl | Cl | H | H | F | H | |
| B-6 | 1 | Cl | Cl | F | F | H | H | |
| B-7 | 1 | Cl | Cl | H | F | F | H | |
| B-8 | 2 | Cl | Cl | H | F | H | H | |
| B-9 | 2 | Cl | Cl | H | F | F | H | |
| B-10 | 1 | Cl | Me | H | H | H | H | |
| B-11 | 1 | Cl | Me | F | H | H | H | |
| B-12 | 1 | Cl | Me | H | F | H | H | |
| B-13 | 1 | Cl | Me | H | H | F | H | |
| B-14 | 1 | Cl | Me | F | F | H | H | |
| B-15 | 1 | Cl | Me | F | H | F | H | |
| B-16 | 1 | Cl | Me | H | F | F | H | |
| B-17 | 1 | Cl | Br | H | H | H | H | |
| B-18 | 1 | Cl | Br | F | H | H | H | |
| B-19 | 1 | Cl | Br | H | H | F | H | |
| B-20 | 1 | Cl | Br | F | F | H | H | |
| B-21 | 1 | Cl | Br | F | H | F | H | |
| B-22 | 1 | Cl | Br | H | F | F | H | |
| B-23 | 1 | Cl | $CHF_2$ | H | F | H | H | |
| B-24 | 1 | Cl | $CHF_2$ | H | F | F | H | |
| B-25 | 1 | Cl | Cl | F | H | F | H | |
| B-26 | 1 | Cl | $CHF_2$ | H | H | H | H | |
| B-27 | 1 | Cl | $CHF_2$ | F | H | H | H | |
| B-28 | 1 | Cl | $CHF_2$ | H | H | F | H | |
| B-29 | 1 | Cl | $CHF_2$ | F | F | H | H | |
| B-30 | 1 | Cl | $CHF_2$ | F | H | F | H | |
| No. | n | $X^1$ | $Y^1$ | $Y^2$ | $Y^3$ | $Y^4$ | $Y^5$ | Remarks |
| B-31 | 1 | Cl | $CF_3$ | H | H | H | H | |

(continued)

| No. | n | $X^1$ | $Y^1$ | $Y^2$ | $Y^3$ | $Y^4$ | $Y^5$ | Remarks |
|---|---|---|---|---|---|---|---|---|
| B-32 | 1 | Cl | $CF_3$ | F | H | H | H | |
| B-33 | 1 | Cl | $CF_3$ | H | F | H | H | |
| B-34 | 1 | Cl | $CF_3$ | H | H | F | H | |
| B-35 | 1 | Cl | $CF_3$ | F | F | H | H | |
| B-36 | 1 | Cl | $CF_3$ | H | F | F | H | |
| B-37 | 1 | Cl | $CF_3$ | F | H | F | H | |
| B-38 | 1 | Cl | $NO_2$ | H | H | H | H | |
| B-39 | 1 | Cl | $NO_2$ | F | H | H | H | |
| B-40 | 1 | Cl | $NO_2$ | H | F | H | H | |
| B-41 | 1 | Cl | $NO_2$ | H | H | F | H | |
| B-42 | 1 | Cl | $NO_2$ | F | F | H | H | |
| B-43 | 1 | Cl | $NO_2$ | H | F | F | H | |
| B-44 | 1 | Cl | $NO_2$ | F | H | F | H | |
| B-45 | 1 | Cl | CN | H | H | H | H | |
| B-46 | 1 | Cl | CN | F | H | H | H | |
| B-47 | 1 | Cl | CN | H | F | H | H | |
| B-48 | 1 | Cl | CN | H | H | F | H | |
| B-49 | 1 | Cl | CN | F | F | H | H | |
| B-50 | 1 | Cl | CN | H | F | F | H | |
| B-51 | 1 | Cl | CN | F | H | F | H | |
| B-52 | 1 | Cl | Cl | H | H | H | H | HCl salt |
| B-53 | 1 | Cl | Cl | H | H | H | H | TsOH salt |
| B-54 | 1 | Cl | Cl | H | H | H | H | Na salt |
| B-55 | 1 | Cl | F | H | H | H | H | |
| B-56 | 1 | Cl | F | F | H | H | H | |
| B-57 | 1 | Cl | F | H | F | H | H | |
| B-58 | 1 | Cl | F | H | H | F | H | |
| B-59 | 1 | Cl | F | F | F | H | H | |
| B-60 | 1 | Cl | F | H | F | F | H | |
| B-61 | 1 | Cl | F | F | H | F | H | |
| B-62 | 1 | F | Cl | H | H | H | H | |
| B-63 | 1 | F | Cl | F | H | H | H | |
| B-64 | 1 | F | Cl | H | F | H | H | |
| B-65 | 1 | F | Cl | H | H | F | H | |
| B-66 | 1 | F | Cl | F | F | H | H | |
| B-67 | 1 | F | Cl | H | F | F | H | |
| B-68 | 1 | F | Cl | F | H | F | H | |
| B-69 | 1 | F | Br | H | H | H | H | |
| B-70 | 1 | F | Br | F | H | H | H | |

(continued)

| No. | n | X1 | Y1 | Y2 | Y3 | Y4 | Y5 | Remarks |
|---|---|---|---|---|---|---|---|---|
| B-71 | 1 | F | Br | H | F | H | H | |
| B-72 | 1 | F | Br | H | H | F | H | |
| B-73 | 1 | F | Br | F | F | H | H | |
| B-74 | 1 | F | Br | H | F | F | H | |
| B-75 | 1 | F | Br | F | H | F | H | |
| B-76 | 1 | F | F | H | H | H | H | |
| B-77 | 1 | F | F | F | H | H | H | |
| B-78 | 1 | F | F | H | F | H | H | |
| B-79 | 1 | F | F | H | H | F | H | |
| B-80 | 1 | F | F | F | F | H | H | |
| B-81 | 1 | F | F | H | F | F | H | |
| B-82 | 1 | F | F | F | H | F | H | |
| B-83 | 2 | Cl | Cl | H | H | H | H | |
| B-84 | 2 | Cl | Cl | F | H | H | H | |
| B-85 | 2 | Cl | Cl | H | H | F | H | |
| B-86 | 2 | Cl | Cl | F | F | H | H | |
| B-87 | 2 | Cl | Cl | F | H | F | H | |
| B-88 | 3 | Cl | Cl | H | H | H | H | |
| B-89 | 3 | Cl | Cl | H | F | H | H | |
| B-90 | 3 | Cl | Cl | H | F | F | H | |
| B-91 | 2 | Br | Cl | H | H | H | H | |
| B-92 | 2 | Br | Cl | H | F | H | H | |
| B-93 | 2 | Br | Cl | H | F | F | H | |
| B-94 | 2 | F | Cl | H | H | H | H | |
| B-95 | 2 | F | Cl | H | F | H | H | |
| B-96 | 2 | F | Cl | H | F | F | H | |
| B-97 | 1 | Cl | Et | H | H | H | H | |
| B-98 | 1 | Cl | Et | H | F | H | H | |
| B-99 | 1 | Cl | Et | H | F | F | H | |
| B-100 | 1 | Cl | I | H | H | H | H | |
| B-101 | 1 | Cl | I | F | H | H | H | |
| B-102 | 1 | Cl | I | H | F | H | H | |
| B-103 | 1 | Cl | I | H | H | F | H | |
| B-104 | 1 | Cl | I | F | F | H | H | |
| B-105 | 1 | Cl | I | H | F | F | H | |
| B-106 | 1 | Cl | I | F | H | F | H | |
| B-107 | 1 | Br | Cl | H | H | H | H | |
| B-108 | 1 | Br | Cl | H | F | H | H | |
| B-109 | 1 | Br | Cl | H | F | F | H | |

(continued)

| No. | n | $X^1$ | $Y^1$ | $Y^2$ | $Y^3$ | $Y^4$ | $Y^5$ | Remarks |
|---|---|---|---|---|---|---|---|---|
| B-110 | 1 | Cl | F | Cl | H | H | F | |
| B-111 | 1 | F | Cl | H | F | F | H | |
| B-112 | 1 | F | Cl | H | H | F | H | |
| B-113 | 1 | F | Cl | F | H | H | H | |
| B-114 | 1 | F | Cl | F | F | H | H | |
| B-115 | 1 | F | Me | H | F | F | H | |
| B-116 | 1 | F | Me | H | F | H | H | |
| B-117 | 1 | F | Me | H | H | F | H | |
| B-118 | 1 | F | Me | F | H | H | H | |
| B-119 | 1 | F | Me | F | F | H | H | |
| B-120 | 1 | F | Br | H | F | H | H | |
| B-121 | 1 | F | Br | H | H | F | H | |
| B-122 | 1 | F | Br | H | F | F | H | |
| B-123 | 1 | F | Br | F | F | H | H | |
| B-124 | 1 | F | Br | F | H | H | H | |
| B-125 | 1 | F | F | H | F | H | H | |
| B-126 | 1 | F | F | H | H | F | H | |
| B-127 | 1 | F | F | F | H | H | H | |
| B-128 | 1 | F | F | H | F | F | H | |

Physical properties of the compound (I) synthesized in accordance with the above Production Process and Examples are shown in Tables 2 and 3. Table 2 illustrates the melting points of the compound (I). In Table 3, $^1$H-NMR spectrum data of the compound (I) (measured by $^1$H-nuclear magnetic resonance spectroscopy; $\delta$ is the chemical shift value (ppm)). In Tables 2 and 3, No. is as defined for Tables 1-1 and 1-2.

The melting point indicated with *1 in Table 2 represents a temperature at which the compound was decomposed when the melting point of the compound was to be measured.

In the $^1$H-NMR spectrum data in Table 3, s is singlet, brs is broad singlet, d is doublet, t is triplet, q is quartet, and m is multiplet.

Table 2

| No. | Melting point (°C) |
|---|---|
| A-3 | 214.9 |
| A-5 | 189.8 |
| A-6 | 169.2 |
| A-7 | 228.6 |
| A-8 | 169.1 |
| A-11 | 162.0 |
| A-12 | 171.6 |
| A-13 | 143.7 |
| A-15 | 182.3 |
| A-16 | 168.0 |
| A-17 | 162.1 |

(continued)

| No. | Melting point (°C) |
|---|---|
| A-19 | 186.9 |
| A-21 | 180.0 |
| A-22 | 179.3 |
| A-23 | 198.8 |
| A-24 | 188.8 |
| A-25 | 172.5 |
| A-26 | 208.8 |
| A-27 | 195.1 |
| A-28 | 210.1 |
| A-29 | 153.4 |
| A-30 | 180.2 |
| A-31 | 156.4 |
| A-32 | 156.0 |
| A-33 | 178.6 |
| A-34 | 144.2 |
| A-35 | 165.0 |
| A-36 | 169.3 |
| A-37 | 193.6 |
| A-38 | 128.9 |
| A-39 | 182.0 |
| A-40 | 179.0 |
| A-41 | 156.1 |
| A-42 | 195.8 |
| A-43 | 182.5 |
| A-45 | 173.7 |
| A-46 | 189.5 |
| A-47 | 199.6 |
| A-51 | 208.4 |
| A-52 | 177.6 |
| A-54 | 190.8 |
| A-55 | 180.8 |
| A-56 | 168.0 |
| A-57 | 166.7 |
| A-58 | 194.0 |
| A-59 | 188.1 |
| A-60 | 190.2 |
| A-61 | 207.9 |
| A-62 | 188.7 |
| A-64 | 181.7 |

(continued)

| No. | Melting point (°C) |
|---|---|
| A-66 | 185.8 |
| A-67 | 187.9 |
| A-68 | 180.8 |
| A-69 | 181.5 |
| A-70 | 197.8 |
| A-71 | 205.0 |
| A-72 | 211.3 |
| A-73 | 188.9 |
| A-74 | 210.1 |
| A-75 | 199.5 |
| A-76 | 138.7 |
| A-77 | 153.4 |
| A-78 | 137.1 |
| A-79 | 148.5 |
| A-82 | 154.2 |
| A-83 | 162.4 |
| A-84 | 156.7 |
| A-86 | 171.7 |
| A-87 | 173.6 |
| A-88 | 183.1 |
| A-89 | 189.0 |
| A-91 | 157.4 |
| A-92 | 158.0 |
| A-93 | 184.0 |
| A-94 | 184.8 |
| A-95 | 170.3 |
| A-96 | 186.8 |
| A-97 | 172.2 |
| A-98 | 196.3 |
| A-99 | 172.4 |
| A-100 | 195.6 |
| A-101 | 161.9 |
| A-102 | 194.5 |
| A-103 | 186.8 |
| A-104 | 183.3 |
| A-105 | 164.0 |
| A-106 | 188.7 |
| A-108 | 199.1 |
| A-109 | 193.8 |

(continued)

| No. | Melting point (°C) |
|---|---|
| A-110 | 185.4 |
| A-111 | 177.6 |
| A-112 | 195.6 |
| A-113 | 184.8 |
| A-114 | 193.7 |
| A-115 | 242.6 (*1) |
| A-116 | 238.3 (*1) |
| A-117 | 251.0 (*1) |
| A-118 | 246.0 (*1) |
| A-119 | 248.4 (*1) |
| A-120 | 244.1 (*1) |
| A-121 | 248.5 (*1) |
| A-122 | 223.3 (*1) |
| A-123 | 176.8 |
| A-124 | 163.5 |
| A-128 | 243.2 (*1) |
| A-129 | 219.5 (*1) |
| A-131 | 227.1 (*1) |
| A-138 | 169.5 |
| A-211 | 224.5 (*1) |
| A-212 | 234.2 (*1) |
| A-213 | 239.4 (*1) |
| A-214 | 230.1 (*1) |
| A-215 | 238.7 (*1) |
| A-216 | 234.2 (*1) |
| A-217 | 238.2 (*1) |
| A-218 | 233.0 (*1) |
| A-219 | 231.1 (*1) |
| A-220 | 230.4 (*1) |
| A-221 | 170.0 |
| A-222 | 142.2 |
| A-223 | 187.3 |
| A-224 | 168.2 |
| A-225 | 181.8 |
| A-226 | 174.9 |
| A-230 | 145.6 |
| A-231 | 160.8 |
| A-234 | 196.3 |
| A-235 | 187.5 |

(continued)

| No. | Melting point (°C) |
|---|---|
| A-236 | 141.6 |
| A-237 | 179.2 |
| A-238 | 183.6 |
| B-1 | 210.8 |
| B-2 | 180.5 |
| B-3 | 159.0 |
| B-4 | 209.7 |
| B-5 | 269.3 |
| B-6 | 214.6 |
| B-8 | 187.7 |
| B-9 | 178.9 |
| B-10 | 156.5 |
| B-11 | 154.4 |
| B-12 | 204.1 |
| B-13 | 165.1 |
| B-14 | 188.5 |
| B-15 | 150.4 |
| B-16 | 177.0 |
| B-17 | 184.6 |
| B-18 | 155.1 |
| B-19 | 197.8 |
| B-20 | 195.0 |
| B-22 | 179.4 |
| B-25 | 169.4 |
| B-40 | 174.7 |
| B-47 | 168.2 |
| B-55 | 154.1 |
| B-57 | 171.5 |
| B-89 | 143.0 |
| B-108 | 192.0 |
| B-111 | 212.9 (*1) |
| B-112 | 203.4 (*1) |
| B-113 | 210.9 (*1) |
| B-114 | 215.3 (*1) |
| B-115 | 213.8 (*1) |
| B-116 | 204.3 (*1) |
| B-117 | 208.9 (*1) |
| B-118 | 204.2 (*1) |
| B-119 | 209.9 (*1) |

(continued)

| No. | Melting point (°C) |
|---|---|
| B-120 | 216.8 (*1) |
| B-121 | 207.7 (*1) |
| B-122 | 211.3 (*1) |
| B-123 | 219.2 (*1) |
| B-124 | 210.3 (*1) |
| B-125 | 201.2 (*1) |
| B-126 | 197.4 (*1) |
| B-127 | 207.7 (*1) |
| B-128 | 203.8 (*1) |

Table 3

| No. | $^1$H-NMR(300 MHz or 500 MHz, measurement solvent) δ(ppm) |
|---|---|
| A-1 | (300 MHz, DMSO-$d_6$) 8.00 (brs, 2H), 7.79 (dd, 1H), 7.41 (dd, 1H), 4.92 (s, 2H), 3.67 (s, 3H). |
| A-2 | (300 MHz, DMSO-$d_6$) 7.99 (brs, 2H), 7.79 (dd, 1H), 7.42 (dd, 1H), 4.93 (s, 2H), 3.67 (s, 3H). |
| A-4 | (300 MHz, DMSO-$d_6$) 8.16 (brs, 2H), 7.78 (dd, 1H), 7.39 (dd, 1H), 4.88 (s, 2H), 3.67 (s, 3H). |
| A-10 | (300 MHz, DMSO-$d_6$) 7.97 (brs, 2H), 7.48 (dd, 1H), 7.39 (dd, 1H), 7.25 (td, 1H), 4.94 (s, 2H), 3.65 (s, 3H). |
| A-14 | (300 MHz, DMSO-$d_6$) 8.00 (brs, 2H), 7.90 (dd, 1H), 7.37 (dd, 1H), 4.89 (s, 2H), 3.66 (s, 3H). |
| A-18 | (300 MHz, DMSO-$d_6$) 8.35 (dd, 1H), 8.02 (brs, 2H), 7.47 (dd, 1H), 5.18 (s, 2H), 3.68 (s, 3H). |
| A-20 | (300 MHz, DMSO-$d_6$) 7.98 (brs, 2H), 7.51-7.45 (m, 1H), 7.38-7.29 (m, 3H), 4.97 (s, 2H), 3.66 (s, 3H). |
| A-48 | (500 MHz, acetone-$d_6$) 7.22 (t, 1H), 7.16 (brs, 2H), 7.04 (d, 1H), 6.97-6.94 (m, 1H), 4.92 (s, 2H), 3.67 (d, 3H), 2.33 (s, 3H). |
| A-50 | (500 MHz, acetone-$d_6$) 7.17-7.10 (m, 4H), 4.92 (s, 2H), 3.64 (s, 3H), 2.31 (d, 3H). |
| A-65 | (500 MHz, acetone-$d_6$) 7.62 (d, 1H), 7.38 (d, 2H), 7.27-7.22 (m, 1H), 7.17 (brs, 2H), 5.02 (s, 2H), 3.72 (s, 3H). |
| A-85 | (500 MHz, acetone-$d_6$) 7.79 (dd, 1H), 7.47 (dd, 1H), 7.24 (brs, 2H), 5.10 (s, 2H), 3.75 (s, 3H). |
| A-94 | (300 MHz, DMSO-$d_6$) 7.96 (brs, 2H), 7.49-7.46 (m, 1H), 7.36-7.29 (m, 3H), 4.96 (s, 2H), 3.65 (s, 3H). |
| A-105 | (300 MHz, DMSO-$d_6$) 7.93 (brs, 2H), 7.21 (dd, 1H), 7.08-6.97 (m, 2H), 4.84 (s, 2H), 3.59 (s, 3H), 2.29 (s, 3H). |
| A-120 | (300 MHz, DMSO-$d_6$) 8.10 (brs, 2H), 7.20 (dd, 1H), 7.07-6.96 (m, 2H), 4.80 (s, 2H), 3.56 (s, 3H), 2.29 (s, 3H). |
| A-132 | (500 MHz, DMSO-$d_6$) 8.16 (brs, 2H), 7.90 (dd, 1H), 7.35 (dd, 1H), 4.85 (s, 2H), 3.67 (s, 3H). |
| A-133 | (500 MHz, DMSO-$d_6$) 8.12 (brs, 2H), 7.30 (dd, 1H), 7.18 (dd, 1H), 4.79 (s, 2H), 3.63 (s, 3H), 2.26 (s, 3H). |
| A-135 | (300 MHz, DMSO-$d_6$) 8.00 (brs, 2H), 7.79 (dd, 1H), 7.41 (dd, 1H), 4.92 (s, 2H), 3.67 (s, 3H). |
| A-145 | (300 MHz, DMSO-$d_6$) 7.92 (brs, 2H), 7.45 (dd, 1H), 7.36 (dd, 1H), 7.26 (dt, 1H), 4.89 (brs, 2H), 1.21 (s, 9H). |
| A-152 | (300 MHz, DMSO-$d_6$) 7.95 (brs, 2H), 7.47 (dd, 1H), 7.33 (dd, 1H), 7.25 (dt, 1H), 4.90 (s, 2H), 4.46-4.41 (m, 1H), 2.11-1.96 (m, 4H), 165-1.62 (m, 1H), 1.50-1.43 (m, 1H). |
| A-184 | (500 MHz, DMSO-$d_6$) 8.14 (brs, 2H), 7.45-7.40 (m, 3H), 7.26 (t, 1H), 4.97 (s, 2H), 3.45 (s, 3H). |
| A-191 | (500 MHz, acetone-$d_6$) 7.86 (dd, 1H) , 7.28 (dd, 1H), 7.20 (brs, 2H), 4.89 (s, 2H), 3.72 (s, 3H). |
| A-198 | (500 MHz, DMSO-$d_6$) 8.15 (brs, 2H), 7.48 (q, 1H), 7.23-7.19 (m, 1H), 4.93 (s, 2H), 3.67 (s, 3H). |
| A-199 | (500 MHz, DMSO-$d_6$) 8.17 (brs, 2H), 7.51 (dt, 1H), 7.05-7.03 (m, 1H), 4.96 (s, 2H), 3.69 (s, 3H). |

(continued)

| No. | $^1$H-NMR(300 MHz or 500 MHz, measurement solvent) δ(ppm) |
|---|---|
| A-200 | (500 MHz, DMSO-$d_6$) 8.13 (brs, 2H), 7.60-7.55 (m, 1H), 7.43-7.38 (m, 1H), 4.84 (s, 2H), 3.68 (s, 3H). |
| A-201 | (500 MHz, DMSO-$d_6$) 8.15 (brs, 2H), 7.43 (ddd, 1H), 7.34-7.30 (m, 1H), 7.12 (d, 1H), 4.94 (s, 2H), 3.67 (s, 3H). |
| A-202 | (500 MHz, DMSO-$d_6$) 8.17 (brs, 2H), 7.69 (dd, 1H), 7.16 (dt, 1H), 7.08 (dd, 1H), 4.88 (s, 2H), 3.68 (s, 3H). |
| A-203 | (500 MHz, DMSO-$d_6$) 8.15 (brs, 2H), 7.51 (dd, 1H), 7.20-7.17 (m, 1H), 4.91 (s, 2H), 3.66 (s, 3H). |
| A-204 | (500 MHz, DMSO-$d_6$) 8.17 (brs, 2H), 7.47 (dt, 1H), 7.00 (d, 1H), 4.93 (s, 2H), 3.69 (t, 3H). |
| A-205 | (500 MHz, DMSO-$d_6$) 8.11 (brs, 2H), 7.48-7.46 (m, 1H), 7.40-7.37 (m, 1H), 7.27-7.23 (m, 1H), 4.89 (s, 2H), 3.92 (q, 2H), 1.17 (t, 3H). |
| A-206 | (500 MHz, DMSO-$d_6$) 8.13 (brs, 2H), 7.77 (dd, 1H), 7.38 (dd, 1H), 4.88 (s, 2H), 3.93 (q, 2H), 1.18 (t, 3H). |
| A-207 | (500 MHz, acetone-$d_6$) 7.31 (brs, 2H), 7.22-7.18 (m, 1H), 7.13 (d, 1H), 7.03 (t, 1H), 4.92 (s, 2H), 3.63 (s, 3H), 2.26 (d, 3H). |
| A-208 | (500 MHz, acetone-$d_6$) 7.31 (brs, 2H), 7.21 (dd, 1H), 7.02 (dd, 1H), 6.95 (dt, 1H), 4.88 (s, 2H), 3.68 (s, 3H), 2.32 (s, 3H). |
| A-209 | (500 MHz, acetone-$d_6$) 7.31 (brs, 2H), 7.16-7.10 (m, 2H), 4.89 (s, 2H), 3.64 (s, 3H), 2.31 (d, 3H). |
| A-210 | (500 MHz, acetone-$d_6$) 7.34 (brs, 2H), 6.95-6.89 (m, 2H), 4.93 (s, 2H), 3.69 (s, 3H), 2.23 (s, 3H). |
| A-227 | (300 MHz, DMSO-$d_6$) 7.97 (brs, 2H), 7.66 (dd, 1H), 7.22 (td, 1H), 4.99 (s, 2H), 3.65 (s, 3H). |
| A-232 | (300 MHz, DMSO-$d_6$) 8.11 (brs, 2H), 7.50 (dd, 1H), 7.38 (dd, 1H), 7.27 (td, 1H), 5.04 (s, 2H), 5.02 (s, 2H). |
| B-7 | (300 MHz, DMSO-$d_6$) 8.01 (brs, 2H), 7.78 (dd, 1H), 7.41 (dd, 1H), 4.96 (s, 2H), 4.66 (d, 2H), 3.69 (t, 1H). |
| B-21 | (300 MHz, DMSO-$d_6$) 8.04 (brs, 2H), 7.48 (td, 1H), 7.05-7.01 (m, 1H), 5.01 (s, 2H), 4.69 (d, 2H), 3.71 (t, 1H). |
| B-24 | (300 MHz, DMSO-$d_6$) 8.01 (brs, 2H), 7.73 (dd, 1H), 7.41 (dd, 1H), 7.22 (t, 1H), 5.05 (s, 2H), 4.67 (d, 2H), 3.70 (t, 1H). |
| B-43 | (300 MHz, DMSO-$d_6$) 8.37 (dd, 1H), 8.06 (brs, 2H), 7.46 (dd, 1H), 5.22 (s, 2H), 4.68 (d, 2H), 3.71 (t, 1H). |
| B-50 | (300 MHz, DMSO-$d_6$) 8.21 (dd, 1H), 8.04 (brs, 2H), 7.54 (dd, 1H), 5.05 (s, 2H), 4.69 (d, 2H), 3.72 (t, 1H). |
| B-55 | (300 MHz, DMSO-$d_6$) 7.98 (brs, 2H), 7.40-7.29 (m, 2H), 7.23-7.16 (m, 2H), 4.97 (s, 2H), 4.64 (d, 2H), 3.68 (t, 1H). |
| B-57 | (300 MHz, DMSO-$d_6$) 7.98 (brs, 2H), 7.42-7.34 (m, 1H), 7.30-7.22 (m, 1H), 7.13-7.06 (m, 1H), 4.93 (s, 2H), 4.64 (d, 2H), 3.68 (t, 1H). |
| B-64 | (300 MHz, DMSO-$d_6$) 8.18 (brs, 2H), 7.48 (dd, 1H), 7.35 (dd, 1H), 7.25 (dt, 1H), 4.95 (s, 2H), 4.65 (d, 2H), 3.70 (t, 1H). |
| B-105 | (500 MHz, acetone-$d_6$) 7.86 (dd, 1H), 7.30 (dd, 1H), 7.22 (brs, 2H), 4.95 (s, 2H), 4.65 (d, 2H), 3.18 (t, 1H). |

[Test examples]

Preparation of each chemical solution containing Invention Compound:

The Invention Compound and acetone or dimethyl sulfoxide were mixed to dissolve the Invention Compound. The solution of the Invention Compound was diluted with water to a predetermined concentration of the Invention Compound as an active ingredient (400 ppm) to obtain chemical solutions. The chemical solutions thus obtained were used in the following Test Examples 1 to 3.

Test Example 1: Test on fungicidal effect against tomato late blight (Phytophthora infestans) (test on preventive effect)

Tomato was cultivated in a polyethylene pot having a diameter of 6 cm, and when the tomato reached 4.5- to 5.5-leaf stage, 10 ml of the chemical solution was applied by means of a spray gun. After the chemical solution dried (the day of application), the tomato was sprayed and inoculated with a suspension of spores of Phytophthora infestans, and the

tomato was placed in an inoculation box at a temperature of 20°C under a humidity of 95% or higher for 16 hours. Then, the tomato was placed in a constant temperature room at 20°C. 3 Days after the inoculation, the lesion area ratio was examined, and the control value was calculated in accordance with the following formula. As a result, chemical solutions containing each of the following test compounds (active ingredient concentration: 400 ppm) had a control value of 90% or higher on tomato late blight.

[Calculation of control value]

$$\text{Control value (\%)} = 100 - (X/Y) \times 100$$

X: lesion area ratio (%) in treated plot, Y: lesion area ratio (%) in non-treated plot

Test compounds: compound Nos. A-3, A-5, A-6, A-7, A-8, A-10, A-11, A-12, A-14, A-15, A-16, A-18, A-19, A-20, A-21, A-22, A-23, A-24, A-25, A-26, A-27, A-28, A-29, A-30, A-31, A-32, A-33, A-34, A-35, A-36, A-37, A-38, A-39, A-40, A-41, A-42, A-43, A-45, A-46, A-47, A-48, A-50, A-51, A-52, A-54, A-55, A-56, A-57, A-58, A-59, A-60, A-61, A-62, A-64, A-65, A-66, A-67, A-68, A-69, A-70, A-71, A-72, A-73, A-74, A-75, A-76, A-77, A-78, A-79, A-82, A-83, A-84, A-85, A-86, A-87, A-88, A-89, A-91, A-92, A-93, A-94, A-95, A-96, A-97, A-98, A-99, A-100, A-101, A-102, A-103, A-104, A-105, A-106, A-108, A-109, A-110, A-111, A-112, A-113, A-114, A-115, A-116, A-117, A-118, A-119, A-120, A-121, A-122, A-123, A-124, A-128, A-129, A-131, A-132, A-133, A-135, A-138, A-152, A-184, A-191, A-198, A-199, A-200, A-201, A-202, A-203, A-204, A-205, A-206, A-207, A-208, A-209, A-210, A-211, A-212, A-213, A-214, A-215, A-216, A-217, A-218, A-219, A-220, A-221, A-222, A-224, A-225, A-226, A-227, A-230, A-232, A-234, A-235, A-236, A-237, A-238, B-1, B-2, B-3, B-4, B-5, B-6, B-7, B-8, B-9, B-10, B-11, B-12, B-13, B-14, B-15, B-16, B-17, B-18, B-19, B-20, B-21, B-22, B-24, B-25, B-40, B-43, B-47, B-50, B-55, B-57, B-64, B-89, B-105, B-108, B-111, B-112, B-113, B-114, B-115, B-116, B-117, B-118, B-119, B-120, B-121, B-122, B-123, B-124, B-125, B-126, B-127, B-128

Test Example 2: Test on fungicidal effect against cucumber downy mildew (<u>Pseudoperonospora</u> <u>cubensis</u>) (test on preventive effect)

Cucumber was cultivated in a polyethylene pot having a diameter of 6 cm, and when the cucumber reached 1.2- to 1.5-leaf stage, 10 ml of the chemical solution was applied by means of a spray gun. After the chemical solution dried (the day of application), the cucumber was sprayed and inoculated with a suspension of spores of <u>Pseudoperonospora cubensis</u>, and the cucumber was placed in an inoculation box at a temperature of 20°C under a humidity of 95% or higher for 24 hours. Then, the cucumber was placed in a constant temperature room at 20°C. 7 Days after the inoculation, the lesion area ratio was examined, and the control value was calculated in accordance with the same formula as in Test Example 1. As a result, chemical solutions containing each of the following test compounds (active ingredient concentration: 400 ppm) had a control value of 90% or higher on cucumber downy mildew.

Test Compounds: compound Nos. A-3, A-5, A-6, A-10, A-12, A-15, A-16, A-18, A-19, A-20, A-21, A-22, A-23, A-24, A-26, A-28, A-29, A-30, A-31, A-32, A-33, A-35, A-38, A-39, A-40, A-42, A-43, A-45, A-47, A-48, A-50, A-51, A-52, A-54, A-55, A-57, A-58, A-59, A-62, A-64, A-65, A-67, A-68, A-69, A-71, A-72, A-73, A-75, A-76, A-77, A-78, A-79, A-82, A-83, A-85, A-91, A-92, A-94, A-95, A-96, A-97, A-98, A-99, A-100, A-102, A-103, A-106, A-109, A-111, A-112, A-113, A-114, A-115, A-116, A-117, A-118, A-119, A-120, A-121, A-122, A-123, A-124, A-128, A-129, A-131, A-132, A-133, A-135, A-138, A-145, A-152, A-184, A-191, A-198, A-199, A-200, A-201, A-202, A-203, A-204, A-205, A-206, A-207, A-208, A-209, A-210, A-211, A-212, A-213, A-214, A-215, A-216, A-217, A-218, A-219, A-220, A-221, A-222, A-223, A-224, A-225, A-226, A-232, A-234, A-237, A-238, B-2, B-3, B-4, B-5, B-6, B-7, B-8, B-9, B-10, B-11, B-13, B-14, B-15, B-16, B-17, B-18, B-19, B-20, B-21, B-22, B-24, B-25, B-40, B-43, B-47, B-50, B-55, B-57, B-64, B-105, B-108, B-111, B-112, B-113, B-114, B-115, B-116, B-117, B-118, B-119, B-120, B-121, B-122, B-123, B-124, B-125, B-126, B-127, B-128

Test Example 3: Test on fungicidal effect against tomato late blight (<u>Phytophthora infestans</u>) (test on curative effect)

Tomato was cultivated in a polyethylene pot having a diameter of 6 cm, and when the tomato reached 4.5- to 5.5-leaf stage, the tomato was sprayed and inoculated with a suspension of spores of <u>Phytophthora infestans</u> and placed in an inoculation box at a temperature of 20°C under a humidity of 95% or higher for 4 hours. Then, 10 ml of the chemical solution was applied by means of a spray gun. After the chemical solution dried (the day of application), the tomato was placed in a constant temperature room at 20°C. 3 Days after the inoculation, the lesion area ratio was examined, and the control value was calculated in accordance with the same formula as in Test Example 1. As a result, chemical solutions containing each of the following test compounds (active ingredient concentration: 400 ppm) had a control value of 90% or higher on tomato late blight.

Test Compounds: compound Nos. A-2, A-5, A-6, A-10, A-11, A-12, A-13, A-14, A-16, A-18, A-19, A-20, A-21, A-22, A-23, A-29, A-31, A-32, A-33, A-34, A-35, A-36, A-38, A-40, A-41, A-43, A-45, A-48, A-50, A-52, A-55, A-57, A-58,

A-60, A-64, A-65, A-67, A-69, A-71, A-77, A-78, A-79, A-85, A-86, A-87, A-88, A-89, A-91, A-92, A-93, A-95, A-97, A-99, A-101, A-109, A-111, A-113, A-115, A-116, A-117, A-118, A-119, A-120, A-121, A-122, A-123, A-124, A-128, A-129, A-132, A-133, A-184, A-191, A-198, A-199, A-200, A-202, A-203, A-204, A-205, A-206, A-207, A-208, A-209, A-210, A-211, A-213, A-214, A-215, A-216, A-217, A-220, A-226, A-230, A-232, A-238, B-7, B-9, B-16, B-22, B-50, B-64, B-111, B-112, B-114, B-115, B-116, B-118, B-119, B-121, B-122, B-123, B-125, B-126, B-128

Comparative Test

A comparative test was conducted using as the active ingredient Compound No. A-10 of the Invention Compound and a compound as described in Patent Document 1, production example 2 (hereinafter referred to as Comparative Compound 1).

Preparation of chemical solution containing each compound: Each of chemical solutions was prepared in the same method as above using each of the test compounds shown in the following Table 4 at a predetermined concentration of the active ingredient (400 ppm, 200 ppm, 100 ppm or 50 ppm). Each of the chemical solutions adjusted to each predetermined concentration was used for the following Comparative Test Example 1.

Comparative Test Example 1: Test on fungicidal effect against cucumber downy mildew (Pseudoperonospora cubensis) (test on preventive effect)

Using each of the chemical solutions adjusted to the above predetermined concentrations, in accordance with the method described in Test Example 2, test on fungicidal effect against cucumber downy mildew was conducted.

Results with the chemical solutions containing the respective test compounds at the respective active ingredient concentrations are shown in Table 4. A indicates a control value of 100%, B indicates a control value of 90-99%, C indicates a control value of 70-89%, D indicates a control value of 30-69%, and E indicates a control value of 0-29%.

Table 4

| Compound No. | Test compound | Active ingredient concentration (ppm) | | | |
|---|---|---|---|---|---|
| | | 400 | 200 | 100 | 50 |
| A-10 (Invention Compound) | 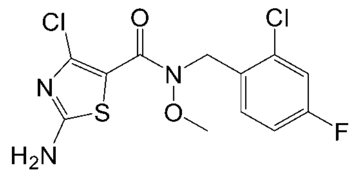 | A | A | A | A |
| Comparative Compound 1 | 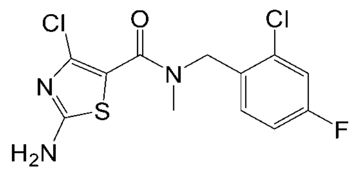 | C | C | C | D |

Table 4 shows an excellent control value of compound No. A-10 which is the N-substituted oxyamide compound of the present invention against cucumber downy mildew, as compared with the amide compound in Patent Document 1 (Comparative Compound 1).

Accordingly, it was found from Examples that the N-substituted oxyamide compound of the present invention has excellent controlling effects against harmful plant diseases. Further, it was also found that the N-substituted oxyamide compound of the present invention has more excellent controlling effects against harmful plant disease, than the amide compound of Patent Document 1.

Now, formulation of the Compound (I) will be specifically described. However, the mix ratio, the dosage form, etc. are not limited to the following Formulation Examples.

FORMULATION EXAMPLE 1

| | |
|---|---|
| (1) Compound (I) | 20 parts by weight |

(continued)

| | |
|---|---|
| (2) Clay | 72 parts by weight |
| (3) Sodium lignin sulfonate | 8 parts by weight |

The above components are uniformly mixed to obtain a wettable powder.

FORMULATION EXAMPLE 2

| | |
|---|---|
| (1) Compound (I) | 5 parts by weight |
| (2) Talc | 95 parts by weight |

The above components are uniformly mixed to obtain a dust.

FORMULATION EXAMPLE 3

| | |
|---|---|
| (1) Compound (I) | 20 parts by weight |
| (2) N,N-dimethylacetamide | 20 parts by weight |
| (3) Polyoxyethylene alkyl phenyl ether | 10 parts by weight |
| (4) Xylene | 50 parts by weight |

The above components are uniformly mixed and dissolved to obtain an emulsifiable concentrate.

FORMULATION EXAMPLE 4

| | |
|---|---|
| (1) Clay | 68 parts by weight |
| (2) Sodium lignin sulfonate | 2 parts by weight |
| (3) Polyoxyethylene alkyl aryl sulfate | 5 parts by weight |
| (4) Pulverized silica | 25 parts by weight |

The mixture of the above components is mixed with the Compound (I) in a weight ratio of 4:1 to obtain a wettable powder.

FORMULATION EXAMPLE 5

| | |
|---|---|
| (1) Compound (I) | 50 parts by weight |
| (2) Polyoxyethylene alkyl phenyl ether phosphate triethanolamine salt | 2 parts by weight |
| (3) Silicone | 0.2 part by weight |
| (4) Water | 47.8 parts by weight |

The above components are uniformly mixed and pulverized to obtain a base liquid, and

| | |
|---|---|
| (5) Sodium polycarboxylate | 5 parts by weight |
| (6) Anhydrous sodium sulfate | 42.8 parts by weight |

are added, and the mixture is uniformly mixed, granulated and dried to obtain a water-dispersible granule.

FORMULATION EXAMPLE 6

| | |
|---|---|
| (1) Compound (I) | 5 parts by weight |
| (2) Polyoxyethylene octylphenyl ether | 1 part by weight |
| (3) Polyoxyethylene phosphoric acid ester | 0.1 part by weight |
| (4) Granular calcium carbonate | 93.9 parts by weight |

The above components (1) to (3) are preliminarily uniformly mixed and diluted with a proper amount of acetone, and then the mixture is sprayed onto the component (4), and acetone is removed to obtain a granule.

FORMULATION EXAMPLE 7

| | |
|---|---|
| (1) Compound (I) | 2.5 parts by weight |
| (2) N-methyl-2-pyrrolidone | 2.5 parts by weight |
| (3) Soybean oil | 95.0 parts by weight |

The above components are uniformly mixed and dissolved to obtain an ultra low volume formulation.

FORMULATION EXAMPLE 8

| | |
|---|---|
| (1) Compound (I) | 20 parts by weight |
| (2) Polyoxyethylene alkyl phenyl ether phosphate triethanolamine salt | 2 parts by weight |
| (3) Silicone | 0.2 part by weight |
| (4) Xanthan gum | 0.1 part by weight |
| (5) Ethylene glycol | 5 parts by weight |
| (6) Water | 72.7 parts by weight |

The above components are uniformly mixed and pulverized to obtain a water-based suspension.

The entire disclosures of Japanese Patent Application No. 2023-148047 filed on September 13, 2023 and Japanese Patent Application No. 2023-148050 filed on September 13, 2023 including specifications, claims, drawings and abstracts are incorporated herein by reference in their entireties.

## Claims

**1.** A compound represented by the formula (I) or a salt thereof:

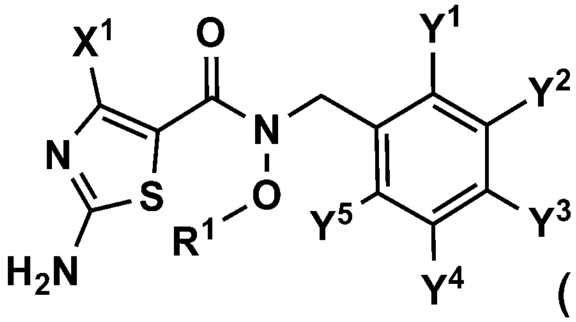

(I)

wherein $R^1$ is a ($C_1$-$C_5$)-chain hydrocarbon or a ($C_3$-$C_4$)-cycloalkyl (provided that the ($C_1$-$C_5$)-chain hydrocarbon and the ($C_3$-$C_4$)-cycloalkyl may be substituted with at least one $T^1$),
$T^1$ is a halogen, cyano or -$U^1$-$R^2$,
$U^1$ is O, S or C(=O)O,
$R^2$ is a ($C_1$-$C_3$)-alkyl,
$X^1$ is a halogen, and
$Y^1$, $Y^2$, $Y^3$, $Y^4$ and $Y^5$ are each independently a halogen, a ($C_1$-$C_6$)-alkyl, a ($C_1$-$C_6$)-haloalkyl, nitro, cyano or a hydrogen atom, provided that when $R^1$ is a ($C_1$-$C_4$)-alkyl or a ($C_3$-$C_4$)-cycloalkyl, all of $Y^1$, $Y^2$, $Y^3$, $Y^4$ and $Y^5$ are not hydrogen atoms at the same time.

**2.** The compound or a salt thereof according to claim 1, wherein $R^1$ is a ($C_1$-$C_4$)-alkyl, a ($C_3$-$C_4$)-cycloalkyl or a ($C_3$-$C_5$)-alkynyl.

**3.** The compound or a salt thereof according to claim 1, wherein the compound of the formula (I) is a N-(cyclo) alkoxyamide compound represented by the formula (IA):

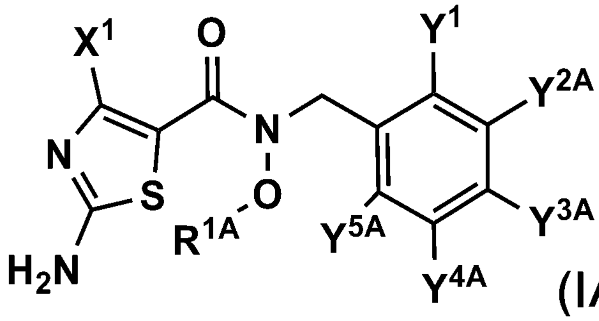

(IA)

wherein $R^{1A}$ is a ($C_1$-$C_4$)-alkyl or a ($C_3$-$C_4$)-cycloalkyl,
$X^1$ is a halogen,
$Y^1$ is a halogen, a ($C_1$-$C_6$)-alkyl, a ($C_1$-$C_6$)-haloalkyl, nitro, cyano or a hydrogen atom,
$Y^{2A}$, $Y^{3A}$ and $Y^{4A}$ are each independently a hydrogen atom or a halogen, and
$Y^{5A}$ is a hydrogen atom, provided that when Y' is a hydrogen atom, all of $Y^{2A}$, $Y^{3A}$ and $Y^{4A}$ are not hydrogen atoms at the same time.

**4.** The compound or a salt thereof according to claim 1, wherein the compound of the formula (I) is a N-alkynyloxyamide compound represented by the formula (IB):

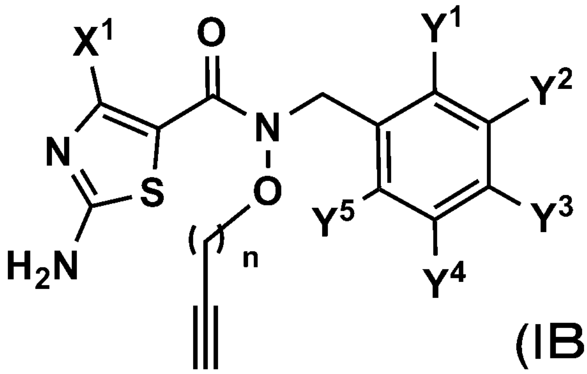

(IB)

wherein n is 1, 2 or 3,
$X^1$ is a halogen, and
$Y^1$, $Y^2$, $Y^3$, $Y^4$ and $Y^5$ are each independently a hydrogen atom, a halogen, a ($C_1$-$C_6$)-alkyl, a ($C_1$-$C_6$)-haloalkyl, nitro or cyano.

**5.** The compound or a salt thereof according to claim 1, wherein the compound of the formula (I) is a N-(cyclo) alkoxyamide compound of the formula (IA):

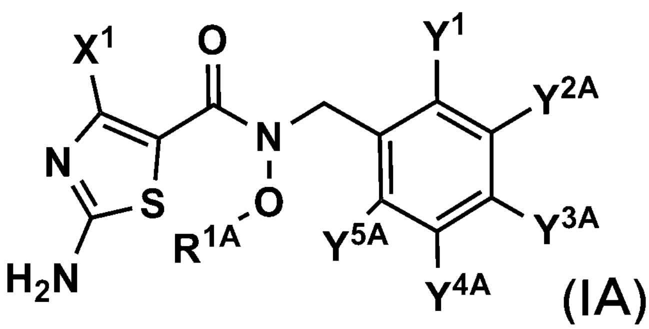

(IA)

wherein $R^{1A}$ is a ($C_1$-$C_4$)-alkyl or a ($C_3$-$C_4$)-cycloalkyl,
$X^1$ is a halogen,
$Y^1$ is a halogen, a ($C_1$-$C_6$)-alkyl, a ($C_1$-$C_6$)-haloalkyl, nitro, cyano or a hydrogen atom,
$Y^{2A}$, $Y^{3A}$ and $Y^{4A}$ are each independently a hydrogen atom or a halogen, and
$Y^{5A}$ is a hydrogen atom, provided that when $Y^1$ is a hydrogen atom, all of $Y^{2A}$, $Y^{3A}$ and $Y^{4A}$ are not hydrogen atoms at the same time, or
a N-alkynyloxyamide compound of the formula (IB):

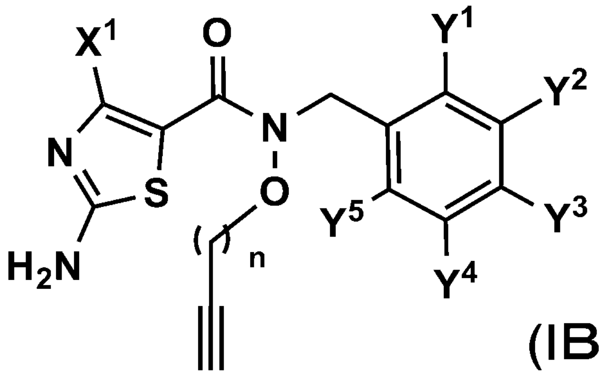

(IB)

wherein n is 1, 2 or 3,
$X^1$ is a halogen, and
$Y^1$, $Y^2$, $Y^3$, $Y^4$ and $Y^5$ are each independently a hydrogen atom, a halogen, a ($C_1$-$C_6$)-alkyl, a ($C_1$-$C_6$)-haloalkyl, nitro or cyano.

**6.** The compound or a salt thereof according to any one of claims 1 to 5, wherein Y' is a halogen, a ($C_1$-$C_6$)-alkyl, a ($C_1$-$C_6$)-haloalkyl, nitro or cyano.

**7.** The compound or a salt thereof according to claim 3 or 5, wherein $R^{1A}$ is methyl, ethyl or cyclopropyl.

**8.** The compound or a salt thereof according to any one of claims 1, 2, 4 and 5, wherein $Y^5$ is a hydrogen atom.

**9.** An agricultural and horticultural fungicide, which comprises as an active ingredient the compound or a salt thereof as defined in any one of claims 1 to 5.

**10.** A method for controlling harmful plant diseases, which comprises applying an effective amount of the compound or a salt thereof as defined in any one of claims 1 to 5 to a plant body, phytopathogen or soil.

## INTERNATIONAL SEARCH REPORT

International application No. **PCT/JP2024/030537**

**A. CLASSIFICATION OF SUBJECT MATTER**

***C07D 277/56***(2006.01)i; ***A01N 43/78***(2006.01)i; ***A01P 3/00***(2006.01)i
FI: C07D277/56 CSP; A01N43/78 D; A01P3/00

According to International Patent Classification (IPC) or to both national classification and IPC

**B. FIELDS SEARCHED**

Minimum documentation searched (classification system followed by classification symbols)

C07D277/56; A01N43/78; A01P3/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2011-001358 A (SUMITOMO CHEMICAL COMPANY, LIMITED) 06 January 2011 (2011-01-06)<br>entire text, all drawings | 1-10 |
| A | REGISTRY(STN) [online], 03 April 2019, p. 1, [retrieved on 17 October 2024], CAS: 2299658-69-6<br>entire text | 1-10 |
| A | JP 09-031069 A (NISSAN CHEMICAL INDUSTRIES, LTD.) 04 February 1997 (1997-02-04)<br>entire text, all drawings | 1-10 |
| P, A | WO 2023/176458 A1 (ISHIHARA SANGYO KAISHA, LTD.) 21 September 2023 (2023-09-21)<br>entire text, all drawings | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 October 2024** | **29 October 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2024/030537**

| Patent document cited in search report | Publication date (day/month/year) | Patent family member(s) | Publication date (day/month/year) |
|---|---|---|---|
| JP 2011-001358 A | 06 January 2011 | (Family: none) | |
| JP 09-031069 A | 04 February 1997 | (Family: none) | |
| WO 2023/176458 A1 | 21 September 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010134634 A
- WO 2011023645 A
- WO 2020028141 A
- WO 2009100171 A
- WO 2012006760 A
- WO 2006138350 A
- US 20140378399 A
- WO 2010049946 A
- WO 2012008999 A
- JP 2023148047 A
- JP 2023148050 A


### Non-patent literature cited in the description


- *CHEMICAL ABSTRACTS*, 2301497-10-7
- *CHEMICAL ABSTRACTS*, 2299658-69-6
- *Journal of Medicinal Chemistry*, 1969, vol. 12 (1), 45-48
- **PETER G.M.WUTS** ; **THEODORA W.GREENE**. Greene's PROTECTIVE GROUPS in ORGANIC SYNTHESIS. John Wiley and Sons, 2007
- *Bioorganic & Medicinal Chemistry*, 2004, vol. 12, 6171-6182
- *Journal of Organic Chemistry*, 2005, vol. 70, 567-574
- *Organic Process Research and Development*, 2021, vol. 25, 1167-1175
- *Organic & Biomolecular Chemistry*, 2020, vol. 18, 3281-3287
- *The Journal of Antibiotics*, 2000, vol. 53, 1071-1085
- *HETEROCYCLES*, 2009, vol. 78, 463-470
- *Organic Letters*, 2004, vol. 6, 2361-2364